(19) Europäisches Patentamt
European Patent Office
Office européen des brevets

(11) EP 4 241 846 A1

(12) **EUROPEAN PATENT APPLICATION**
published in accordance with Art. 153(4) EPC

(43) Date of publication:
**13.09.2023 Bulletin 2023/37**

(21) Application number: **21889026.7**

(22) Date of filing: **20.10.2021**

(51) International Patent Classification (IPC):
***A61P 35/00*** *(2006.01)* ***A61P 43/00*** *(2006.01)*
***C08G 61/12*** *(2006.01)* ***A61K 47/56*** *(2017.01)*
***A61K 47/61*** *(2017.01)* ***A61K 47/64*** *(2017.01)*
***A61K 47/68*** *(2017.01)* ***A61K 49/00*** *(2006.01)*
***A61K 41/00*** *(2020.01)*

(52) Cooperative Patent Classification (CPC):
**A61K 9/16; A61K 31/795; A61K 41/00; A61K 47/54; A61K 47/56; A61K 47/61; A61K 47/62; A61K 47/64; A61K 47/68; A61K 49/00; A61P 35/00; A61P 43/00; C08G 61/12; C08K 5/00; C08L 65/00**

(86) International application number:
**PCT/JP2021/038728**

(87) International publication number:
**WO 2022/097473 (12.05.2022 Gazette 2022/19)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **04.11.2020 JP 2020184168**

(71) Applicant: **SUMITOMO CHEMICAL COMPANY, LIMITED**
**Tokyo 103-6020 (JP)**

(72) Inventors:
- **SATO, Takahiro**
  **Tsukuba-shi, Ibaraki 300-3294 (JP)**
- **MASE, Kentaro**
  **Osaka-shi, Osaka 554-8558 (JP)**
- **HIGASHI, Kiyoshi**
  **Osaka-shi, Osaka 554-8558 (JP)**

(74) Representative: **Vossius & Partner**
**Patentanwälte Rechtsanwälte mbB**
**Siebertstraße 3**
**81675 München (DE)**

(54) **NANOPARTICLES, PHOTOACOUSTIC CONTRAST AGENT, PHOTODYNAMIC THERAPEUTIC AGENT, AND PHOTOTHERMAL THERAPEUTIC AGENT**

(57) A nanoparticle including at least one amphiphilic molecule and a polymer compound having a constitutional unit represented by Formula (1):

[Chemical Formula 1]

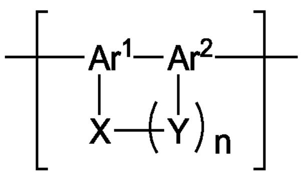

(1)

wherein, $Ar^1$ and $Ar^2$ represent a trivalent aromatic hydrocarbon ring group or a trivalent aromatic heterocyclic group; X and Y represent -O-, -S-, -C(=O)-, -S(=O)-, $-SO_2-$, $-CR_2-$, $-SiR_2-$, -NR-, -BR-, -PR-, or -P(=O) (R)-; and R represents an alkyl group, aryl group, or the like; and n represents an integer of 1 or more.

EP 4 241 846 A1

## Description

TECHNICAL FIELD

The present invention relates to a nanoparticle, photoacoustic contrast agent, photodynamic therapy drug, and photothermal therapy drug.

BACKGROUND ART

In recent years, photoacoustic imaging (PAI) employing a photoacoustic effect has attracted attention as a candidate for a noninvasive molecular probe that can be used for deep diagnosis and is easy to handle. PAI is a technique in which a biological tissue is irradiated with excitation light in a near-infrared light region, which has excellent transmittance to biological tissues, and a photoacoustic signal generated when a light absorber absorbing the excitation light thermally expands is captured. As a photoacoustic material used for the PAI light absorber, for example, Patent Document 1 proposes a nanoparticle including silicon naphthalocyanine and a surfactant.

PRIOR ART DOCUMENT

PATENT DOCUMENT

Patent Document 1: JP-A-2014-129317

SUMMARY OF THE INVENTION

PROBLEMS TO BE SOLVED BY THE INVENTION

However, conventional photoacoustic materials have not necessarily had sufficient generation efficiency of a photoacoustic signal. Therefore, an object of the present invention is to provide a nanoparticle excellent in generation efficiency of a photoacoustic signal, and a photoacoustic contrast agent, a photodynamic therapy drug, and a photothermal therapy drug employing the nanoparticles.

MEANS FOR SOLVING THE PROBLEMS

The present invention provides the following [1] to [26] :

[1] A nanoparticle including at least one amphiphilic molecule and a polymer compound having a constitutional unit represented by Formula (1):

[Chemical Formula 1]

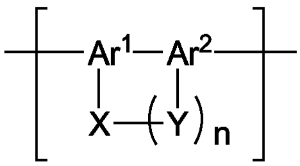

(1)

wherein, $Ar^1$ and $Ar^2$ each independently represent a trivalent aromatic hydrocarbon ring group or a trivalent aromatic heterocyclic group, and these groups optionally have a substituent;
X and Y each independently represent -O-, -S-, - C(=O)-, -S(=O)-, $-SO_2-$, $-CR_2-$, $-SiR_2-$, -NR-, -BR-, -PR-, or -P(=O)(R)-;
R represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, or a heteroaryloxycarbonyl group, and these groups optionally have a substituent;
in the case that a plurality of Rs are present, they may be identical or different from each other;
n represents an integer of 1 or more; and

in the case that n is 2 or more, a plurality of Ys may be identical or different from each other.

[2] The nanoparticle according to [1], wherein $Ar^1$ and $Ar^2$ are trivalent aromatic heterocyclic groups in Formula (1).
[3] The nanoparticle according to [1] or [2], wherein the constitutional unit represented by Formula (1) is a constitutional unit represented by Formula (1a):

[Chemical Formula 2]

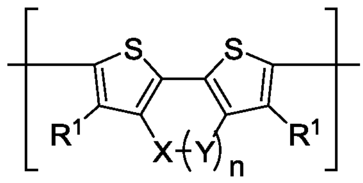
(1a)

wherein X, Y, and n have the same meanings as described above; and
$R^1$ represents a hydrogen atom or an alkyl group optionally having a substituent.

[4] The nanoparticle according to any one of [1] to [3], wherein n is 1.
[5] The nanoparticle according to any one of [1] to [4], wherein the constitutional unit represented by Formula (1) is a constitutional unit represented by Formula (1b):

[Chemical Formula 3]

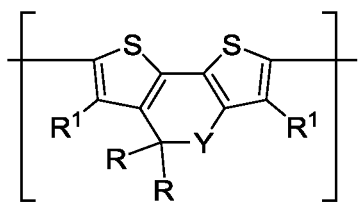
(1b)

wherein, R, $R^1$, and Y have the same meanings as described above.
[6] The nanoparticle according to any one of [1] to [5], wherein Y is -O-.
[7] The nanoparticle according to any one of [1] to [6], wherein the polymer compound further has a constitutional unit represented by Formula (3):

[Chemical Formula 4]

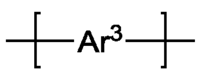
(3)

wherein $Ar^3$ is different from the constitutional unit represented by Formula (1) and represents an arylene group optionally having a substituent or a divalent heterocyclic group optionally having a substituent.
[8] The nanoparticle according to [7], wherein the constitutional unit represented by Formula (3) includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e):

[Chemical Formula 5]

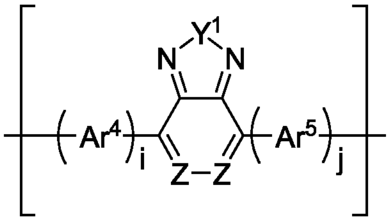

(2a)

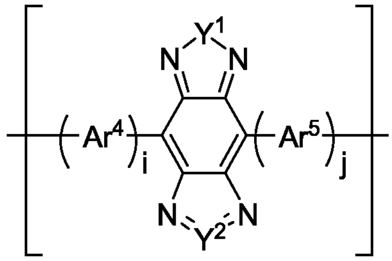

(2b)

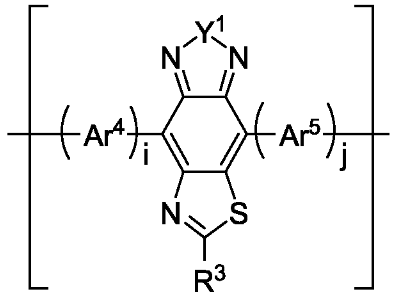

(2c)

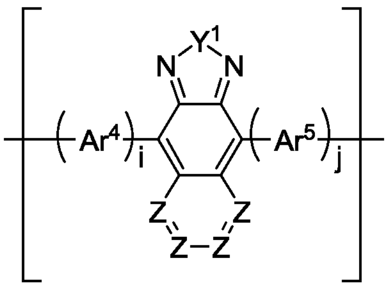

(2d)

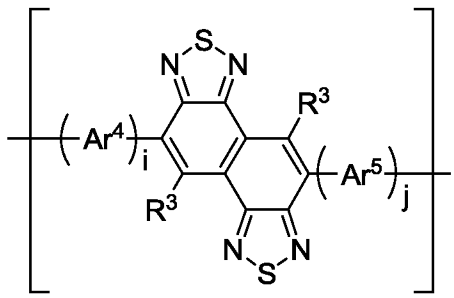

(2e)

wherein, $R^3$ represents a hydrogen atom, a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, an amino group, a silyl group, a silyloxy group, a silylthio group, a silylamino group, a monovalent heterocyclic group, a heteroaryloxy group, a heteroarylthio group, an arylalkenyl group, an arylethynyl group, a carboxyl group, an alkyloxycarbonyl group, an aryloxycarbonyl group, an arylalkyloxycarbonyl group, a heteroaryloxycarbonyl group, a nitro group, or a cyano group; these groups optionally have a substituent; and a plurality of $R^3$s may be identical or different from each other;

$Ar^4$ and $Ar^5$ are different from the constitutional unit represented by Formula (1), and each independently represent an arylene group or a divalent heterocyclic group, and these groups optionally have a substituent;

i and j each independently represent an integer of 0 or 1;

$Y^1$ represents -O-, -S-, -S(=O)-, $-SO_2-$, -Se-, -Te-, or $-N(R^3)-$;

$Y^2$ represents =S=, =Se=, or =Te=;

Z represents $-C(R^4)=$ or -N=; $R^4$ has the same meaning as $R^3$; and a plurality of $R^4$s may be identical or different from each other and may be bound to each other to make a ring; and

a plurality of Zs may be identical or different from each other.

[9] The nanoparticle according to any one of [1] to [8], wherein the amphiphilic molecule has a group represented by Formula (5a):

[Chemical Formula 6]

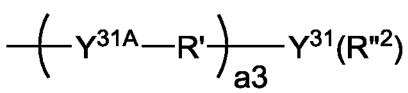

(5a)

wherein, a3 represents an integer of 0 or 1 or more;

R' represents an alkylene group, a cycloalkylene group, or an arylene group, and these groups optionally have a substituent;

in the case that a plurality of R's are present, they may be identical or different from each other;

$R''^2$ represents a hydrogen atom, an alkyl group, a cycloalkyl group, or an aryl group, and these groups optionally

have a substituent;

$Y^{31}$ represents -O-, -S-, -C(=O)O-, or -N(R"$^2$)-;

$Y^{31A}$ represents -O- or -S-; and

in the case that a plurality of $Y^{31A}$s are present, they may be identical or different from each other.

[10] The nanoparticle according to [9], wherein R"$^2$ represents a hydrogen atom, and $Y^{31}$ represents -S-, - C(=O)O-, or -NH- in Formula (5a).

[11] The nanoparticle according to any one of [1] to [10], wherein at least one capture molecule selected from the group consisting of an antibody, a sugar chain, an aptamer, a receptor, a peptide, a transport protein, and an enzyme is bound to at least one of the polymer compound or the amphiphilic molecule.

[12] A nanoparticle including a polymer compound having a group represented by Formula (5) and having a constitutional unit represented by Formula (1A):

[Chemical Formula 7]

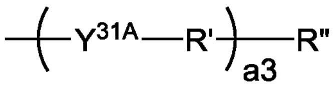

(5)

wherein, a3 represents an integer of 0 or 1 or more;

R' represents an alkylene group, a cycloalkylene group, or an arylene group, and these groups optionally have a substituent;

in the case that a plurality of R's are present, they may be identical or different from each other;

R" represents -$Y^{31}$(R"$^2$) or -$Y^{31}$(M) ;

R"$^2$ represents a hydrogen atom, an alkyl group, a cycloalkyl group, or an aryl group, and these groups optionally have a substituent;

M represents an alkali metal cation or a quaternary ammonium;

$Y^{31}$ represents -O-, -S-, -C(=O)O-, or -N(R"$^2$)-;

$Y^{31A}$ represents -O- or -S-; and

in the case that a plurality of $Y^{31A}$s are present, they may be identical or different from each other, and

[Chemical Formula 8]

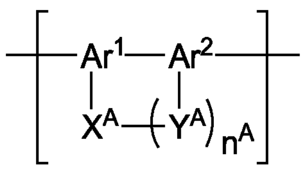

(1A)

wherein, $Ar^1$ and $Ar^2$ each independently represent a trivalent aromatic hydrocarbon ring group or a trivalent aromatic heterocyclic group, and these groups optionally have a substituent;

$X^A$ and $Y^A$ each independently represent -O-, -S-, - C(=O)-, -S(=O)-, -$SO_2$-, -$CR^A{}_2$-, -$SiR^A{}_2$-, -$NR^A$-, -$BR^A$-, -$PR^A$-, or -P(=O)($R^A$)-;

$R^A$ represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, a heteroaryloxycarbonyl group, or the group represented by Formula (5), and a group other than the group represented by Formula (5) optionally has the group represented by Formula (5) or a substituent;

in the case that a plurality of $R^A$s are present, they may be identical or different from each other;

$n^A$ represents an integer of 1 or more; and

in the case that $n^A$ is 2 or more, a plurality of $Y^A$s may be identical or different from each other.

[13] The nanoparticle according to [12], wherein $Ar^1$ and $Ar^2$ are trivalent aromatic heterocyclic groups in Formula (1A).

[14] The nanoparticle according to [12] or [13], wherein the constitutional unit represented by Formula (1A) is a constitutional unit represented by Formula (1Aa):

[Chemical Formula 9]

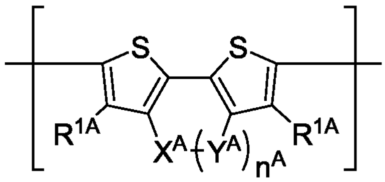

(1Aa)

wherein, $X^A$, $Y^A$, and $n^A$ have the same meanings as described above; and
$R^{1A}$ represents a hydrogen atom, the group represented by Formula (5), or an alkyl group optionally having the group represented by Formula (5) or a substituent.

[15] The nanoparticle according to any one of [12] to [14], wherein $n^A$ is 1.
[16] The nanoparticle according to any one of [12] to [15], wherein the constitutional unit represented by Formula (1A) is a constitutional unit represented by Formula (1Ab):

[Chemical Formula 10]

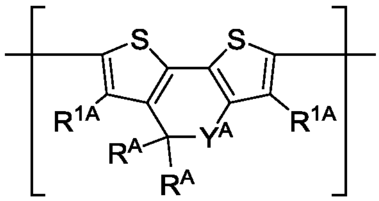

(1Ab)

wherein, $R^A$, $R^{1A}$ and $Y^A$ have the same meanings as described above.
[17] The nanoparticle according to any one of [12] to [16], wherein $Y^A$ is -O-.
[18] The nanoparticle according to any one of [12] to [17], wherein the polymer compound further has a constitutional unit represented by Formula (3):

[Chemical Formula 11]

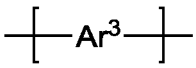

(3)

wherein $Ar^3$ is different from the constitutional unit represented by Formula (1A) and represents an arylene group optionally having a substituent or a divalent heterocyclic group optionally having a substituent.
[19] The nanoparticle according to [18], wherein the constitutional unit represented by Formula (3) includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e):

[Chemical Formula 12]

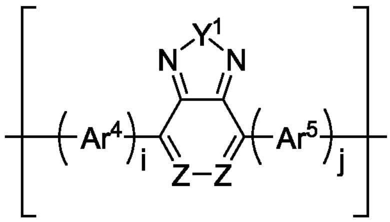

(2a)

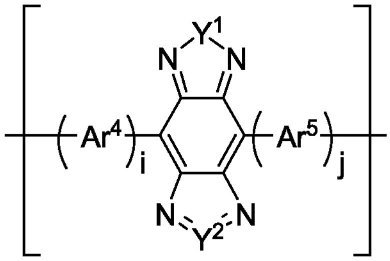

(2b)

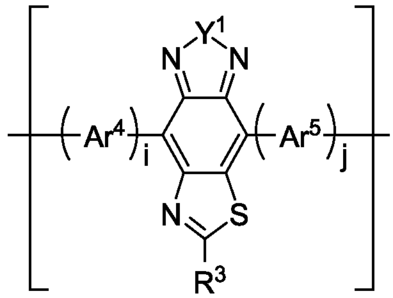

(2c)

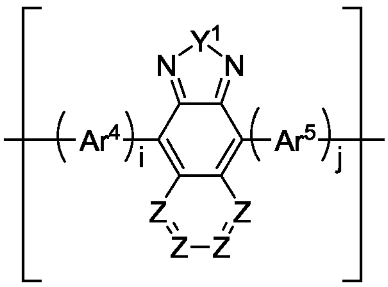

(2d)

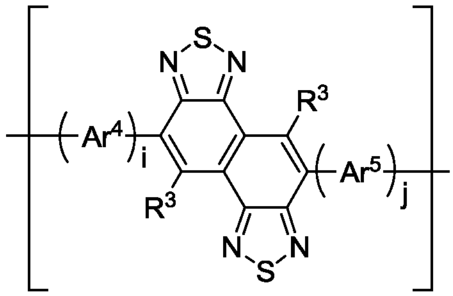

(2e)

wherein, $R^3$ represents a hydrogen atom, a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, an amino group, a silyl group, a silyloxy group, a silylthio group, a silylamino group, a monovalent heterocyclic group, a heteroaryloxy group, a heteroarylthio group, an arylalkenyl group, an arylethynyl group, a carboxyl group, an alkyloxycarbonyl group, an aryloxycarbonyl group, an arylalkyloxycarbonyl group, a heteroaryloxycarbonyl group, a nitro group, or a cyano group; these groups optionally have a substituent; and a plurality of $R^3$s may be identical or different from each other;

$Ar^4$ and $Ar^5$ are different from the constitutional unit represented by Formula (1A) and each independently represent an arylene group or a divalent heterocyclic group, and these groups optionally have a substituent;

i and j each independently represent an integer of 0 or 1;

$Y^1$ represents -O-, -S-, -S(=O)-, $-SO_2-$, -Se-, -Te-, or $-N(R^3)-$;

$Y^2$ represents =S=, =Se=, or =Te=;

Z represents $-C(R^4)=$ or -N=; $R^4$ has the same meaning as $R^3$; and a plurality of $R^4$s may be identical or different from each other and may be bound to each other to make a ring; and

a plurality of Zs may be identical or different from each other.

[20] The nanoparticle according to any one of [12] to [19], wherein the group represented by Formula (5) is a group represented by Formula (5a):

[Chemical Formula 13]

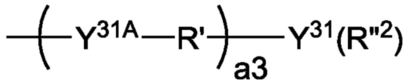

(5a)

wherein, a3, R', $Y^{31A}$, $R''^2$, and $Y^{31}$ have the same meanings as described above.

[21] The nanoparticle according to [20], wherein $R''^2$ represents a hydrogen atom, and $Y^{31}$ represents -S-, - C(=O)O-,

or -NH- in Formula (5a).

[22] The nanoparticle according to any one of [12] to [21], wherein at least one capture molecule selected from the group consisting of an antibody, a sugar chain, an aptamer, a receptor, a peptide, a transport protein, and an enzyme is bound to the polymer compound.

[23] The nanoparticle according to any one of [1] to [22], which is used for a photoacoustic contrast agent, a photodynamic therapy drug, or a photothermal therapy drug.

[24] A photoacoustic contrast agent containing the nanoparticle according to any one of [1] to [22].

[25] A photodynamic therapy drug containing the nanoparticle according to any one of [1] to [22].

[26] A photothermal therapy drug containing the nanoparticle according to any one of [1] to [22].

EFFECT OF THE INVENTION

According to the present invention, it is possible to provide a nanoparticle having excellent generation efficiency of a photoacoustic signal. In addition, according to the present invention, it is possible to provide a photoacoustic contrast agent, a photodynamic therapy drug, and a photothermal therapy drug employing the nanoparticles.

BRIEF DESCRIPTION OF THE DRAWINGS

Fig. 1 is a fluorescence microscopic observation image for evaluating accumulation of antibody-conjugated nanoparticles and nanoparticles in a cancer cell line. Fig. 1(a) is a fluorescence microscopic observation image in the case of using antibody-conjugated nanoparticles 1 of Example 10, Fig. 1(b) is a fluorescence microscopic observation image in the case of using antibody-conjugated nanoparticles 2 of Example 11, and Fig. 1(c) is a fluorescence microscopic observation image in the case of using nanoparticles 2 of Example 2.

Fig. 2 is a fluorescence microscopic observation image for evaluating accumulation of antibody-conjugated nanoparticles in a cancer cell line. Fig. 2(a) is a fluorescence microscopic observation image in the case of using antibody-conjugated nanoparticles 3 of Example 12, and Fig. 2(b) is a fluorescence microscopic observation image in the case of using antibody-conjugated nanoparticles 4 of Example 13.

Fig. 3 is a fluorescence microscopic observation image for evaluating accumulation of antibody-conjugated nanoparticles in a cancer cell line. Fig. 3 (a) is a fluorescence microscopic observation image in the case of using antibody-conjugated nanoparticles 5 of Example 14, and Fig. 3 (b) is a fluorescence microscopic observation image in the case of using antibody-conjugated nanoparticles 6 of Example 15.

MODE FOR CARRYING OUT THE INVENTION

Hereinafter, preferred embodiments of the present embodiment will be described in detail.

[Nanoparticles]

The nanoparticles of the present embodiment are particles having a particle diameter of nm (nanometer) order, that is, less than 1000 nm. However, even if particles having a particle diameter of 1000 nm or more are contained, aggregation of particles having an average particle diameter of less than 1000 nm is included in the definition of the nanoparticles of the present embodiment.

<First Embodiment>

A nanoparticle of the first embodiment includes a polymer compound having a constitutional unit represented by Formula (1) and at least one amphiphilic molecule.

(Polymer Compound)

The polymer compound has a constitutional unit represented by Formula (1). The polymer compound having the constitutional unit represented by Formula (1) tends to exhibit an absorption maximum in a wavelength range with high organism transmittance (NIR-I (700 to 950 nm) or NIR-II (1000 to 1400 nm)), and is useful as a light absorber of a photoacoustic probe.

[Chemical Formula 14]

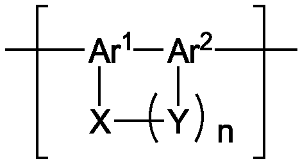

(1)

In Formula (1), $Ar^1$ and $Ar^2$ each independently represent a trivalent aromatic hydrocarbon ring group or a trivalent aromatic heterocyclic group, and these groups optionally have a substituent. As shown in Formula (1), $Ar^1$ has three binding sites, one of which is a site binding with $Ar^2$, another is a site binding with X, and the other indicates a site binding with a hydrogen atom or another atom. Another atom may be one of atoms constituting another constitutional unit. As shown in Formula (1), $Ar^2$ has three binding sites, one of which is a site binding with $Ar^1$, another is a site binding with Y, and the other indicates a site binding with a hydrogen atom or another atom. Another atom may be one of atoms constituting another constitutional unit.

The trivalent aromatic hydrocarbon ring group means a group obtained by removing three hydrogen atoms each directly bound to a carbon atom as the ring member from an aromatic hydrocarbon. Examples of the aromatic hydrocarbon ring in the trivalent aromatic hydrocarbon ring group include benzene, naphthalene, anthracene, phenanthrene, pyrene, perylene, tetracene, and pentacene rings. The aromatic hydrocarbon ring is preferably a benzene ring or a naphthalene ring, and more preferably a benzene ring. The trivalent aromatic hydrocarbon ring group optionally has a substituent.

The trivalent aromatic heterocyclic group means a group obtained by removing three hydrogen atoms each directly bound to a carbon atom as the ring member from an aromatic heterocyclic compound. Examples of the aromatic heterocycle in the trivalent aromatic heterocyclic group include pyridine, pyrimidine, pyridazine, pyrazine, quinoline, isoquinoline, quinoxaline, quinazoline, acridine, phenanthroline, thiophene, benzothiophene, dibenzothiophene, furan, benzofuran, dibenzofuran, pyrrole, indole, dibenzopyrrole, silole, benzosilole, dibenzosilole, borole, benzoborole, and dibenzoborole rings. The aromatic heterocycle is preferably a thiophene ring, a furan ring, or a pyrrole ring, more preferably a thiophene ring or a furan ring, and still more preferably a thiophene ring. The trivalent aromatic heterocyclic group optionally has a substituent.

$Ar^1$ and $Ar^2$ are preferably trivalent aromatic heterocyclic groups from the viewpoint of increasing the wavelength of the absorption maximum of the polymer compound.

In Formula (1), X and Y each independently represent -O-, -S-, -C(=O)-, -S(=O)-, $-SO_2-$, $-CR_2-$, $-SiR_2-$, -NR-, - BR-, -PR-, or -P(=O)(R)-. Here, R represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, or a heteroaryloxycarbonyl group, and these groups optionally have a substituent. In the case that a plurality of Rs are present, they may be identical or different from each other.

The alkyl group represented by R may be linear, branched, or cyclic. The number of carbon atoms of the alkyl group is normally 1 to 30, excluding the number of carbon atoms of a substituent. The alkyl group optionally has a substituent, and examples of the substituent include halogen atoms such as fluorine, chlorine, bromine, and iodine atoms. Specific examples of the alkyl group include chain alkyl groups such as methyl, ethyl, propyl, isopropyl, butyl, isobutyl, sec-butyl, tert-butyl, pentyl, isopentyl, 2-methylbutyl, 1-methylbutyl, hexyl, isohexyl, 3-methylpentyl, 2-methylpentyl, 1-methylpentyl, heptyl, octyl, isooctyl, 2-ethylhexyl, 3,7-dimethyloctyl, nonyl, decyl, undecyl, dodecyl, tetradecyl, hexadecyl, octadecyl, and eicosyl groups; and cycloalkyl groups such as cyclopentyl, cyclohexyl, and adamantyl groups.

The alkenyl group represented by R may be linear, branched, or cyclic. The number of carbon atoms of the alkenyl group is normally 2 to 30, excluding the number of carbon atoms of a substituent. The alkenyl group optionally has a substituent, and examples of the substituent include a halogen atom. Specific examples of the alkenyl group include vinyl, 1-propenyl, 2-propenyl, 1-butenyl, 2-butenyl, 3-butenyl, 3-pentenyl, 4-pentenyl, 1-hexenyl, 5-hexenyl, and 7-octenyl groups.

The alkynyl group represented by R may be linear, branched, or cyclic. The number of carbon atoms of the alkynyl group is normally 2 to 20, excluding the number of carbon atoms of a substituent. The alkynyl group optionally has a substituent, and examples of the substituent include a halogen atom. Specific examples of the alkynyl group include ethynyl, 1-propynyl, 2-propynyl, 1-butynyl, 2-butynyl, 3-butynyl, 3-pentynyl, 4-pentynyl, 1-hexynyl, and 5-hexynyl groups.

The alkyl group in the alkyloxy group represented by R may be linear, branched, or cyclic. The number of carbon atoms of the alkyloxy group is normally 1 to 20, excluding the number of carbon atoms of a substituent. The

alkyloxy group optionally has a substituent, and examples of the substituent include a halogen atom and an alkoxy group (e.g., 1 to 20 carbon atoms). Specific examples of the alkyloxy group include methoxy, ethoxy, propoxy, isopropoxy, butoxy, isobutoxy, tert-butoxy, pentyloxy, hexyloxy, cyclohexyloxy, heptyloxy, octyloxy, 2-ethylhexyloxy, nonyloxy, decyloxy, 3,7-dimethyloctyloxy, and lauryloxy groups. Specific examples of the alkyloxy group having a substituent(s) include trifluoromethoxy, pentafluoroethoxy, perfluorobutoxy, perfluorohexyloxy, perfluorooctyloxy, methoxymethyloxy, and 2-methoxyethyloxy groups.

The aryl group represented by R means a group obtained by removing one hydrogen atom directly bound to a carbon atom as the ring member from an aromatic hydrocarbon. The number of carbon atoms of the aryl group is normally 6 to 60, excluding the number of carbon atoms of a substituent. The aryl group optionally has a substituent, and examples of the substituent include a halogen atom and an alkoxy group (e.g., 1 to 20 carbon atoms). Specific examples of the aryl group optionally having a substituent(s) include a phenyl group, a C1-C12 alkoxyphenyl group (Here, the C1-C12 alkoxy group refers to an alkoxy group having 1 to 12 carbon atoms. The C1-C12 alkoxy group is preferably a C1-C8 alkoxy group, and more preferably a C1-C6 alkoxy group. The C1-C8 alkoxy group refers to an alkoxy group having 1 to 8 carbon atoms, and the C1-C6 alkoxy group refers to an alkoxy group having 1 to 6 carbon atoms. Specific examples of the C1-C12 alkoxy group, the C1-C8 alkoxy group, and the C1-C6 alkoxy group include those described and exemplified above for the alkoxy group. The same applies hereinafter.), a C1-C12 alkylphenyl group (The C1-C12 alkyl group refers to an alkyl group having 1 to 12 carbon atoms. The C1-C12 alkyl group is preferably a C1-C8 alkyl group, and more preferably a C1-C6 alkyl group. The C1-C8 alkyl group refers to an alkyl group having 1 to 8 carbon atoms, and the C1-C6 alkyl group refers to an alkyl group having 1 to 6 carbon atoms. Specific examples of the C1-C12 alkyl group, the C1-C8 alkyl group, and the C1-C6 alkyl group include those described and exemplified above for the alkyl group. The same applies hereinafter.), a 1-naphthyl group, a 2-naphthyl group, and a pentafluorophenyl group.

The number of carbon atoms of the aryloxy group represented by R is normally 6 to 60, excluding the number of carbon atoms of a substituent. The aryl group in the aryloxy group optionally has a substituent. Specific examples of the aryloxy group optionally having a substituent(s) include phenoxy, C1-C12 alkyloxyphenoxy, C1-C12 alkylphenoxy, 1-naphthyloxy, 2-naphthyloxy, and pentafluorophenyloxy groups.

The number of carbon atoms of the arylalkyl group represented by R is normally 7 to 60, excluding the number of carbon atoms of a substituent. The arylalkyl group optionally has a substituent. Specific examples of the arylalkyl group optionally having a substituent(s) include phenyl-C1-C12 alkyl, C1-C12 alkyloxyphenyl-C1-C12 alkyl, C1-C12 alkylphenyl-C1-C12 alkyl, 1-naphthyl-C1-C12 alkyl, and 2-naphthyl-C1-C12 alkyl groups.

The number of carbon atoms of the acyl group represented by R is normally 2 to 20, excluding the number of carbon atoms of a substituent. Specific examples of the acyl group include acetyl, propionyl, butyryl, isobutyryl, pivaloyl, benzoyl, trifluoroacetyl, and pentafluorobenzoyl groups.

The number of carbon atoms of the acyloxy group represented by R is normally 2 to 20, excluding the number of carbon atoms of a substituent. Specific examples of the acyloxy group include acetoxy, propionyloxy, butyryloxy, isobutyryloxy, pivaloyloxy, benzoyloxy, trifluoroacetyloxy, and pentafluorobenzoyloxy groups.

The number of carbon atoms of the amide group represented by R is normally 1 to 20, excluding the number of carbon atoms of a substituent. The amide group means a group obtained by removing a hydrogen atom bound to the nitrogen atom from an amide. Specific examples of the amide group include formamide, acetamide, propioamide, butyroamide, benzamide, trifluoroacetamide, pentafluorobenzamide, diformamide, diacetamide, dipropioamide, dibutyroamide, dibenzamide, ditrifluoroacetamide, and dipentafluorobenzamide groups.

The amino group represented by R means a $NH_2$ group and a substituted amino group having an optionally substituted alkyl group or an optionally substituted aryl group. The number of carbon atoms of the substituted amino group is normally 1 to 40, excluding the number of carbon atoms of a substituent. Specific examples of the amino group include methylamino, dimethylamino, ethylamino, diethylamino, propylamino, dipropylamino, isopropylamino, diisopropylamino, butylamino, isobutylamino, tert-butylamino, pentylamino, hexylamino, cyclohexylamino, heptylamino, octylamino, 2-ethylhexylamino, nonylamino, decylamino, 3,7-dimethyloctylamino, laurylamino, cyclopentylamino, dicyclopentylamino, cyclohexylamino, dicyclohexylamino, pyrrolidyl, piperidyl, ditrifluoromethylamino, phenylamino, diphenylamino, C1-C12 alkokyloxyphenylamino, di(C1-C12 alkyloxyphenyl)amino, di(C1-C12 alkylphenyl)amino, 1-naphthylamino, 2-naphthylamino, pentafluorophenylamino, pyridylamino, pyridazinylamino, pyrimidylamino, pyrazylamino, triazylamino, phenyl-C1-C12 alkylamino, C1-C12 alkyloxyphenyl-C1-C12 alkylamino, C1-C12 alkylphenyl-C1-C12 alkylamino, di(C1-C12 alkyloxyphenyl-C1-C12 alkyl)amino, di(C1-C12 alkylphenyl-C1-C12 alkyl)amino, 1-naphthyl-C1-C12 alkylamino, and 2-naphthyl-C1-C12 alkylamino groups.

Examples of the monovalent aromatic heterocyclic group represented by R include a group obtained by removing one hydrogen atom directly bound to a carbon atom as the ring member from an aromatic heterocyclic compound such as furan, thiophene, pyrrole, oxazole, isoxazole, thiazole, isothiazole, imidazole, pyrazole, furazan, triazole, thiadiazole, oxadiazole, tetrazole, pyridine, pyridazine, pyrimidine, pyrazine, triazine, benzofuran, isobenzofuran, benzothiophene, indole, isoindole, indolizine, isoquinoline, benzimidazole, benzothiazole, indazole, naphthyridine, quinoxaline, quinazoline, quinazolidine, cinnoline, phthalazine, purine, pteridine, carbazole, phenanthridine, acridine, β-carboline, perimidine, or phenanthroline. The monovalent aromatic heterocyclic group optionally has a substituent.

The heteroaryl group in the heteroaryloxy group represented by R has the same meaning as the above-described monovalent aromatic heterocyclic group. The number of carbon atoms of the heteroaryloxy group is normally 2 to 60, excluding the number of carbon atoms of a substituent. The heteroaryloxy group optionally has a substituent. Specific examples of the heteroaryloxy group optionally having a substituent(s) include thienyloxy, C1-C12 alkylthienyloxy, pyrrolyloxy, furyloxy, pyridyloxy, C1-C12 alkylpyridyloxy, imidazolyloxy, pyrazolyloxy, triazolyloxy, oxazolyloxy, thiazoloxy, and thiadiazoloxy groups.

The heteroaryl group in the heteroarylthio group represented by R has the same meaning as the above-described monovalent aromatic heterocyclic group. The number of carbon atoms of the heteroarylthio group is normally 2 to 60, excluding the number of carbon atoms of a substituent. The heteroarylthio group optionally has a substituent. Specific examples of the heteroarylthio group optionally having a substituent(s) include thienylmercapto, C1-C12 alkylthienylmercapto, pyrrolylmercapto, furylmercapto, pyridylmercapto, C1-C12 alkylpyridylmercapto, imidazolylmercapto, pyrazolylmercapto, triazolylmercapto, oxazolylmercapto, thiazole mercapto, and thiadiazole mercapto groups.

The alkyloxy group in the alkyloxycarbonyl group represented by R has the same meaning as the above-described alkyloxy group. The number of carbon atoms of the alkyloxycarbonyl group is normally 1 to 20, excluding the number of carbon atoms of a substituent. The alkyloxycarbonyl group optionally has a substituent. Specific examples of the alkyloxycarbonyl group include a group having a methyl ester structure, a group having an ethyl ester structure, and a group having a butyl ester structure.

The aryloxy group in the aryloxycarbonyl group represented by R has the same meaning as the above-described aryloxy group. The number of carbon atoms of the aryloxycarbonyl group is normally 6 to 60, excluding the number of carbon atoms of a substituent. The aryloxycarbonyl group optionally has a substituent. Specific examples of the aryloxycarbonyl group include a group having a phenyl ester structure.

The arylalkyl group in the arylalkyloxycarbonyl group represented by R has the same meaning as the above-described arylalkyl group. The number of carbon atoms of the arylalkyloxycarbonyl group is normally 7 to 60, excluding the number of carbon atoms of a substituent. The arylalkyloxycarbonyl group optionally has a substituent. Specific examples of the arylalkyloxycarbonyl group include a group having the above-described arylalkyl group.

The heteroaryl group in the heteroaryloxycarbonyl group represented by R has the same meaning as the above-described monovalent aromatic heterocyclic group. The number of carbon atoms of the heteroaryloxycarbonyl group is normally 2 to 60, excluding the number of carbon atoms of a substituent. The heteroaryloxycarbonyl group optionally has a substituent. Specific examples of the arylalkyloxycarbonyl group include a group having the above-described monovalent aromatic heterocyclic group.

In this specification, examples of the substituent include halogen atoms (such as fluorine, chlorine, bromine, and iodine atoms), and cyano, alkyl, aryl, monovalent aromatic heterocyclic, alkoxy, aryloxy, amino, alkenyl, and alkynyl groups.

X is preferably -C(=O)- or $-CR_2-$, and more preferably $-CR_2-$, from the viewpoint of ease of production of a monomer to be a raw material of the polymer compound. R in X is preferably an alkyl group or an aryl group from the viewpoint of ease of production of a monomer to be a raw material of the polymer compound.

Y is preferably -O-, -S-, -C(=O)-, $-CR_2-$, or -NR-, more preferably -O-, -C(=O)-, or $-CR_2-$, and still more preferably -O-, from the viewpoint of ease of production of a monomer to be a raw material of the polymer compound. R in Y is preferably an alkyl group or an aryl group from the viewpoint of ease of production of a monomer to be a raw material of the polymer compound.

n represents an integer of 1 or more, in the case that n is 2 or more, a plurality of Ys may be identical or different, n is preferably 1 from the viewpoint of ease of production of a monomer to be a raw material of the polymer compound.

Examples of -X-(Y)n- include groups represented by Formulae (26) to (28).

[Chemical Formula 15]

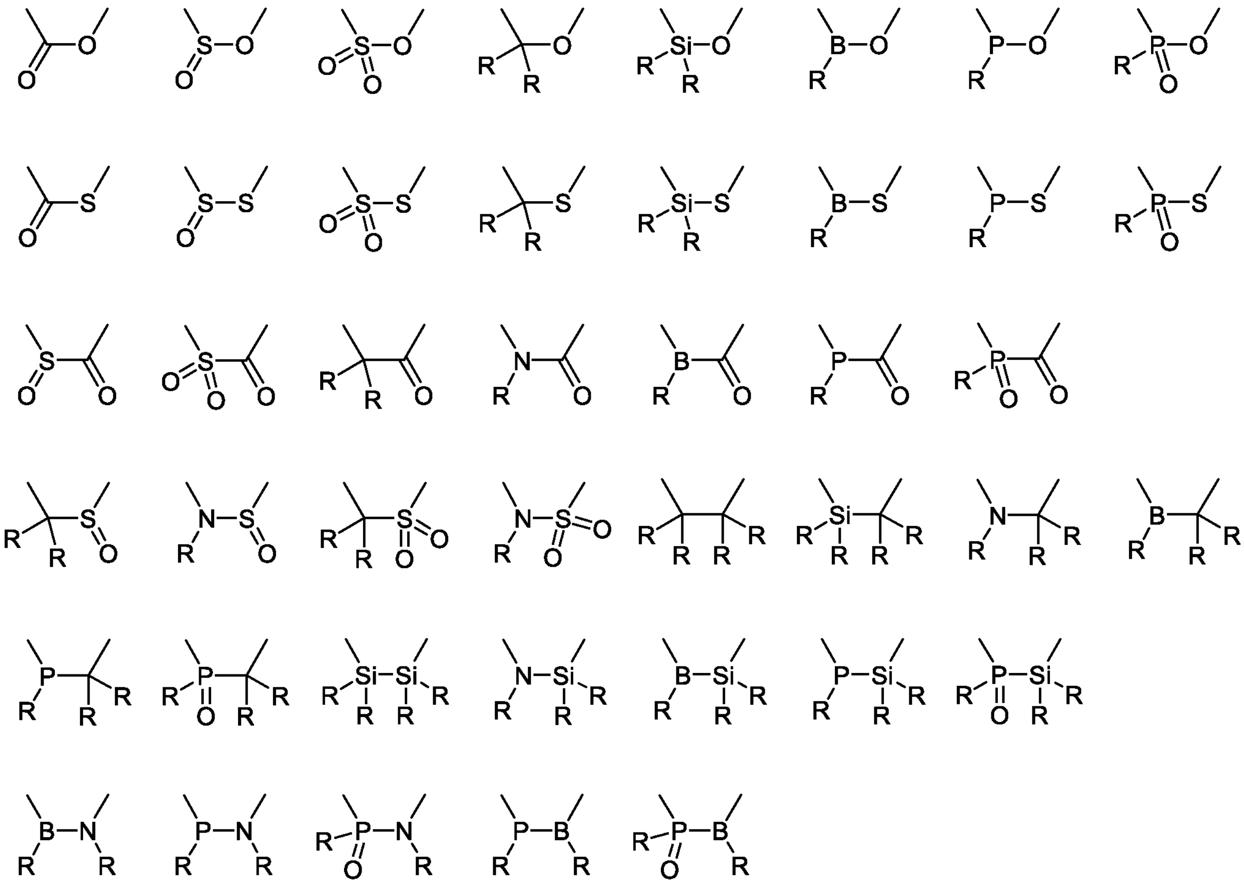

(26)

[Chemical Formula 16]

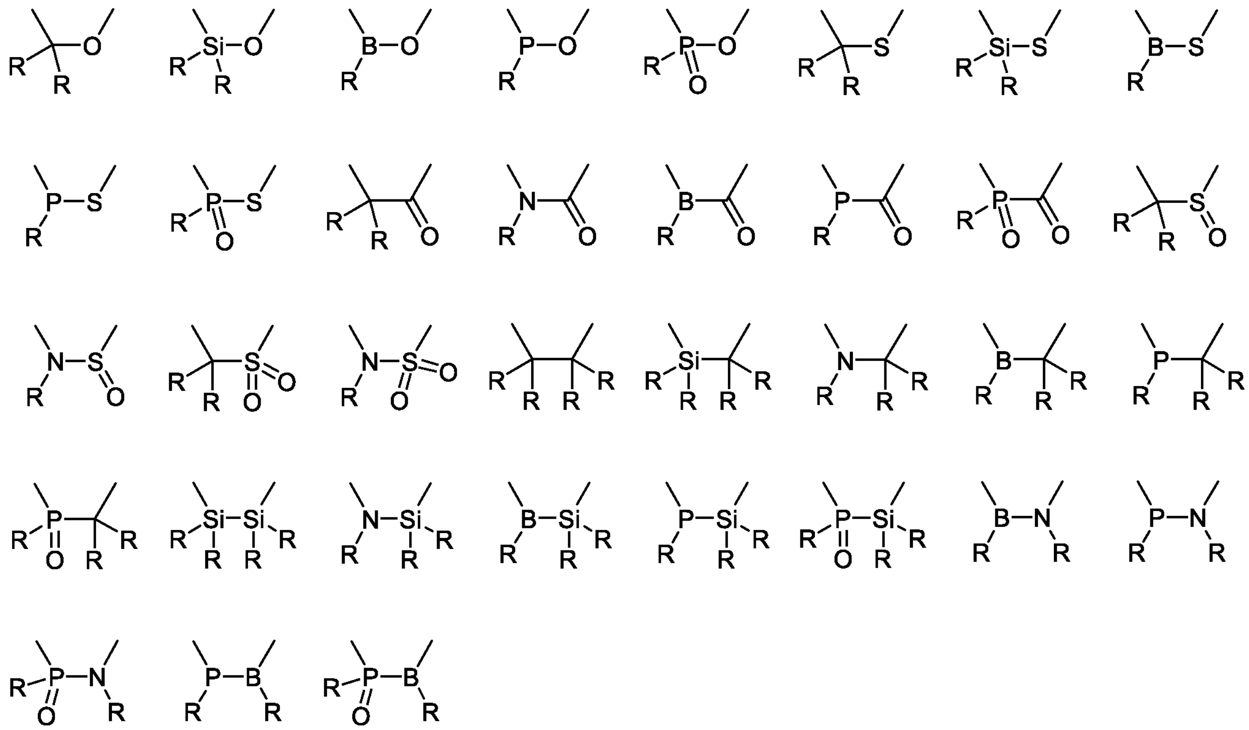

(27)

[Chemical Formula 17]

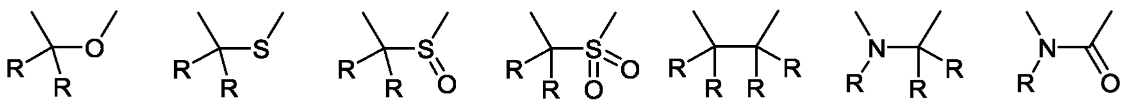

(28)

-X-(Y)n- is preferably a group represented by Formula (27) or (28), and more preferably a group represented by Formula (28), from the viewpoint of stability of the polymer compound.

The constitutional unit represented by Formula (1) is preferably a constitutional unit represented by Formula (1').

[Chemical Formula 18]

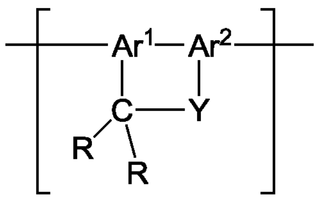

(1')

In Formula (1'), $Ar^1$, $Ar^2$, Y, and R have the same meanings as described above.

Examples of the constitutional unit represented by Formula (1) (or the constitutional unit represented by Formula (1')) include constitutional units represented by Formulae (301) to (345). In the formulae, $R^{AA}$ represents a hydrogen atom or a substituent.

[Chemical Formula 19]

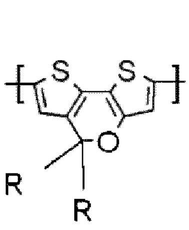
(301)

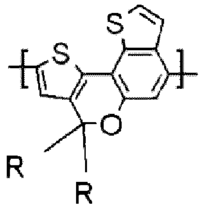
(302)

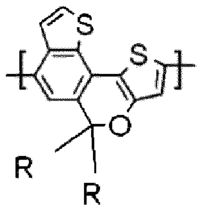
(303)

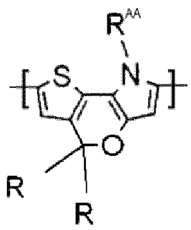
(304)

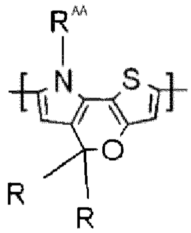
(305)

[Chemical Formula 20]

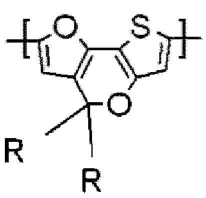
(306)

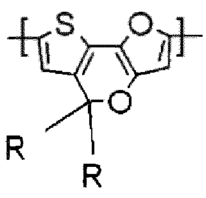
(307)

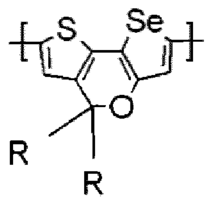
(308)

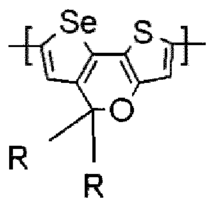
(309)

[Chemical Formula 21]

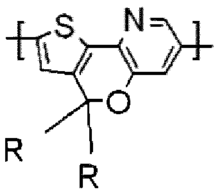
(310)

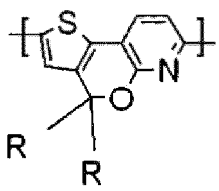
(311)

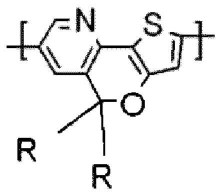
(312)

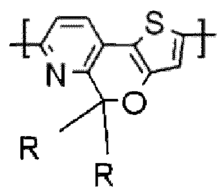
(313)

[Chemical Formula 22]

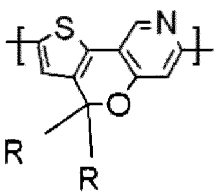
(314)

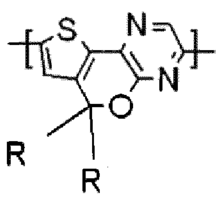
(315)

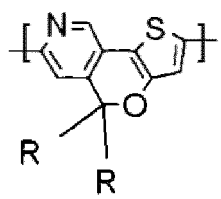
(316)

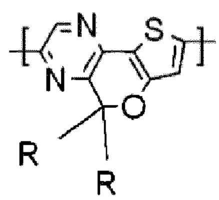
(317)

[Chemical Formula 23]

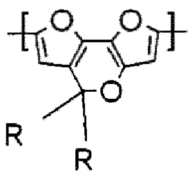
(318)

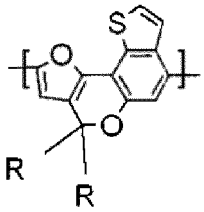
(319)

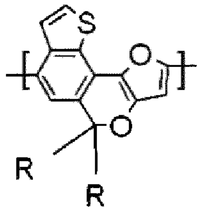
(320)

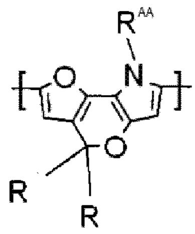
(321)

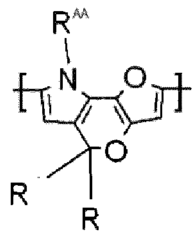
(322)

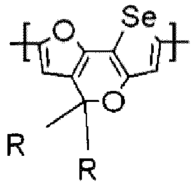
(323)

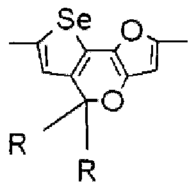
(324)

[Chemical Formula 24]

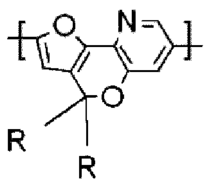
(325)

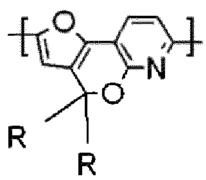
(326)

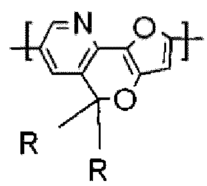
(327)

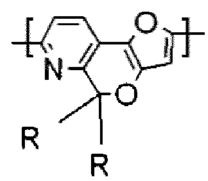
(328)

[Chemical Formula 25]

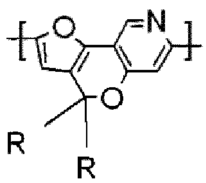
(329)

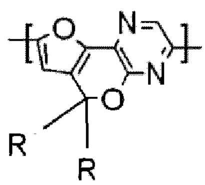
(330)

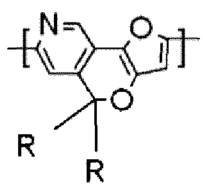
(331)

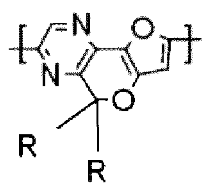
(332)

[Chemical Formula 26]

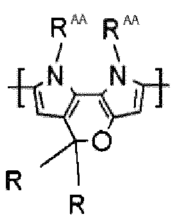
(333)

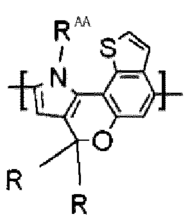
(334)

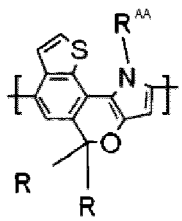
(335)

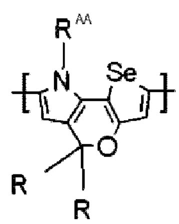
(336)

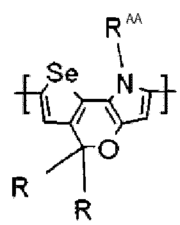
(337)

[Chemical Formula 27]

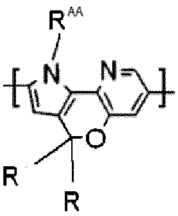
(338)

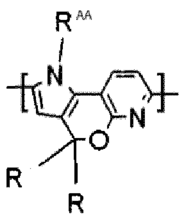
(339)

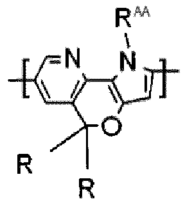
(340)

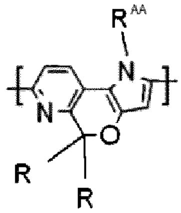
(341)

[Chemical Formula 28]

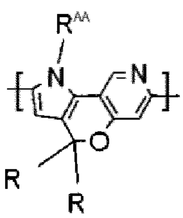
(342)

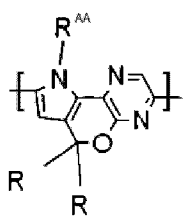
(343)

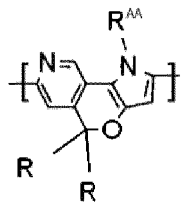
(344)

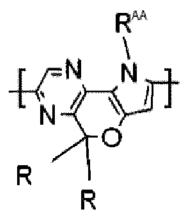
(345)

The constitutional unit represented by Formula (1) is preferably a constitutional unit represented by Formula (1a) .

[Chemical Formula 29]

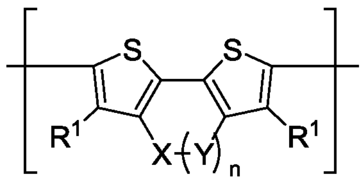
(1a)

In Formula (1a), X, Y, and n have the same meanings as described above. n is preferably 1 from the viewpoint of ease of production of a monomer to be a raw material of the polymer compound.

$R^1$ represents a hydrogen atom or an alkyl group optionally having a substituent. The alkyl group represented by $R^1$ has the same meaning as the alkyl group represented by R.

The constitutional unit represented by Formula (1) is preferably a constitutional unit represented by Formula (1b) .

[Chemical Formula 30]

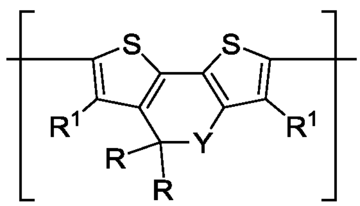
(1b)

In Formula (1b), R, $R^1$, and Y have the same meanings as described above. Y is preferably -O-.R is preferably an alkyl group or an aryl group from the viewpoint of ease of production of a monomer to be a raw material of the polymer

compound.

The polymer compound further has a constitutional unit represented by Formula (3).

[Chemical Formula 31]

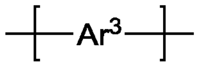 (3)

In Formula (3), $Ar^3$ is different from the constitutional unit represented by Formula (1) and represents an arylene group optionally having a substituent or a divalent heterocyclic group optionally having a substituent.

The arylene group represented by $Ar^3$ means a group obtained by removing two hydrogen atoms each directly bound to a carbon atom as the ring member from an aromatic hydrocarbon, and the number of carbon atoms of the arylene group is normally 6 to 60, excluding the number of carbon atoms of a substituent. The arylene group optionally has a substituent. Examples of the substituent include a halogen atom and an alkoxy group (e.g., 1 to 20 carbon atoms).

Examples of the arylene group include a group obtained by removing two hydrogen atoms each directly bound to a carbon atom as the ring member from a monocyclic aromatic hydrocarbon (examples thereof include benzene) or a polycyclic aromatic hydrocarbon (examples thereof include bicyclic aromatic hydrocarbons such as naphthalene and indene; tricyclic aromatic hydrocarbons such as anthracene, phenanthrene, dihydrophenanthrene, and fluorene; tetracyclic aromatic hydrocarbons such as benzanthracene, benzophenanthrene, benzofluorene, pyrene, and fluoranthene; pentacyclic aromatic hydrocarbons such as dibenzanthracene, dibenzophenanthrene, dibenzofluorene, perylene, and benzofluoranthene; hexacyclic aromatic hydrocarbons such as spirobifluorene; and heptacyclic aromatic hydrocarbons such as benzospirobifluorene and acenaphthofluoranthene). The arylene groups include a group obtained by removing two hydrogen atoms each directly bound to a carbon atom as the ring member from an aromatic hydrocarbon formed by linking a plurality of the above aromatic hydrocarbons (e.g., biphenyldiyl and terphenyldiyl groups).

The divalent heterocyclic group represented by $Ar^3$ means a group obtained by removing two hydrogen atoms each directly bound to a carbon atom as the ring member from a heterocyclic compound such as furan, thiophene, pyrrole, pyrroline, pyrrolidine, oxazole, isoxazole, thiazole, isothiazole, imidazole, imidazoline, imidazolidine, pyrazole, pyrazoline, prazolidine, furazan, triazole, thiadiazole, oxadiazole, tetrazole, pyran, pyridine, piperidine, thiopyran, pyridazine, pyrimidine, pyrazine, piperazine, morpholine, triazine, benzofuran, isobenzofuran, benzothiophene, indole, isoindole, indolizine, indoline, isoindoline, chromene, chroman, isochroman, benzopyran, quinoline, isoquinoline, quinolidine, benzimidazole, benzothiazole, indazole, naphthyridine, quinoxaline, quinazoline, quinazolidine, cinnoline, phthalazine, phthalazine, purine, pteridine, carbazole, xanthene, phenanthridine, acridine, β-carboline, perimidine, phenanthroline, thianthrene, phenoxathiin, phenoxazine, phenothiazine, or phenazine. The divalent heterocyclic group optionally has a substituent. Examples of the substituent include a halogen atom and an alkoxy group (e.g., 1 to 20 carbon atoms). The divalent heterocyclic groups include a group obtained by removing two hydrogen atoms each directly bound to a carbon atom as the ring member from a heterocyclic compound formed by linking a plurality of the above heterocyclic compounds.

Examples of the divalent heterocyclic group represented by $Ar^3$ also include groups represented by Formulae (2Aa) to (2Ae).

[Chemical Formula 32]

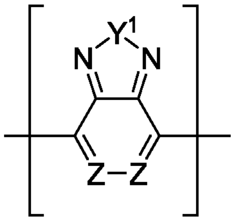

(2Aa)

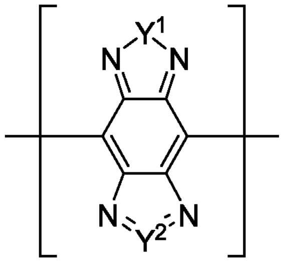

(2Ab)

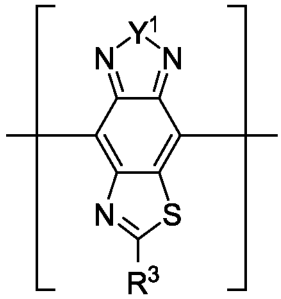

(2Ac)

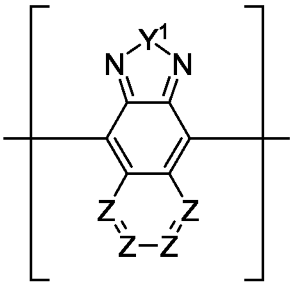

(2Ad)

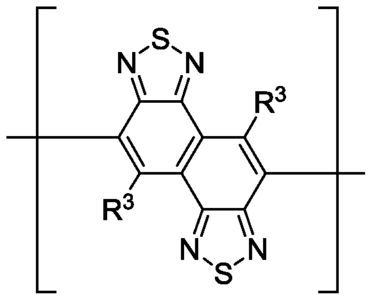

(2Ae)

In Formulae (2Aa) to (2Ae), $R^3$ represents a hydrogen atom, a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, an amino group, a silyl group, a silyloxy group, a silylthio group, a silylamino group, a monovalent heterocyclic group, a heteroaryloxy group, a heteroarylthio group, an arylalkenyl group, an arylethynyl group, a carboxyl group, an alkyloxycarbonyl group, an aryloxycarbonyl group, an arylalkyloxycarbonyl group, a heteroaryloxycarbonyl group, a nitro group, or a cyano group, and these groups optionally have a substituent.

Alkyl, alkyloxy, aryl, aryloxy, arylalkyl, acyl, acyloxy, amide, amino, heteroaryloxy, heteroarylthio, carboxyl, alkyloxycarbonyl, aryloxycarbonyl, arylalkyloxycarbonyl, and heteroaryloxycarbonyl groups each represented by $R^3$ have the same meanings as those each represented by R, respectively.

The alkyl group in the alkylthio group represented by $R^3$ may be linear, branched, or cyclic. The number of carbon atoms of the alkylthio group is normally 1 to 20, excluding the number of carbon atoms of a substituent. The alkylthio group optionally has a substituent, and examples of the substituent include a halogen atom. Specific examples of the alkylthio group include methylthio, ethylthio, propylthio, isopropylthio, butylthio, isobutylthio, tert-butylthio, pentylthio, hexylthio, cyclohexylthio, heptylthio, octylthio, 2-ethylhexylthio, nonylthio, decylthio, 3,7-dimethyloctylthio, and laurylthio groups. Specific examples of the alkylthio group having a substituent(s) include a trifluoromethylthio group.

The number of carbon atoms of the arylthio group represented by $R^3$ is normally 6 to 60, excluding the number of carbon atoms of a substituent. The aryl group in the arylthio group optionally has a substituent. The substituents include a halogen atom and an alkoxy group (e.g., 1 to 20 carbon atoms). Specific examples of the arylthio group optionally having a substituent(s) include phenylthio, C1-C12 alkoxyphenylthio, C1-C12 alkylphenylthio, 1-naphthylthio, 2-naphthylthio, and pentafluorophenylthio groups.

The arylalkyl group in the arylalkyloxy group represented by $R^3$ has the same meaning as the arylalkyl group represented by R. The number of carbon atoms of the arylalkyloxy group is normally 7 to 60, excluding the number of carbon atoms of a substituent. The arylalkyl group optionally has a substituent.

The arylalkyl group in the arylalkylthio group represented by $R^3$ has the same meaning as the arylalkyl group represented by R. The number of carbon atoms of the arylalkylthio group is normally 7 to 60, excluding the number of carbon atoms of a substituent. The arylalkyl group optionally has a substituent.

The acid imide group represented by $R^3$ means a group obtained by removing a hydrogen atom bound to the nitrogen atom from an acid imide. Specific examples of the acid imide group include a succinimide group and a phthalic

acid imide group.

The imine residue represented by $R^3$ means a residue obtained by, from an imine compound represented by the formula: $H-N=C(R^{47})_2$ or the formula: $H-C(R^{48})=N-R^{49}$, removing the "H" in the formulae. In the formulae, $R^{47}$, $R^{48}$, and $R^{49}$ each independently represent an alkyl group represented by R, an aryl group represented by R, an alkenyl group represented by R, an alkynyl group represented by R, or a monovalent heterocyclic group mentioned later. The alkyl, aryl, alkenyl, alkynyl, and monovalent heterocyclic groups in $R^{47}$, $R^{48}$, and $R^{49}$ optionally have a substituent. A plurality of $R^{47}$s may be identical or different from each other, and may link to each other to form a ring structure.

The silyl group represented by $R^3$ means a silyl group having an optionally substituted alkyl group or an optionally substituted aryl group. Specific examples of the silyl group include trimethylsilyl, triethylsilyl, tri-n-propylsilyl, tri-iso-propylsilyl, tertbutyldimethylsilyl, triphenylsilyl, tri-p-xylylsilyl, tribenzylsilyl, diphenylmethylsilyl, tert-butyldiphenylsilyl, and dimethylphenylsilyl groups.

The silyloxy group represented by $R^3$ is a group in which an oxygen atom is bound to the above-described silyl group. Specific examples of the silyloxy group include trimethylsilyloxy, triethylsilyloxy, tri-n-propylsilyloxy, tri-iso-propylsilyloxy, tert-butyldimethylsilyloxy, triphenylsilyloxy, tri-p-xylylsilyloxy, tribenzylsilyloxy, diphenylmethylsilyloxy, tert-butyldiphenylsilyloxy, and dimethylphenylsilyloxy groups.

The silylthio group represented by $R^3$ is a group in which a sulfur atom is bound to the above-described silyl group. Specific examples of the silylthio group include trimethylsilylthio, triethylsilylthio, tri-n-propylsilylthio, tri-iso-propylsilylthio, tert-butyldimethylsilylthio, triphenylsilylthio, tri-p-xylylsilylthio, tribenzylsilylthio, diphenylmethylsilylthio, tert-butyldiphenylsilylthio, and dimethylphenylsilylthio groups.

The monovalent heterocyclic group represented by $R^3$ means a group obtained by removing one hydrogen atom directly bound to a carbon atom as the ring member from a heterocyclic compound such as furan, thiophene, pyrrole, pyrroline, pyrrolidine, oxazole, isoxazole, thiazole, isothiazole, imidazole, imidazoline, imidazolidine, pyrazole, pyrazoline, prazolidine, furazan, triazole, thiadiazole, oxadiazole, tetrazole, pyran, pyridine, piperidine, thiopyran, pyridazine, pyrimidine, pyrazine, piperazine, morpholine, triazine, benzofuran, isobenzofuran, benzothiophene, indole, isoindole, indolizine, indoline, isoindoline, chromene, chroman, isochroman, benzopyran, quinoline, isoquinoline, quinolidine, benzimidazole, benzothiazole, indazole, naphthyridine, quinoxaline, quinazoline, quinazolidine, cinnoline, phthalazine, phthalazine, purine, pteridine, carbazole, xanthene, phenanthridine, acridine, β-carboline, perimidine, phenanthroline, thianthrene, phenoxathiin, phenoxazine, phenothiazine, or phenazine. The monovalent heterocyclic group optionally has a substituent. The monovalent heterocyclic group is preferably a monovalent aromatic heterocyclic group represented by R.

The number of carbon atoms of the arylalkenyl group represented by $R^3$ is normally 8 to 20, excluding the number of carbon atoms of a substituent. The aryl group in the arylalkenyl group optionally has a substituent. The substituents include a halogen atom and an alkoxy group (e.g., 1 to 20 carbon atoms). Specific examples of the arylalkenyl group include a styryl group.

The number of carbon atoms of the arylalkynyl group represented by $R^3$ is normally 8 to 20, excluding the number of carbon atoms of a substituent. The aryl group in the arylalkynyl group optionally has a substituent. The substituents include a halogen atom and an alkoxy group (e.g., 1 to 20 carbon atoms). Specific examples of the arylalkynyl group include a phenylacetylenyl group.

$R^3$ is preferably a hydrogen atom, an alkyl group, or an aryl group from the viewpoint of ease of production of a monomer to be a raw material of the polymer compound.

$Y^1$ represents -O-, -S-, -S(=O)-, $-SO_2-$, -Se-, -Te-, or $-N(R^3)$ -, and is preferably -O-, -S-, or -Se- from the viewpoint of increasing the wavelength of the absorption maximum of the polymer compound. $Y^2$ represents =S=, =Se=, or =Te=, and is preferably =S= or =Se= from the viewpoint of ease of production of a monomer to be a raw material of the polymer compound.

Z represents $-C(R^4)=$ or -N=. $R^4$ has the same meaning as $R^3$. A plurality of $R^4$s may be identical or different from each other and may be bound to each other to make a ring. A plurality of Zs may be identical or different from each other.

The constitutional unit represented by Formula (3) preferably includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e).

[Chemical Formula 33]

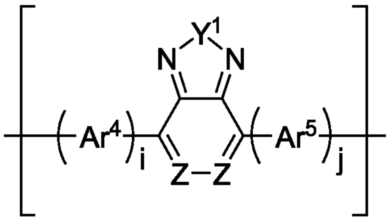

(2a)

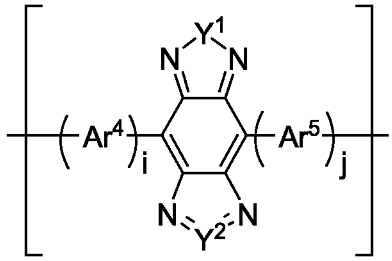

(2b)

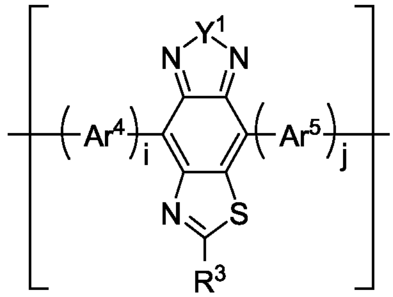

(2c)

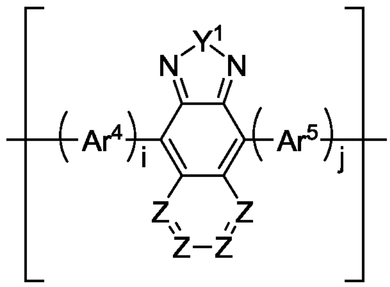

(2d)

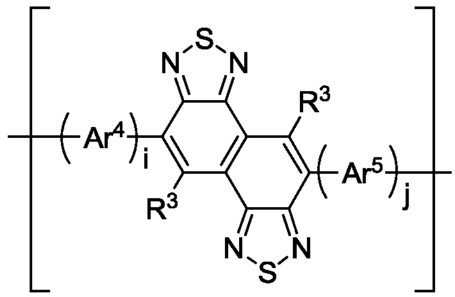

(2e)

In Formulae (2a) to (2e), $R^3$, $Y^1$, $Y^2$, and Z have the same meanings as described above.

i and j each independently represent an integer of 0 or 1. i and j are preferably 0 from the viewpoint of increasing the wavelength of the absorption maximum of the polymer compound. Meanwhile, i and j are preferably 1 from the viewpoint of improving the absorption coefficient of the polymer compound.

$Ar^4$ and $Ar^5$ are different from the constitutional unit represented by Formula (1) and each independently represent an arylene group or a divalent heterocyclic group, and these groups optionally have a substituent. The arylene group has the same meaning as the arylene group represented by $Ar^3$, and the divalent heterocyclic group has the same meaning as the divalent heterocyclic group represented by $Ar^3$. $Ar^4$ and $Ar^5$ are preferably a phenylene group or a thiophene ring group (thiophenediyl group), and more preferably a thiophene ring group (thiophenediyl group) from the viewpoint of increasing the wavelength of the absorption maximum of the polymer compound. These groups optionally have a substituent.

Among the constitutional units represented by Formulae (2a) to (2e), the constitutional unit represented by Formula (2a) or the constitutional unit represented by Formula (2d) is preferable from the viewpoint of improving the light stability of the polymer compound, and the constitutional unit represented by Formula (2d) is more preferable from the viewpoint of increasing the wavelength of the absorption maximum of the polymer compound.

Examples of the constitutional unit represented by Formula (2a) include constitutional units represented by Formulae (2a-1) to (2a-6). Among them, the constitutional unit represented by Formula (2a-1), (2a-2), or (2a-3) is preferable from the viewpoint of ease of production of a monomer to be a raw material of the polymer compound, and the constitutional unit represented by Formula (2a-2) or (2a-3) is more preferable from the viewpoint of increasing the wavelength of the absorption maximum of the polymer compound.

[Chemical Formula 34]

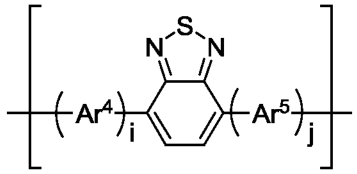

(2a-1)

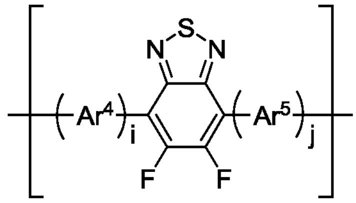

(2a-2)

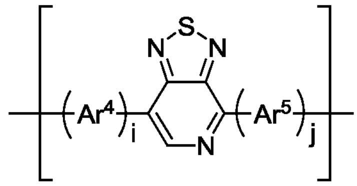

(2a-3)

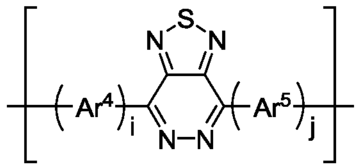

(2a-4)

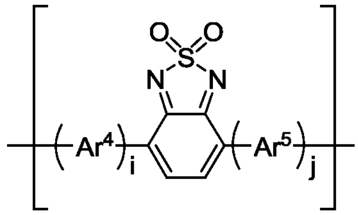

(2a-5)

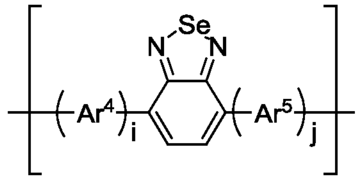

(2a-6)

Examples of the constitutional unit represented by Formula (2b) include constitutional units represented by Formulae (2b-1) to (2b-4). Among them, the constitutional unit represented by Formula (2b-1) is preferable from the viewpoint of ease of production of a monomer to be a raw material of the polymer compound.

[Chemical Formula 35]

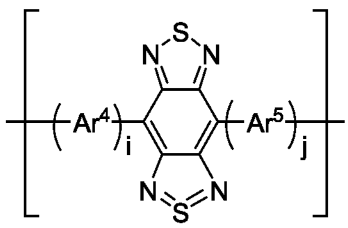

(2b-1)

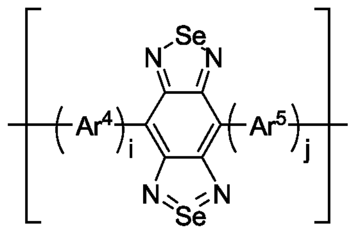

(2b-2)

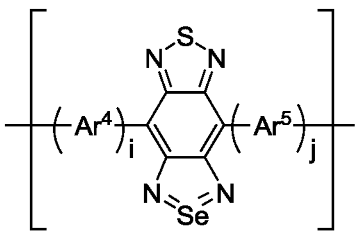

(2b-3)

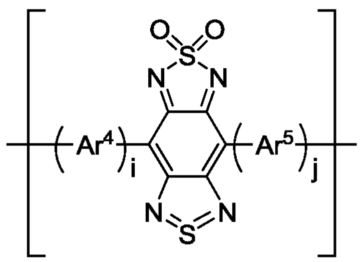

(2b-4)

Examples of the constitutional unit represented by Formula (2c) include constitutional units represented by Formulae (2c-1) to (2c-4).

[Chemical Formula 36]

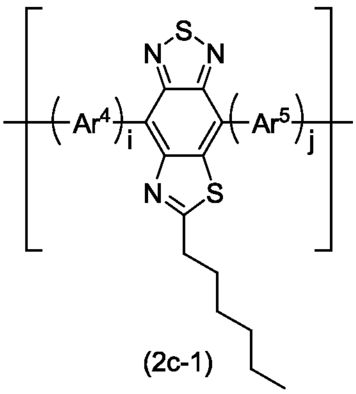

(2c-1)

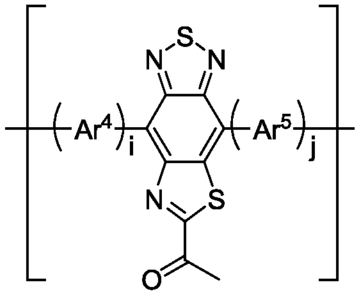

(2c-2)

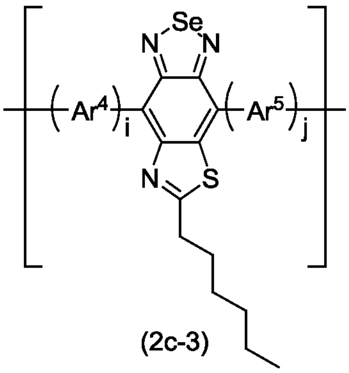

(2c-3)

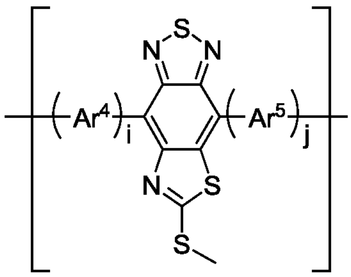

(2c-4)

Examples of the constitutional unit represented by Formula (2d) include constitutional units represented by Formulae (2d-1) to (2d-4). Among them, the constitutional unit represented by Formula (2d-1) or (2d-2) is preferable from the viewpoint of ease of production of a monomer to be a raw material of the polymer compound, and the constitutional unit represented by Formula (2d-2) is more preferable from the viewpoint of increasing the wavelength of the absorption maximum of the polymer compound.

[Chemical Formula 37]

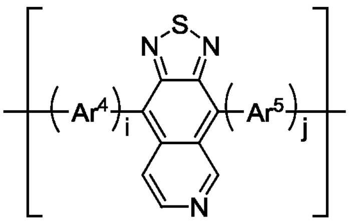

(2d-1)

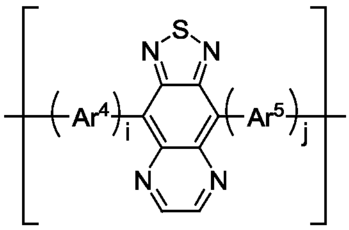

(2d-2)

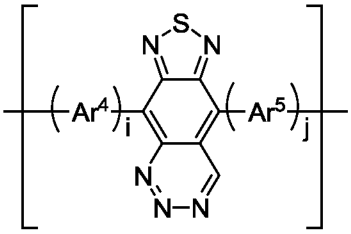

(2d-3)

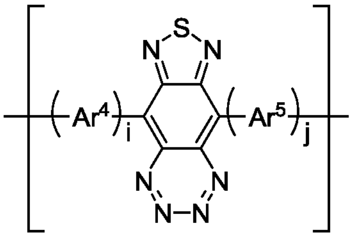

(2d-4)

Examples of the constitutional unit represented by Formula (2e) include constitutional units represented by Formulae (2e-1) to (2e-3).

[Chemical Formula 38]

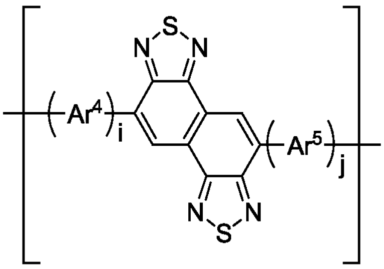

(2e-1)

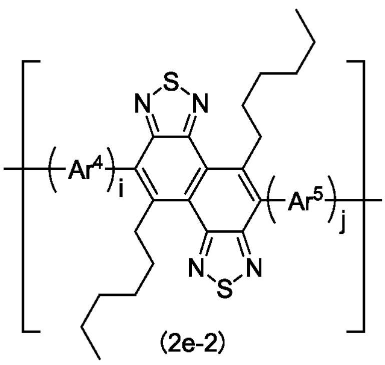

(2e-2)

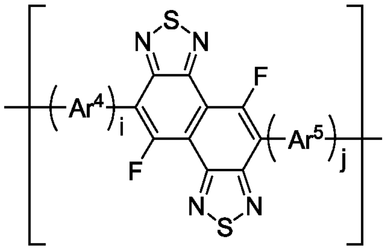

(2e-3)

In the case that the polymer compound contains the constitutional unit represented by Formula (1) and the constitutional unit represented by Formula (3), the content of the constitutional unit represented by Formula (1) is preferably 20 to 80% by mass, and the content of the constitutional unit represented by Formula (3) is preferably 20 to 80% by mass, based on all the constitutional units of the polymer compound.

The polymer compound of the present embodiment is generally a compound having a weight-average molecular weight of 1000 or more. The weight-average molecular weight of the polymer compound is preferably 1000 to 10000000, more preferably 1000 to 5000000, and still more preferably 1000 to 1000000. If the weight-average molecular weight is less than 1000, nanoparticle aggregation or deterioration of photoacoustic characteristics of the nanoparticles may occur, and if the weight-average molecular weight is more than 10000000, a yield of the nanoparticles may decrease.

As used herein, the weight-average molecular weight means a weight-average molecular weight in terms of polystyrene, which is calculated using gel permeation chromatography (GPC) and using a polystyrene standard sample.

The polymer compound of the present embodiment desirably has high solubility in a solvent for use in the nanoparticles from the viewpoint of ease of nanoparticle preparation. Specifically, the polymer compound of the present embodiment preferably has solubility such that a solution containing 0.01% by mass or more of the polymer compound can be prepared, more preferably has solubility such that a solution containing 0.1% by mass or more of the polymer

compound can be prepared, and still more preferably has solubility such that a solution containing 0.4% by mass or more of the polymer compound can be prepared.

The method for producing the polymer compound including the constitutional unit represented by Formula (1) and the constitutional unit represented by Formula (3) is not particularly limited, but a method using a Suzuki coupling reaction or a Stille coupling reaction is preferable from the viewpoint of ease of synthesis of the polymer compound.

Examples of the method using the Suzuki coupling reaction include a production method having a step of reacting one or more compounds represented by Formula (100) :

$$Q^{100}\text{-}E^{1}\text{-}Q^{200} \quad (100)$$

wherein $E^1$ represents the constitutional unit represented by Formula (3), and $Q^{100}$ and $Q^{200}$ each independently represent a dihydroxyboryl group [$-B(OH)_2$] or a boric acid ester residue,
with two or more compounds represented by Formula (200) :

$$T^{1}\text{-}E^{2}\text{-}T^{2} \quad (200)$$

wherein $E^2$ represents the constitutional unit represented by Formula (1), and $T^1$ and $T^2$ each independently represent a halogen atom or a sulfonic acid residue,
in the presence of a palladium catalyst and a base. $E^1$ preferably includes at least one constitutional unit selected from the group consisting of the constitutional unit represented by Formula (2a), the constitutional unit represented by Formula (2b), the constitutional unit represented by Formula (2c), the constitutional unit represented by Formula (2d), and the constitutional unit represented by Formula (2e).

In the above case, the total number of moles of two or more compounds represented by Formula (200) used in the reaction is preferably excessive with respect to the total number of moles of one or more compounds represented by Formula (100). The total number of moles of one or more compounds represented by Formula (100) is preferably 0.6 to 0.99 mol, and more preferably 0.7 to 0.95 mol, in the case that the total number of moles of two or more compounds represented by Formula (200) used in the reaction is 1 mol.

The boric acid ester residue means a group obtained by removing the hydroxyl group from a boric acid diester, and examples thereof include a dialkyl ester residue, diaryl ester residue, and di(arylalkyl) ester residue. Specific examples of the boric acid ester residue include groups represented by the following formulae:

[Chemical Formula 39]

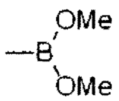 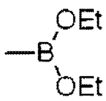 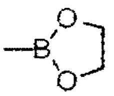 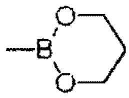 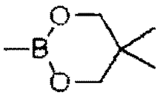 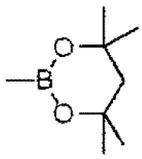 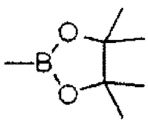 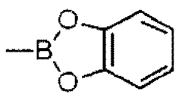

wherein Me represents a methyl group, and Et represents an ethyl group.

The halogen atoms represented by $T^1$ and $T^2$ in Formula (200) include fluorine, chlorine, bromine, and iodine atoms. The halogen atom is preferably a bromine atom or an iodine atom, and more preferably a bromine atom, from the viewpoint of ease of synthesis of the polymer compound.

The sulfonic acid residue represented by $T^1$ or $T^2$ in Formula (200) means an atomic group (a group) obtained by removing the acidic hydrogen from sulfonic acid (-SOsH). Specific examples of the sulfonic acid residue include alkyl sulfonate groups (e.g., methanesulfonate and ethanesulfonate groups), aryl sulfonate groups (e.g., benzenesulfonate and p-toluenesulfonate groups), aryl alkyl sulfonate groups (e.g., a benzylsulfonate group), and a trifluoromethanesulfonate group.

Specific examples of a method for performing the Suzuki coupling reaction include a method in which a reaction is performed in the presence of a base using a palladium catalyst as a catalyst in any solvent.

Examples of the palladium catalyst used in the Suzuki coupling reaction include Pd(0) and Pd(II) catalysts. More specific examples include palladium[tetrakis(triphenylphosphine)], dichlorobis(triphenylphosphine)palladium, palladium acetate, tris(dibenzylideneacetone)dipalladium, and bis(dibenzylideneacetone)palladium. The palladium catalyst is preferably dichlorobis(triphenylphosphine)palladium, palladium acetate, or tris(dibenzylideneacetone)dipalladium from the viewpoint of ease of reaction (polymerization) operation and reaction (polymerization) rate.

The amount of the palladium catalyst to be added is not particularly limited as long as it is an effective amount as a catalyst, but is normally 0.0001 mol to 0.5 mol, and preferably 0.0003 mol to 0.1 mol, with respect to 1 mol of the compound represented by Formula (100).

In the case that palladium acetates are used as the palladium catalyst for use in the Suzuki coupling reaction, for example, a phosphorus compound such as triphenylphosphine, tri(o-tolyl)phosphine, or tri(o-methoxyphenyl)phosphine can be added as a ligand. In this case, the amount of the ligand to be added is normally 0.5 mol to 100 mol, preferably 0.9 mol to 20 mol, and more preferably 1 mol to 10 mol, with respect to 1 mol of the palladium catalyst.

Examples of the base used in the Suzuki coupling reaction include an inorganic base, organic base, and inorganic salt. Examples of the inorganic base include potassium carbonate, sodium carbonate, and barium hydroxide. Examples of the organic base include triethylamine and tributylamine. Examples of the inorganic salt include cesium fluoride.

The amount of the base to be added is normally 0.5 mol to 100 mol, preferably 0.9 mol to 20 mol, and more preferably 1 mol to 10 mol, with respect to 1 mol of the compound represented by Formula (100).

The Suzuki coupling reaction is normally performed in a solvent. Examples of the solvent include N,N-dimethylformamide, toluene, dimethoxyethane, and tetrahydrofuran. The solvent is preferably toluene or tetrahydrofuran from the viewpoint of the solubility of the polymer compound of the present embodiment. The base may be added as an aqueous solution and reacted in a two-phase system. In the case that the inorganic salt is used as the base, it is normally added as an aqueous solution and reacted from the viewpoint of the solubility of the inorganic salt. In the case that the base is added as an aqueous solution and reacted in a two-phase system, a phase transfer catalyst such as a quaternary ammonium salt may be added, as necessary.

The temperature at which the Suzuki coupling reaction is performed depends on the solvent used, but is normally 50 to 160°C, and is preferably 60 to 120°C from the viewpoint of increasing the molecular weight of the polymer compound. In addition, the temperature may be raised to near the boiling point of the solvent, and the solvent may be refluxed. The end point of the reaction may be when the desired degree of polymerization is reached, but the reaction time is normally 0.1 hours to 200 hours, and 1 hour to 30 hours is efficient and preferable.

The Suzuki coupling reaction is performed in a reaction system such that the Pd(0) catalyst is not deactivated in an inert atmosphere such as argon gas or nitrogen gas. For example, the Suzuki coupling reaction is performed in a system in which air is sufficiently removed with argon gas, nitrogen gas, or the like. Specifically, the inside of a polymerization vessel (reaction system) is sufficiently replaced with nitrogen gas to remove air before the polymerization vessel is charged with the compound represented by Formula (100), the compound represented by Formula (200), and dichlorobis(triphenylphosphine)palladium(II), and the inside of the polymerization vessel is further sufficiently replaced with nitrogen gas to remove air. Subsequently, the solvent such as toluene, from which air is removed by bubbling with nitrogen gas in advance, is added, and then the base such as an aqueous sodium carbonate solution, from which air is removed by bubbling with nitrogen gas in advance, is added dropwise to the solution. Thereafter, the polymerization is performed by heating and raising the temperature, for example, at a reflux temperature for 8 hours, while an inert atmosphere is maintained.

Examples of the method using the Stille coupling reaction include a production method having a step of reacting one or more compounds represented by Formula (300) :

$$Q^{300}\text{-}E^{3}\text{-}Q^{400} \quad (300)$$

wherein $E^3$ represents the constitutional unit represented by Formula (3), and $Q^{300}$ and $Q^{400}$ each independently represent a substituted stannyl group, with two or more compounds represented by Formula (200) in the presence of a palladium catalyst. $E^3$ preferably includes at least one constitutional unit selected from the group consisting of the constitutional unit represented by Formula (2a), the constitutional unit represented by Formula (2b), the constitutional unit represented by Formula (2c), the constitutional unit represented by Formula (2d), and the constitutional unit represented by Formula (2e).

The substituted stannyl groups include a group represented by $-SnR^{100}_3$. Here, $R^{100}$ represents a monovalent organic group. Examples of the monovalent organic group include an alkyl group and an aryl group.

The number of carbon atoms of the alkyl group is normally 1 to 30. Specific examples of the alkyl group include chain alkyl groups such as methyl, ethyl, propyl, isopropyl, butyl, isobutyl, sec-butyl, tert-butyl, pentyl, isopentyl, 2-methylbutyl, 1-methylbutyl, hexyl, isohexyl, 3-methylpentyl, 2-methylpentyl, 1-methylpentyl, heptyl, octyl, isooctyl, 2-ethylhexyl, nonyl, decyl, undecyl, dodecyl, tetradecyl, hexadecyl, octadecyl, and eicosyl groups; and cycloalkyl groups such as cyclopentyl, cyclohexyl, and adamantyl groups. Examples of the aryl group include a phenyl group and a naphthyl group.

The substituted stannyl group is preferably $-SnMe_3$, $-SnEt_3$, $-SnBu_3$, or $-SnPh_3$, and more preferably $-SnMe_3$, $-SnEt_3$, or $-SnBu_3$. Here, Me represents a methyl group, Et represents an ethyl group, Bu represents a butyl group, and Ph represents a phenyl group.

The halogen atoms represented by $T^1$ and $T^2$ in Formula (200) include fluorine, chlorine, bromine, and iodine atoms. The halogen atom is preferably a bromine atom or an iodine atom for ease of synthesis of the polymer compound.

Examples of the alkyl sulfonate group represented by $T^1$ or $T^2$ in Formula (200) include methanesulfonate, ethanesulfonate, and trifluoromethanesulfonate groups. Examples of the aryl sulfonate group include benzenesulfonate and p-toluenesulfonate groups. Examples of the aryl sulfonate group include a benzylsulfonate group.

Specifically, the method using the Stille coupling reaction include a method in which a reaction is performed in any solvent under a catalyst, for example, a palladium catalyst.

Examples of the palladium catalyst used in the Stille coupling reaction include Pd(0) and Pd(II) catalysts. More specific examples include palladium[tetrakis(triphenylphosphine)], dichlorobis(triphenylphosphine)palladium, palladium acetate, tris(dibenzylideneacetone)dipalladium, and bis(dibenzylideneacetone)palladium. The palladium catalyst is preferably palladium[tetrakis(triphenylphosphine)] or tris(dibenzylideneacetone)dipalladium from the viewpoint of ease of reaction (polymerization) operation and reaction (polymerization) rate.

The amount of the palladium catalyst to be added and used for the Stille coupling reaction is not particularly limited as long as it is an effective amount as a catalyst, but is normally 0.0001 mol to 0.5 mol, and preferably 0.0003 mol to 0.2 mol, with respect to 1 mol of the compound represented by Formula (100).

In the Stille coupling reaction, a ligand or cocatalyst can also be used as necessary. Examples of the ligand include phosphorus compounds such as triphenylphosphine, tri(o-tolyl)phosphine, tri(o-methoxyphenyl)phosphine, and tris(2-furyl)phosphine; and arsenic compounds such as triphenylarsine and triphenoxyarsine. Examples of the cocatalyst include copper iodide, copper bromide, copper chloride, and copper(I) 2-tenoylate.

When the ligand or the cocatalyst is used, the amount of the ligand or the cocatalyst to be added is normally 0.5 mol to 100 mol, preferably 0.9 mol to 20 mol, more preferably 1 mol to 10 mol, with respect to 1 mol of the palladium catalyst.

The Stille coupling reaction is normally performed in a solvent. Examples of the solvent include N,N-dimethylformamide, N,N-dimethylacetamide, toluene, dimethoxyethane, and tetrahydrofuran. The solvent is preferably toluene or tetrahydrofuran from the viewpoint of the solubility of the polymer compound of the present embodiment. The temperature at which the Stille coupling reaction is performed depends on the solvent used, but is normally 50 to 160°C, and is preferably 60 to 120°C from the viewpoint of increasing the molecular weight of the polymer compound. In addition, the temperature may be raised to near the boiling point of the solvent, and the solvent may be refluxed. The end point of the reaction may be when the desired degree of polymerization is reached, but the time for performing the reaction (reaction time) is normally 0.1 hours to 200 hours, and 1 hour to 30 hours is efficient and preferable.

The Stille coupling reaction is performed in a reaction system such that the Pd catalyst is not deactivated in an inert atmosphere such as argon gas or nitrogen gas. For example, the Suzuki coupling reaction is performed in a system in which air is sufficiently removed with argon gas, nitrogen gas, or the like. Specifically, the inside of a polymerization vessel (reaction system) is sufficiently replaced with nitrogen gas to remove air before the polymerization vessel is charged with the compound represented by Formula (300), the compound represented by Formula (200), and the palladium catalyst, and the inside of the polymerization vessel is further sufficiently replaced with nitrogen gas to remove air. Subsequently, the solvent such as toluene, from which air is removed by bubbling with nitrogen gas in advance, is added, and then the ligand or the cocatalyst is added thereto as necessary. Thereafter, the polymerization is performed by heating and raising the temperature, for example, at a reflux temperature for 8 hours, while an inert atmosphere is maintained.

The number-average molecular weight of the polymer compound of the present embodiment is preferably 1000 to 100000000. If the number-average molecular weight is less than 1000, nanoparticle aggregation or deterioration of photoacoustic characteristics of the nanoparticles may occur, and if the weight-average molecular weight is more than 100000000, a yield of the nanoparticles may decrease.

As used herein, the number-average molecular weight means a number-average molecular weight in terms of polystyrene, which is calculated using gel permeation chromatography (GPC) and using a polystyrene standard sample.

The end group of the polymer compound of the present embodiment may be protected with a stable group because nanoparticle aggregation or deterioration of photoacoustic characteristics of the nanoparticles may occur if the polymerizable active group remains. The stable group is preferably a group having a conjugated bond continuous with the conjugated structure of the main chain. In addition, the stable group may be, for example, a group having a structure bound to an aryl group or a heterocyclic group via a vinylene group.

A monomer to be a raw material of the polymer compound of the present embodiment can be synthesized, for example, according to the description of WO 2011/052709 A.

The wavelength of the absorption maximum of the polymer compound of the present embodiment is preferably 500 to 2000 nm. The wavelength of the absorption maximum of the polymer compound is more preferably 700 to 1500 nm, and still more preferably 750 to 1300 nm, from the viewpoint of enhancing the transmittance to an organism in photoacoustic imaging of the nanoparticles including the polymer compound.

The wavelength of the absorption maximum of the polymer compound can be measured using a spectrophotometer. The measurement procedure can be performed, for example, as follows: First, an organic solvent (e.g., xylene) is added to 3 mg of the polymer compound to adjust the whole amount to 300 mg, thereby preparing a 1% by mass organic solvent solution of the polymer compound. Next, the solution prepared is diluted at predetermined ratios with an organic solvent (e.g., xylene) to prepare diluted solutions. Each solution thus prepared is placed in a 1 cm square quartz cell, and an optical spectrum is measured using a spectrophotometer. Thus, the wavelength of the absorption maximum of the polymer compound can be determined.

The fluorescence quantum yield of the polymer compound of the present embodiment is preferably less than 20%. The fluorescence quantum yield of the polymer compound is more preferably less than 10%, still more preferably less than 1%, and particularly preferably less than 0.1%, from the viewpoint of enhancing generation efficiency of a photoacoustic signal of the nanoparticles including the polymer compound.

The fluorescence quantum yield of the polymer compound can be measured, for example, by the following method: First, an aliquot of 1 mL of a solution containing the polymer compound is placed into a 10 mL volumetric flask, to which a solvent is then added until the meniscus is aligned with the marked line of the 10 mL volumetric flask. Next, using an apparatus for measuring the absolute PL quantum yield of the polymer compound solution prepared, the fluorescence quantum yield is measured at an excitation light wavelength of 794 nm at room temperature (25°C) in the atmosphere. Thus, the fluorescence quantum yield of the polymer compound can be determined.

(Amphiphilic Molecule)

Examples of the amphiphilic molecule of the present embodiment include a nonionic surfactant, anionic surfactant, cationic surfactant, polymeric surfactant, phospholipid, and polysaccharide. One type of these amphiphilic molecule may be used singly, or two or more types thereof may be used in combination. The amphiphilic molecule is preferably a surfactant or a phospholipid.

Examples of the nonionic surfactant include polyoxyethylene sorbitane fatty acid esters (e.g., a compound represented by Formula (20)). Examples of the polyoxyethylene sorbitane fatty acid ester include Tween 20, Tween 40, Tween 60, Tween 80, and Tween 85.

[Chemical Formula 40]

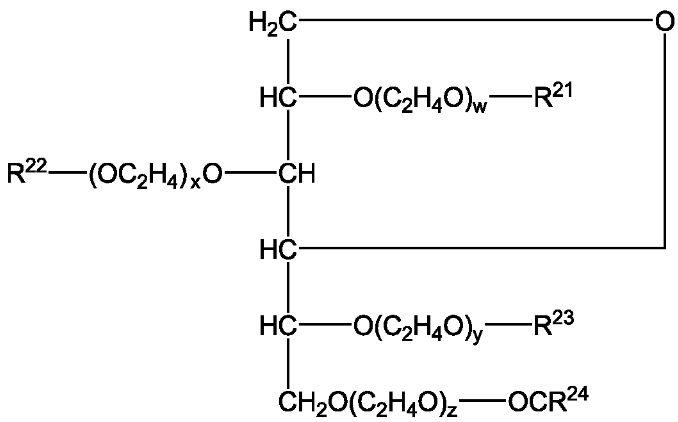

(20)

In Formula (20), $R^{21}$, $R^{22}$, $R^{23}$, and $R^{24}$ each independently represent a hydrogen atom or $-OCR^{20'}$. $R^{20'}$ represents a saturated or unsaturated alkyl group having 1 to 18 carbon atoms. w, x, y, and z represent integers such that the sum of w, x, y, and z is 10 to 30.

Examples of the anionic surfactant include dodecylsulfuric acid, dodecylbenzenesulfonate, decylbenzenesulfonate, undecylbenzenesulfonate, tridecylbenzenesulfonate, and nonylbenzenesulfonate, and sodium, potassium, or ammonium salts thereof; and sodium, potassium, or ammonium salts of myristic acid, palmitic acid, stearic acid, and oleic acid.

Examples of the cationic surfactant include cetyltrimethylammonium bromide, hexadecylpyridinium chloride, dodecyltrimethylammonium chloride, and hexadecyltrimethylammonium chloride.

Examples of the polymeric surfactant include polyvinyl alcohol, a polyoxyethylene polyoxypropylene block copolymer, and gelatin. Examples of the polyoxyethylene polyoxypropylene block copolymer include a compound represented by Formula (30).

[Chemical Formula 41]

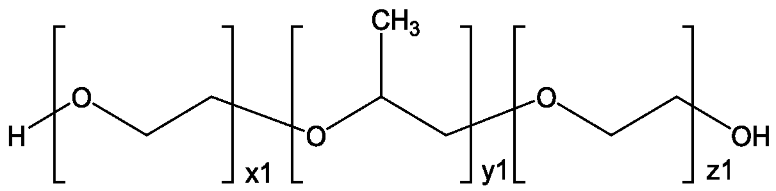

(30)

In Formula (30), x1 and z1 are each independently an integer of 70 to 110 and preferably an integer of 75 to 106. y1 is an integer of 20 to 80 and preferably an integer of 30 to 70.

The phospholipid is preferably a phosphatidyl phospholipid having a functional group of any of a hydroxyl group, a mercapto group, a methoxy group, an amino group, or a carboxyl group. The phospholipid may have a polyethylene glycol (PEG) chain.

Examples of the polysaccharide include dextran and heparin.

The amphiphilic molecule preferably has a group represented by Formula (5a) from the viewpoint of improving the shape stability of the nanoparticle.

[Chemical Formula 42]

$$-\left(Y^{31A}-R'\right)_{a3}-Y^{31}(R''^{2}) \qquad (5a)$$

In Formula (5a), a3 represents an integer of 0 or 1 or more. a3 is normally an integer of 0 to 20, preferably an integer of 1 to 20, and more preferably an integer of 2 to 10.

R' represents an alkylene group, a cycloalkylene group, or an arylene group, and these groups optionally have a substituent. In the case that a plurality of R's are present, they may be identical or different from each other. R' is preferably an alkylene group.

The number of carbon atoms of the alkylene group represented by R' is normally 1 to 10, preferably 2 to 10, and more preferably 2 to 6, excluding the number of carbon atoms of a substituent.

The number of carbon atoms of the cycloalkylene group represented by R' is normally 3 to 50, preferably 3 to 30, and more preferably 4 to 20, excluding the number of carbon atoms of a substituent.

Examples of the alkylene group represented by R' or the cycloalkylene group represented by R' include methylene, ethylene, 1,3-propylene, 1,2-butylene, 1,3-butylene, 1,4-butylene, and 1,4-cyclohexylene groups. These groups optionally have a substituent.

The number of carbon atoms of the arylene group represented by R' is normally 6 to 60, preferably 6 to 30, and more preferably 6 to 18, excluding the number of carbon atoms of a substituent.

Examples of the arylene group represented by R' include 1,3-phenylene, 1,4-phenylene, 1,4-naphthylene, and 2,6-naphthylene. These groups optionally have a substituent.

R' is preferably an alkylene group or an arylene group, and more preferably an alkylene group.

$R''^2$ represents a hydrogen atom, an alkyl group, a cycloalkyl group, or an aryl group, and these groups optionally have a substituent.

The number of carbon atoms of the alkyl group represented by $R''^2$ is normally 1 to 50, preferably 3 to 30, and more preferably 4 to 20, excluding the number of carbon atoms of a substituent.

Examples of the alkyl group represented by $R''^2$ include methyl, ethyl, propyl, and butyl groups. These groups optionally have a substituent.

The number of carbon atoms of the cycloalkyl group represented by $R''^2$ is normally 3 to 50, preferably 3 to 30, and more preferably 4 to 20, excluding the number of carbon atoms of a substituent.

Examples of the cycloalkyl group represented by $R''^2$ include cyclohexyl, cyclohexylmethyl, and cyclohexylethyl groups. These groups optionally have a substituent.

The number of carbon atoms of the aryl group represented by $R''^2$ is normally 6 to 60, preferably 6 to 20, and more preferably 6 to 10, excluding the number of carbon atoms of a substituent.

Examples of the aryl group represented by $R''^2$ include phenyl,1-naphthyl, 2-naphthyl, 1-anthracenyl, 2-anthracenyl, 9-anthracenyl, 1-pyrenyl, 2-pyrenyl, 4-pyrenyl, 2-fluorenyl, 3-fluorenyl, 4-fluorenyl, 2-phenylphenyl, 3-phenylphenyl, and 4-phenylphenyl groups. These groups optionally have a substituent.

$R''^2$ is preferably a hydrogen atom, an alkyl group, or a cycloalkyl group.

$Y^{31}$ represents -O-, -S-, -C(=O)O-, or -N(R"2)-. $Y^{31}$ is preferably -S-, C(=O)O-, or -NH-.

In the case that $Y^{31}$ is -O-, $R''^{2}$ is preferably a hydrogen atom or an alkyl group, and more preferably a hydrogen atom, a methyl group, or an ethyl group. In the case that $Y^{31}$ is -S-, C(=O)O-, or -N(R"2)-, $R''^{2}$ is preferably a hydrogen atom or an alkyl group, and more preferably a hydrogen atom.

$Y^{31A}$ represents -O- or -S-. In the case that a plurality of $Y^{31A}$s are present, they may be identical or different from each other. $Y^{31A}$ is preferably -O-.

Examples of the group represented by Formula (5a) include groups represented by Formulae (5-1) to (5-5). The group represented by Formula (5a) is preferably a group represented by Formula (5-3), (5-4), or (5-5) from the viewpoint of capability of binding to another molecule.

[Chemical Formula 43]

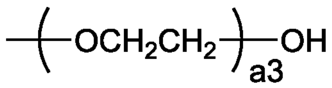

(5-1)

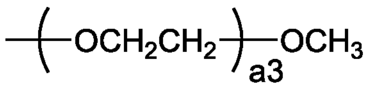

(5-2)

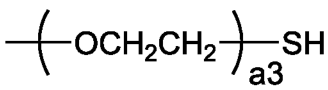

(5-3)

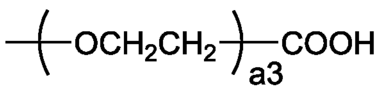

(5-4)

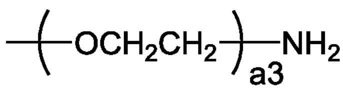

(5-5)

a3 has the same meaning as described above.

The amount (content) of the amphiphilic molecule of the present embodiment used is normally 10 to 10000 parts by mass, preferably 50 to 5000 parts by mass, and more preferably 10 to 1000 parts by mass, in the case that the amount of the polymer compound is 100 parts by mass.

(Method for Producing Nanoparticles)

As a method for producing the nanoparticles of the present embodiment, a known production method can be utilized. Examples of the production method include a Nanoemulsion method and a Nanoprecipitation method.

Examples of an organic solvent used in the production method of the present embodiment include hydrocarbons such as hexane, cyclohexane, and heptane; ketones such as acetone and methyl ethyl ketone; ethers such as diethyl ether and tetrahydrofuran; halogenated hydrocarbons such as dichloromethane, chloroform, carbon tetrachloride, dichloroethane, and trichloroethane; aromatic hydrocarbons such as benzene, toluene, and xylene; esters such as ethyl acetate and butyl acetate; aprotic polar solvents such as N,N-dimethylformamide and dimethyl sulfoxide; and pyridine derivatives.

In the Nanoemulsion method, an emulsion can be prepared by a conventionally known emulsification technique. Examples of the conventionally known emulsification technique include an intermittent shaking method, a stirring method using a mixer such as a propeller type stirrer or a turbine type stirrer, a colloid mill method, a homogenizer method, and an ultrasonic irradiation method. These emulsification techniques may be used singly or in combination of two or more thereof. The emulsion may be prepared by one-step emulsification or by multi-step emulsification. However, the emulsification technique is not limited to the above technique as long as the object of the present invention can be achieved.

In the Nanoprecipitation method, the nanoparticles can be prepared by a conventionally known method in which an organic solvent dispersion liquid containing the polymer compound is mixed with a dispersion aqueous solution containing the amphiphilic molecules and stirred, or a method in which a dispersion aqueous solution containing the amphiphilic molecules is mixed with an organic solvent dispersion liquid containing the polymer compound and stirred.

The mass ratio between the dispersion aqueous solution containing the amphiphilic molecules and the organic solvent dispersion liquid containing the polymer compound in the Nanoemulsion method is not particularly limited as long as an oil-in-water (O/W) type emulsion can be formed, but is preferably 1: 2 to 1 : 1000 in the ratio of the organic solvent dispersion liquid to the dispersion aqueous solution (organic solvent dispersion liquid : dispersion aqueous solution).

The mass ratio between the dispersion aqueous solution containing amphiphilic molecules and the organic solvent dispersion liquid containing the polymer compound in the Nanoprecipitation method is not particularly limited as long as the nanoparticles can be collected, but is preferably 1 : 2 to 1 : 1000 in the ratio of the organic solvent dispersion

liquid to the dispersion aqueous solution (organic solvent dispersion liquid : dispersion aqueous solution).

The content of the polymer compound in the organic solvent dispersion liquid containing the polymer compound is normally 0.01 to 20% by mass, preferably 0.1 to 5% by mass, and more preferably 0.4 to 2% by mass.

The temperature at the time of reacting the dispersion aqueous solution containing the amphiphilic molecules with the organic solvent dispersion liquid containing the polymer compound is normally 0 to 100°C, preferably 4 to 50°C, and more preferably 10 to 40°C.

The organic solvent is removed from the dispersion liquid containing the nanoparticles and the organic solvent, and thereby a dispersion liquid containing the nanoparticles can be obtained. Examples of a method for removing the organic solvent include a removal method by heating and a removal method using a decompression device such as an evaporator. In the Nanoemulsion method, the heating temperature in the removal method by heating is not particularly limited as long as the O/W type emulsion can be maintained, but is preferably a temperature of 0 to 80°C. In the Nanoprecipitation method, the heating temperature in the removal method by heating is not particularly limited as long as higher-order aggregation by which a yield of the nanoparticles decreases can be prevented, but is preferably a temperature of 0 to 80°C. However, the removal method by heating is not limited to the above method as long as the object of the present invention can be achieved.

Thus, a dispersion liquid containing the nanoparticles can be obtained. The obtained dispersion liquid containing the nanoparticles may be subjected to a purification operation. Examples of the purification operation include a size exclusion column chromatography method, an ultrafiltration method, a dialysis method, and a centrifugation method.

The particle diameter of the nanoparticles of the present embodiment is preferably 1 nm or more and less than 1000 nm. The particle diameter of the nanoparticles is more preferably 5 nm or more and less than 1000 nm, still more preferably 10 to 500 nm, and particularly preferably 20 to 250 nm, from the viewpoint of enhancing accumulation of the nanoparticles in tumor tissue by an enhanced permeation and retention (EPR) effect in in-vivo diagnosis. The particle diameter of the nanoparticles means an average particle diameter in a dynamic light scattering method.

The wavelength of the absorption maximum of the nanoparticles of the present embodiment is preferably 500 to 2000 nm. The wavelength of the absorption maximum of the nanoparticles is more preferably 700 to 1500 nm, and still more preferably 750 to 1300 nm, from the viewpoint of increasing the diagnostic depth in photoacoustic diagnosis.

The concentration of the polymer compound in the dispersion liquid containing the nanoparticles of the present embodiment is normally 0.1 to 100000 mg/L, preferably 1 to 10000 mg/L, and more preferably 10 to 1000 mg/L.

The concentration of the polymer compound in the dispersion liquid containing the nanoparticles can be measured by, for example, the following method: A dispersion medium is added to 1 mL of the dispersion liquid containing the nanoparticles until the whole amount reaches $\alpha$ mL. Then, an absorption maximum wavelength $\lambda$max' and an absorbance A at $\lambda$max' for the nanoparticle dispersion liquid prepared are measured using a spectrophotometer. The concentration c of the polymer compound in the dispersion liquid containing the nanoparticles can be calculated from the following Formula (1), based on Lambert-Beer law.

$$c = \alpha A/(\varepsilon l) \quad (1)$$

c represents a concentration of the polymer compound in the dispersion liquid.
$\alpha$ represents a dilution factor.
A represents an absorbance at wavelength $\lambda$max' of the absorption maximum of the polymer compound in the diluted dispersion liquid.
$\varepsilon$ represents a gram absorption coefficient of the polymer compound solution.
l represents an optical path length in spectrophotometric measurement.

The fluorescence quantum yield of the nanoparticles of the present embodiment is preferably less than 20%. The fluorescence quantum yield of the nanoparticles is more preferably less than 10%, still more preferably less than 1%, and particularly preferably less than 0.1%, from the viewpoint of enhancing generation efficiency of a photoacoustic signal of the nanoparticles.

The fluorescence quantum yield of the nanoparticles can be measured, for example, by the following method: First, an aliquot of 1 mL of the dispersion liquid containing the nanoparticles is placed into a 10 mL volumetric flask, to which a dispersion medium is then added until the meniscus is aligned with the marked line of the 10 mL volumetric flask. Then, using an apparatus for measuring the absolute PL quantum yield of the nanoparticles prepared, the fluorescence quantum yield is measured at an excitation light wavelength of 794 nm at room temperature (25°C) in the atmosphere. Thus, the fluorescence quantum yield of the nanoparticles can be determined.

In the nanoparticle of the present embodiment, it is preferable that a capture molecule is bound to at least one of the polymer compound or the amphiphilic molecule included in the nanoparticle. This makes it possible to specifically label a target site. Therefore, specific detection of the target site, and tracking of dynamics, localization, metabolism,

and the like of a target substance can be performed with such nanoparticles.

The capture molecule means a substance that specifically binds to a target site such as a tumor, a substance that specifically binds to a substance present around a target site, and the like. The capture molecule can be arbitrarily selected from biomolecules, chemical substances such as pharmaceuticals, and the like. The capture molecule is preferably at least one selected from the group consisting of an antibody, a sugar chain, an aptamer, a receptor, a peptide, a transport protein, and an enzyme. One type of the capture molecule may be used singly, or two or more types thereof may be used in combination.

The capture molecule is more preferably an antibody. Examples of the antibody include antibodies derived from vertebrates such as mouse, rat, cow, rabbit, goat, sheep, and guinea pig. Among these, the capture molecule is preferably an antibody derived from a mouse.

The isotype of the antibody is not particularly limited. Examples of the isotype include IgG (IgG1, IgG2, IgG3, IgG4), IgA, IgD, IgE, IgG, and IgM. Among these, the isotype of the antibody is preferably IgG or IgM.

The antibody is preferably a cancer antigen-specific antibody from the viewpoint of enhancing the accumulation in tumor tissue.

A method for binding the capture molecule to the nanoparticle is not particularly limited as long as the capture molecule can be chemically bound to the nanoparticle, and a known method can be adopted. Examples of the method for binding the capture molecule to the nanoparticle include a method of chemically binding the terminal functional group of the amphiphilic molecule included in the nanoparticle and the functional group contained in the capture molecule.

Since the nanoparticle of the present embodiment is excellent in generation efficiency of a photoacoustic signal, it can be used as a photoacoustic contrast agent, a photodynamic therapy drug, or a photothermal therapy drug.

(Nanoparticle Including Encapsulant)

The nanoparticle of the present embodiment may include an encapsulant in addition to the polymer compound having the constitutional unit represented by Formula (1) and at least one amphiphilic molecule.

The encapsulant may form a covalent bond with the polymer compound or the amphiphilic molecule, or may be mixed without forming the covalent bond.

When the encapsulant is mixed without forming a covalent bond with the polymer compound or the amphiphilic molecule, the polymer compound and at least one amphiphilic molecule may be completely or partially incorporated into the encapsulant. In this case, the polymer compound and the amphiphilic molecule may be uniformly or non-uniformly distributed in the encapsulant.

The encapsulant may have a core portion and one or more shell layers surrounding the core portion. The core portion may contain the polymer compound and the amphiphilic molecule. The core portion may also contain one or more additional materials, for example, a light absorbing material, and the light absorbing material may form a covalent bond with the encapsulant.

The core portion in the encapsulant may be covered by forming an outer shell. Thereby, the core portion can be completely or partially isolated from the surrounding environment, and the nanoparticle including the encapsulant can reduce the influence of the external environment on the stability or light absorption characteristics of the polymer compound.

In the nanoparticle including the encapsulant, any chemical interaction between a cell tissue or an endogenous substance and the polymer compound or the amphiphilic molecule may be limited from the viewpoint of use as a photoacoustic contrast agent in PAI.

Examples of a material used for the encapsulant include an organic polymer compound and an inorganic polymer compound.

Examples of the organic polymer compound include various polymers, polypeptides, and derivatives thereof, copolymers having two or more types of repeating units, and organic dendrimer compounds. However, the organic polymer compound excludes a polymer compound having the constitutional unit represented by Formula (1). The above organic polymer compounds may be used singly or in combination of two or more thereof.

Examples of the inorganic polymer compound include a composite containing at least one of silica, alumina, or silica and alumina as a main component. These inorganic polymer compounds may be used singly or in combination of two or more thereof.

The material used for the encapsulant is not limited to the organic polymer compound and the inorganic polymer compound, and for example, a low molecular lipid compound, an organic-inorganic hybrid compound, or a mixture of an organic compound and an inorganic compound can also be used.

The material used for the encapsulant is preferably the inorganic polymer compound, more preferably silica, from the viewpoint of improving the shape stability of the nanoparticle.

(Method for Producing Nanoparticles Including Encapsulant)

The nanoparticles including the encapsulant can be formed by, for example, polymerizing a silica monomer in the presence of the polymer compound or the amphiphilic molecule. The silica monomer is preferably soluble in a protic solvent, such as alcohol, water, or a mixture thereof. The nanoparticles including the encapsulant can also be formed according to, for example, the methods described in WO 2018/060722 and references therein.

The particle diameter of the nanoparticles including the encapsulant is preferably 1 nm or more and less than 1000 nm. The particle diameter of the nanoparticles including the encapsulant is more preferably 5 nm or more and less than 1000 nm, still more preferably 10 to 500 nm, and particularly preferably 20 to 250 nm, from the viewpoint of enhancing accumulation of the nanoparticles in tumor tissue by an enhanced permeation and retention (EPR) effect in in-vivo diagnosis. The method for measuring the particle diameter of the nanoparticles including the encapsulant is the same measurement method as the method for measuring the wavelength of the absorption maximum of the nanoparticles of the first embodiment.

The nanoparticle including the encapsulant may have a surface-modifying group. As the surface-modifying group in the nanoparticle including the encapsulant, any group can be selected from the viewpoint of uniform dispersibility of the nanoparticles or binding ability of the capture molecule.

A method for binding the capture molecule to the nanoparticle including the encapsulant is not particularly limited as long as the capture molecule can be chemically bound to the nanoparticle, and a known method can be adopted. Examples of the method for binding the capture molecule to the nanoparticle including the encapsulant include a method of chemically binding the surface-modifying group contained in the nanoparticle and the functional group contained in the capture molecule.

<Second Embodiment>

A nanoparticle of the second embodiment includes a polymer compound having a group represented by Formula (5) and having a constitutional unit represented by Formula (1A) .

Note that each symbol in the nanoparticle of the second embodiment has the same meaning as each symbol in the nanoparticle of the first embodiment. The preferred range and the like of each symbol in the nanoparticle of the second embodiment are also the same as the preferred range and the like of each symbol in the nanoparticle of the first embodiment. Therefore, in the second embodiment, description of parts overlapping with the first embodiment may be omitted.

(Polymer Compound)

The polymer compound has a group represented by Formula (5).

[Chemical Formula 44]

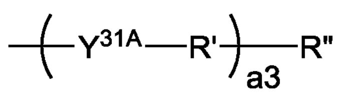

(5)

In Formula (5), a3, R' and $Y^{31A}$ have the same meanings as a3, R' and $Y^{31A}$ in the group represented by Formula (5a) of the amphiphilic molecule of the first embodiment.

R' ' represents $-Y^{31}(R''^{2})$ or $-Y^{31}(M)$. $Y^{31}$ and $R''^{2}$ have the same meanings as $Y^{31}$ and $R''^{2}$ in the group represented by Formula (5a) of the amphiphilic molecule of the first embodiment.

M represents an alkali metal cation or a quaternary ammonium. Examples of the alkali metal cation include $Li^+$, $Na^+$, $K^+$, $Rb^+$, and $Cs^+$. Examples of the quaternary ammonium include tetramethylammonium, tetraethylammonium, and tetrabutylammonium.

The group represented by Formula (5) is preferably a group represented by Formula (5a) from the viewpoint of improving the shape stability of the nanoparticle. The group represented by Formula (5) is the same as the group represented by Formula (5a) of the amphiphilic molecule of the first embodiment.

[Chemical Formula 45]

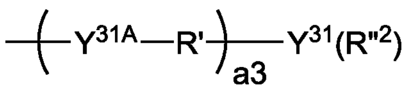

(5a)

The polymer compound has a constitutional unit represented by Formula (1A).

[Chemical Formula 46]

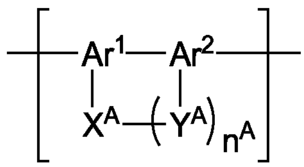

(1A)

In Formula (1A), $Ar^1$ and $Ar^2$ have the same meanings as $Ar^1$ and $Ar^2$ in the constitutional unit represented by Formula (1) of the polymer compound of the first embodiment.

$X^A$ and $Y^A$ each independently represent -O-, -S-, - C(=O)-, -S(=O)-, $-SO_2-$, $-CR^A{}_2-$, $-SiR^A{}_2-$, $-NR^A-$, $-BR^A-$, $-PR^A-$, or $-P(=O)(R^A)-$. Here, $R^A$ represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, a heteroaryloxycarbonyl group, or the group represented by Formula (5), and a group other than the group represented by Formula (5) may optionally have the group represented by Formula (5) or a substituent. In the case that a plurality of $R^A$s are present, they may be identical or different from each other.

Alkyl, alkenyl, alkynyl, alkyloxy, aryl, aryloxy, arylalkyl, acyl, acyloxy, amide, amino, monovalent aromatic heterocyclic, heteroaryloxy, heteroarylthio, alkyloxycarbonyl, aryloxycarbonyl, arylalkyloxycarbonyl, and heteroaryloxycarbonyl groups each represented by $R^A$ have the same meanings as groups each represented by R, respectively. The above groups may be such that some of hydrogen atoms thereof are optionally substituted with the group represented by Formula (5) or a substituent, or may optionally have the group represented by Formula (5) or a substituent.

$X^A$ is preferably -C(=O)- or $-CR^A{}_2-$, and more preferably $-CR^A{}_2-$, from the viewpoint of ease of production of a monomer to be a raw material of the polymer compound. $R^A$ in $X^A$ is preferably the group represented by Formula (5), an alkyl group optionally having the group represented by Formula (5), or an aryl group optionally having the group represented by Formula (5) from the viewpoint of ease of production of a monomer to be a raw material of the polymer compound.

$Y^A$ is preferably -O-, -S-, -C(=O)-, $-CR^A{}_2-$, or -NR-, more preferably -O-, -C(=O)-, or $-CR^A{}_2-$, and still more preferably -O-, from the viewpoint of ease of production of a monomer to be a raw material of the polymer compound. $R^A$ in $Y^A$ is preferably the group represented by Formula (5), an alkyl group optionally having the group represented by Formula (5), or an aryl group optionally having the group represented by Formula (5) from the viewpoint of ease of production of a monomer to be a raw material of the polymer compound.

$n^A$ represents an integer of 1 or more. In the case that $n^A$ is 2 or more, a plurality of $Y^A$s may be identical or different from each other. $n^A$ is preferably 1 from the viewpoint of ease of production of a monomer to be a raw material of the polymer compound.

The constitutional unit represented by Formula (1A) is preferably a constitutional unit represented by Formula (1A').

[Chemical Formula 47]

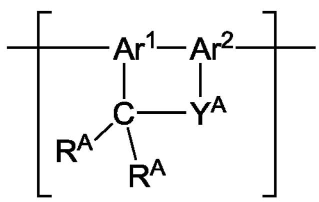

(1A')

In Formula (1A'), $Ar^1$, $Ar^2$, $Y^A$, and $R^A$ have the same meanings as described above.

The constitutional unit represented by Formula (1A) is preferably a constitutional unit represented by Formula (1Aa).

[Chemical Formula 48]

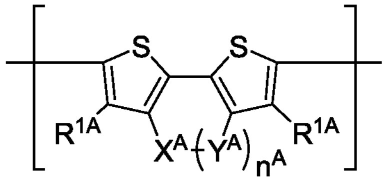

(1Aa)

In Formula (1a), $X^A$, $Y^A$, and $n^A$ have the same meanings as described above. $n^A$ is preferably 1 from the viewpoint of ease of production of a monomer to be a raw material of the polymer compound.

$R^{1A}$ represents a hydrogen atom, the group represented by Formula (5), or an alkyl group optionally having the group represented by Formula (5) or a substituent. The alkyl group represented by $R^1$ has the same meaning as the alkyl group represented by R. $R^{1A}$ is preferably a hydrogen atom or an alkyl group optionally having a substituent.

The constitutional unit represented by Formula (1A) is preferably a constitutional unit represented by Formula (1Ab).

[Chemical Formula 49]

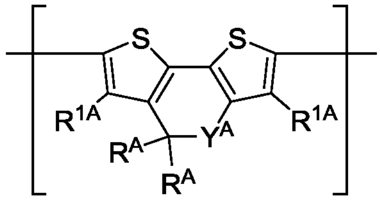

(1Ab)

In Formula (1Ab), $R^A$, $R^{1A}$ and $Y^A$ have the same meanings as described above. $Y^A$ is preferably -O-. $R^A$ is preferably the group represented by Formula (5), an alkyl group optionally having the group represented by Formula (5), or an aryl group optionally having the group represented by Formula (5) from the viewpoint of ease of production of a monomer to be a raw material of the polymer compound.

The polymer compound further has a constitutional unit represented by Formula (3). The constitutional unit represented by Formula (3) is the same as the constitutional unit represented by Formula (3) of the polymer compound of the first embodiment.

[Chemical Formula 50]

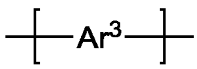

(3)

The constitutional unit represented by Formula (3) preferably includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e). The constitutional unit represented by Formula (2a), (2b), (2c), (2d), or (2e) is the same as the constitutional unit represented by Formula (2a), (2b), (2c), (2d), or (2e) of the polymer compound of the first embodiment.

[Chemical Formula 51]

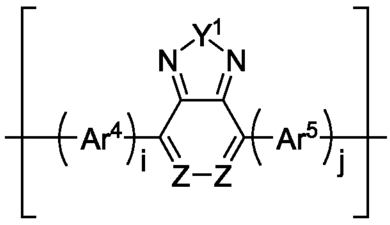

(2a)

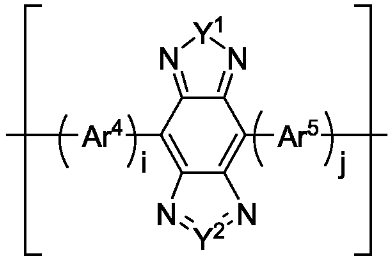

(2b)

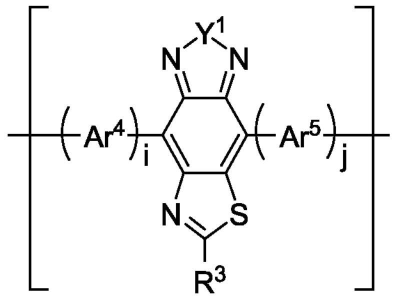

(2c)

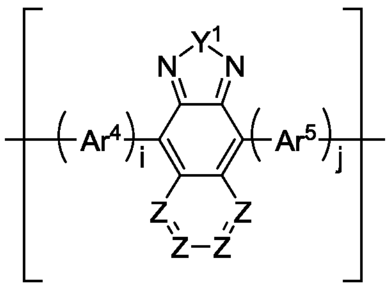

(2d)

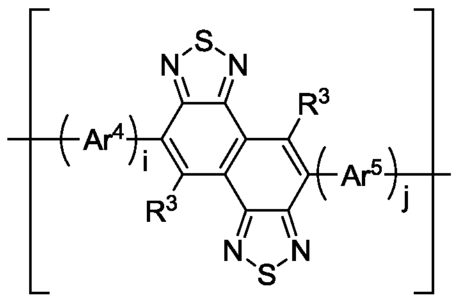

(2e)

In the case that the polymer compound contains the constitutional unit represented by Formula (1A) and the constitutional unit represented by Formula (3), the content of the constitutional unit represented by Formula (1A) is preferably 20 to 80% by mass, and the content of the constitutional unit represented by Formula (3) is preferably 20 to 80% by mass, based on all the constitutional units of the polymer compound.

The polymer compound of the present embodiment is generally a compound having a weight-average molecular weight of 1000 or more. The weight-average molecular weight of the polymer compound is preferably 3000 to 10000000, more preferably 8000 to 5000000, and still more preferably 10000 to 1000000. If the weight-average molecular weight is less than 3000, nanoparticle aggregation or deterioration of photoacoustic characteristics of the nanoparticles may occur, and if the weight-average molecular weight is more than 10000000, a yield of the nanoparticles may decrease.

The polymer compound of the present embodiment desirably has high solubility in a solvent for use in the nanoparticles from the viewpoint of ease of nanoparticle preparation. Specifically, the polymer compound of the present embodiment preferably has solubility such that a solution containing 0.01% by mass or more of the polymer compound can be prepared, more preferably has solubility such that a solution containing 0.1% by mass or more of the polymer compound can be prepared, and still more preferably has solubility such that a solution containing 0.4% by mass or more of the polymer compound can be prepared.

The polymer compound of the second embodiment can be produced by the same method as the production method exemplified by the polymer compound of the first embodiment.

The number-average molecular weight of the polymer compound of the present embodiment is preferably 1000 to 100000000. If the number-average molecular weight is less than 1000, nanoparticle aggregation or deterioration of photoacoustic characteristics of the nanoparticles may occur, and if the weight-average molecular weight is more than 100000000, a yield of the nanoparticles may decrease.

The end group of the polymer compound of the present embodiment may be protected with a stable group because nanoparticle aggregation or deterioration of photoacoustic characteristics of the nanoparticles may occur if the polymerizable active group remains. The stable group is preferably a group having a conjugated bond continuous with the conjugated structure of the main chain. In addition, the stable group may be, for example, a group having a structure bound to an aryl group or a heterocyclic group via a vinylene group.

A monomer to be a raw material of the polymer compound of the present embodiment can be synthesized, for example, according to the description of WO 2011/052709 A.

The wavelength of the absorption maximum of the polymer compound of the present embodiment is preferably

500 to 2000 nm. The wavelength of the absorption maximum of the polymer compound is more preferably 700 to 1500 nm, and still more preferably 750 to 1300 nm, from the viewpoint of enhancing the transmittance to an organism in photoacoustic imaging of the nanoparticles including the polymer compound. The method for measuring the wavelength of the absorption maximum of the polymer compound is the same as the method for measuring the wavelength of the absorption maximum of the polymer compound of the first embodiment.

The fluorescence quantum yield of the polymer compound of the present embodiment is preferably less than 20%. The fluorescence quantum yield of the polymer compound is more preferably less than 10%, still more preferably less than 1%, and particularly preferably less than 0.1%, from the viewpoint of enhancing generation efficiency of a photoacoustic signal of the nanoparticles including the polymer compound. The measurement method for the fluorescence quantum yield of the polymer compound is the same as that for the fluorescence quantum yield of the polymer compound of the first embodiment.

(Method for Producing Nanoparticles)

The polymer compound of the present embodiment has the group represented by Formula (5). Since the group represented by Formula (5) is a group exhibiting hydrophilicity, the nanoparticles can be produced without including the amphiphilic molecule. The nanoparticles can be produced in the same manner as in the first embodiment except that, for example, the amphiphilic molecule is not used.

The particle diameter of the nanoparticles of the present embodiment is preferably 1 nm or more and less than 1000 nm. The particle diameter of the nanoparticles is more preferably 5 nm or more and less than 1000 nm, still more preferably 10 to 500 nm, and particularly preferably 20 to 250 nm, from the viewpoint of enhancing accumulation of the nanoparticles in tumor tissue by an enhanced permeation and retention (EPR) effect in in-vivo diagnosis. The method for measuring the particle diameter of the nanoparticles is the same as the method for measuring the wavelength of the absorption maximum of the nanoparticles of the first embodiment.

The wavelength of the absorption maximum of the nanoparticles of the present embodiment is preferably 500 to 2000 nm. The wavelength of the absorption maximum of the nanoparticles is more preferably 700 to 1500 nm, and still more preferably 750 to 1300 nm, from the viewpoint of increasing the diagnostic depth in photoacoustic diagnosis. The method for measuring the wavelength of the absorption maximum of the nanoparticles is the same as the method for measuring the wavelength of the absorption maximum of the nanoparticles of the first embodiment.

The concentration of the polymer compound in the dispersion liquid containing the nanoparticles of the present embodiment is normally 0.1 to 100000 mg/L, preferably 1 to 10000 mg/L, and more preferably 10 to 1000 mg/L. The method for measuring the concentration of the polymer compound in the dispersion liquid containing the nanoparticles is the same as the method for measuring the concentration of the polymer compound in the dispersion liquid containing the nanoparticles of the first embodiment.

The fluorescence quantum yield of the nanoparticles of the present embodiment is preferably less than 20%. The fluorescence quantum yield of the nanoparticles is more preferably less than 10%, still more preferably less than 1%, and particularly preferably less than 0.1%, from the viewpoint of enhancing generation efficiency of a photoacoustic signal of the nanoparticles. A method for measuring the fluorescence quantum yield of the nanoparticles is the same as the method for measuring the wavelength of the absorption maximum of the nanoparticles of the first embodiment.

In the nanoparticle of the present embodiment, it is preferable that a capture molecule is bound to the polymer compound included in the nanoparticle. This makes it possible to specifically label a target site. Therefore, specific detection of the target site, and tracking of dynamics, localization, metabolism, and the like of a target substance can be performed with such nanoparticles. The capture molecule is the same as the capture molecule in the first embodiment.

A method for binding the capture molecule to the nanoparticle is not particularly limited as long as the capture molecule can be chemically bound to the nanoparticle, and a known method can be adopted. Examples of the method for binding the capture molecule to the nanoparticle include a method of chemically binding a terminal functional group of a polymer compound included in the nanoparticle and a functional group contained in the capture molecule.

Since the nanoparticle of the present embodiment is excellent in generation efficiency of a photoacoustic signal, it can be used as a photoacoustic contrast agent, a photodynamic therapy drug, or a photothermal therapy drug.

(Nanoparticle Including Encapsulant)

The nanoparticle of the present embodiment may include an encapsulant in addition to the polymer compound having the group represented by Formula (5) and having the constitutional unit represented by Formula (1A).

The encapsulant may form a covalent bond with the polymer compound, or may be mixed without forming the covalent bond.

In the case that the encapsulant is mixed without forming a covalent bond with the polymer compound, the polymer compound may be completely or partially incorporated into the encapsulant. In this case, the polymer compound

may be uniformly or non-uniformly distributed in the encapsulant.

The encapsulant may have a core portion and one or more shell layers surrounding the core portion. The core portion may contain the polymer compound. The core portion may also contain one or more additional materials, for example, a light absorbing material, and the light absorbing material may covalently bond with the encapsulant.

The core portion in the encapsulant may be covered by forming an outer shell. Thereby, the core portion can be completely or partially isolated from the surrounding environment, and the nanoparticle including the encapsulant can reduce the influence of the external environment on the stability or light absorption characteristics of the polymer compound.

In the nanoparticle including the encapsulant, any chemical interaction between a cell tissue or an endogenous substance and the polymer compound may be limited from the viewpoint of use as a photoacoustic contrast agent in PAI.

A material used for the encapsulant is the same as the material used for the encapsulant of the nanoparticle including the encapsulant of the first embodiment.

(Method for Producing Nanoparticles Including Encapsulant)

The nanoparticles of the present embodiment including the encapsulant can be formed by, for example, polymerizing a silica monomer in the presence of the polymer compound. The silica monomer is preferably soluble in a protic solvent, such as alcohol, water, or a mixture thereof. The nanoparticles including the encapsulant can also be formed according to, for example, the methods described in WO 2018/060722 and references therein.

The particle diameter of the nanoparticles including the encapsulant is preferably 1 nm or more and less than 1000 nm. The particle diameter of the nanoparticles including the encapsulant is more preferably 5 nm or more and less than 1000 nm, still more preferably 10 to 500 nm, and particularly preferably 20 to 250 nm, from the viewpoint of enhancing accumulation of the nanoparticles in tumor tissue by an enhanced permeation and retention (EPR) effect in in-vivo diagnosis. The method for measuring the particle diameter of the nanoparticles including the encapsulant is the same measurement method as the method for measuring the wavelength of the absorption maximum of the nanoparticles of the first embodiment.

The nanoparticle including the encapsulant may have a surface-modifying group. As the surface-modifying group in the nanoparticle including the encapsulant, any group can be selected from the viewpoint of uniform dispersibility of the nanoparticles or binding ability of the capture molecule.

A method for binding the capture molecule to the nanoparticle including the encapsulant is not particularly limited as long as the capture molecule can be chemically bound to the nanoparticle, and a known method can be adopted. Examples of the method for binding the capture molecule to the nanoparticle including the encapsulant include a method of chemically binding the surface-modifying group contained in the nanoparticle and the functional group contained in the capture molecule.

[Photoacoustic Contrast Agent]

The photoacoustic contrast agent of the present embodiment contains the nanoparticles described above (nanoparticles of the first embodiment or nanoparticles of the second embodiment). The photoacoustic contrast agent may contain a dispersion medium in addition to the nanoparticles. The photoacoustic contrast agent may further contain a pharmacologically acceptable additive.

The dispersion medium is a liquid substance for dispersing nanoparticles. Examples of the dispersion medium include physiological saline, distilled water for injection, and a phosphate buffer. As the photoacoustic contrast agent of the present embodiment, one in which nanoparticles are dispersed in the dispersion medium in advance may be used, or a kit may be used, in which the nanoparticles and the dispersion medium is included, and the nanoparticles are dispersed in the dispersion medium before administered into an organism.

In the nanoparticles of the present embodiment, a predetermined polymer compound hardly leaks, whereby a large amount of the predetermined polymer compound is included in the particles. As described above, since the predetermined polymer compound is useful as a light absorber of a photoacoustic probe, the nanoparticles of the present embodiment can be suitably used for photoacoustic imaging (PAI).

An imaging method for detecting the nanoparticles of the present embodiment administered into an organism using PAI will be described below. The imaging method of the present embodiment includes, for example, the following steps. However, the imaging method of the present embodiment may include a step other than the following steps.

Step (a): a step of administering the nanoparticles of the present embodiment into an organism.

Step (b): a step of applying light to the organism to detect a photoacoustic signal emitted from the inside of the organism.

In step (a), a method for administering the nanoparticles of the present embodiment into an organism is not particularly limited and may be oral administration or parenteral administration. Additionally, PAI and fluorescence imaging

can be used in combination by, for example, binding or adding a fluorescent dye to the nanoparticles.

In step (b), the wavelength of light applied to the organism is normally 500 to 2000 nm, preferably 700 to 1500 nm, and more preferably 750 to 1300 nm.

The imaging method using the nanoparticles of the present embodiment can image a site such as a cancer or tumor through the steps of step (a) and step (b).

[Photodynamic Therapy Drug]

The nanoparticles of the present embodiment can also be suitably used as a photodynamic therapy (PDT) drug. The PDT is a treatment technique in which a light absorber is encouraged to accumulate at a site such as a tumor, and a reaction caused by applying light to the site is utilized.

The photodynamic therapy drug of the present embodiment contains the nanoparticles described above (nanoparticles of the first embodiment or nanoparticles of the second embodiment). The photodynamic therapy drug may contain a dispersion medium in addition to the nanoparticles. Examples of the dispersion medium include the dispersion medium in the photoacoustic contrast agent. The photodynamic therapy drug may further contain a pharmacologically acceptable additive. The photodynamic therapy drug may be used in combination with a PDT agent other than the nanoparticles because the PDT is made more effective.

Examples of the PDT agent other than the nanoparticles include Photofrin, Visudyne, and Foscan.

[Chemical Formula 52]

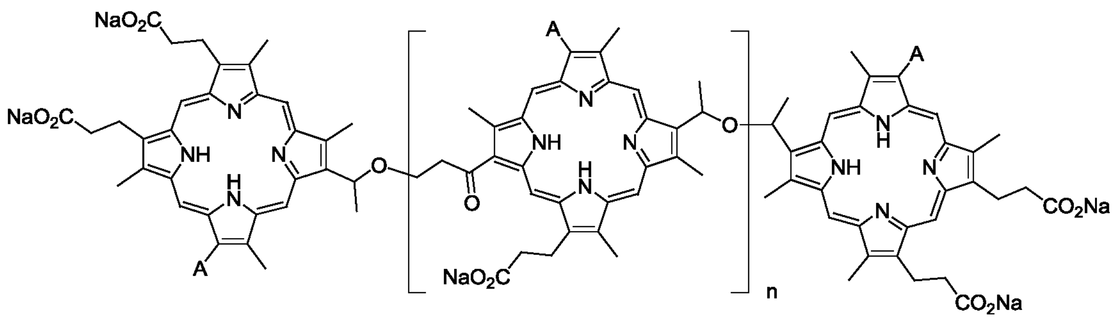

Photofrin

In the formulae, A represents-$CH(OH)CH_3$ or -$CH=CH_2$, and n represents an integer of 0 to 6.

[Chemical Formula 53]

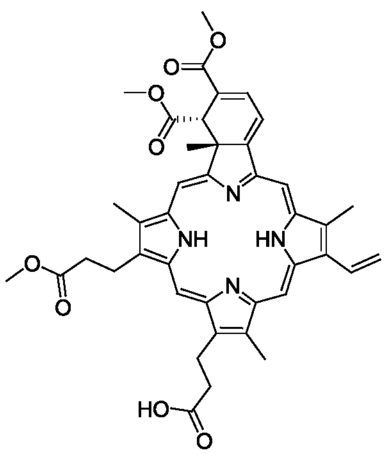

Visudyne

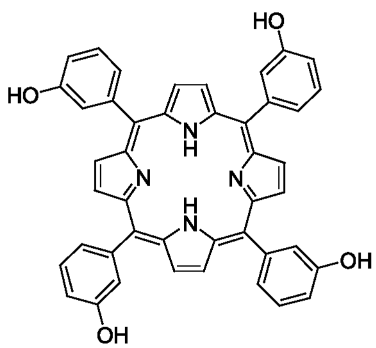

Foscan

Regarding a method of treatment using the nanoparticles (usage of the nanoparticles) of the present embodiment administered into an organism as a PDT, the method of treatment (usage) of the present embodiment includes, for example, the following steps. However, the method of treatment (usage) of the present embodiment may include a step other than the following steps.

Step (c): a step of administering the nanoparticles of the present embodiment into an organism.

Step (d): a step of applying light to the organism, which allows active oxygen (singlet oxygen) to be emitted in the organism, thereby destroying cancer cells or malignant tumor tissue.

In step (c), a method for administering the nanoparticles of the present embodiment into an organism is not particularly limited and may be oral administration or parenteral administration. Additionally, fluorescence imaging can be used in combination by, for example, binding or adding a fluorescent dye to the nanoparticles.

In step (d), the wavelength of light applied to the organism is normally 500 to 2000 nm, preferably 700 to 1500 nm, and more preferably 750 to 1300 nm.

The method of treatment using the nanoparticles (usage of the nanoparticles) of the present embodiment can treat a site such as a cancer or tumor through the steps of step (c) and step (d).

[Photothermal Therapy Drug]

The nanoparticles of the present embodiment can also be suitably used as a photothermal therapy (PTT) drug. The PTT is a treatment technique in which photothermogenic effect around cancer cells is utilized in order to kill only cancer cells relatively vulnerable to heat as compared with normal cells.

The photothermal therapy drug of the present embodiment contains the nanoparticles described above (nanoparticles of the first embodiment or nanoparticles of the second embodiment). The photothermal therapy drug may contain a dispersion medium in addition to the nanoparticles. Examples of the dispersion medium include the dispersion medium in the photoacoustic contrast agent. The photothermal therapy drug may further contain a pharmacologically acceptable additive. The photothermal therapy drug may be used in combination with, for example, a PDT agent other than the nanoparticles. Examples of the PDT agent other than the nanoparticles include the PDT agents other than nanoparticles in the photodynamic therapy drug.

Regarding a method of treatment using the nanoparticles (usage of the nanoparticles) of the present embodiment administered into an organism as a PTT, the method of treatment (usage) of the present embodiment includes, for example, the following steps. However, the method of treatment (usage) of the present embodiment may include a step other than the following steps.

Step (e): a step of administering the nanoparticles of the present embodiment into an organism.

Step (f): a step of applying light to the organism, which allows heat to be emitted in the organism, thereby destroying cancer cells or malignant tumor tissue.

In step (e), a method for administering the nanoparticles of the present embodiment into an organism is not particularly limited and may be oral administration or parenteral administration. Additionally, fluorescence imaging can be used in combination by, for example, binding or adding a fluorescent dye to the nanoparticles.

In step (f), the wavelength of light applied to the organism is normally 500 to 2000 nm, preferably 700 to 1500 nm, and more preferably 750 to 1300 nm.

The method of treatment using the nanoparticles (usage of the nanoparticles) of the present embodiment can treat a site such as a cancer or tumor through the steps of step (e) and step (f).

With the nanoparticles of the present embodiment, it is possible to simultaneously perform two or more of imaging with the photoacoustic contrast agent, the treatment by a photodynamic therapy (PDT), and the treatment by a photothermal therapy (PTT)

EXAMPLES

Hereinafter, the present invention will be described in more detail with Examples, but the present invention is not limited to these Examples.

<Method for Measuring NMR>

NMR was measured by the following method: 10 mg of a sample for the measurement was dissolved in 1 mL of deuterated chloroform ($CDCl_3$), and the measurement was performed using an NMR apparatus (manufactured by Agilent Technologies, Inc., trade name: INOVA 300; or manufactured by JEOL RESONANCE Inc., trade name: JNM-ECZ400S/L1).

<Method for Measuring Weight-average Molecular Weight (Mw) >

The obtained polymer compound was analyzed by GPC under the following conditions for the analysis, and the weight-average molecular weight (Mw) in terms of polystyrene was calculated from the analytical result.

(GPC Conditions for Analysis)

GPC measuring apparatus: CTO-10AC (a column oven manufactured by Shimadzu Corporation), SPD-10A (a detector manufactured by Shimadzu Corporation)
Column: Shodex KD-806 8.0 mm (diameter) × 30 cm (manufactured by Showa Denko K.K.)
Column temperature: 60°C
Mobile phase: o-dichlorobenzene
Flow rate: 1 mL/min
Detection: visible light detection (600 nm wavelength)

1. Synthesis of Polymer Compound

1-1. Synthesis of Polymer Compound 1 and Polymer Compound 2

Polymer compound 1 and polymer compound 2 were synthesized according to the method of WO 2011/052709 A.

[Chemical Formula 54]

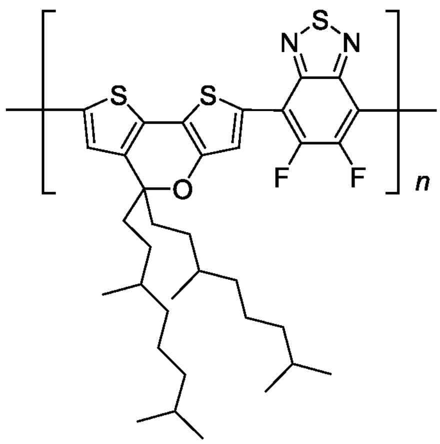

Polymer compound 1

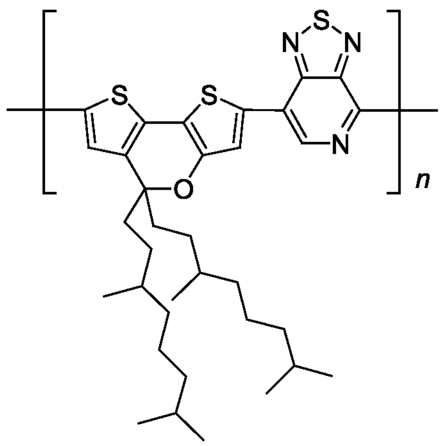

Polymer compound 2

1-2. Synthesis of Polymer Compound 3

Polymer compound 3 was synthesized according to the following synthetic route.

[Chemical Formula 55]

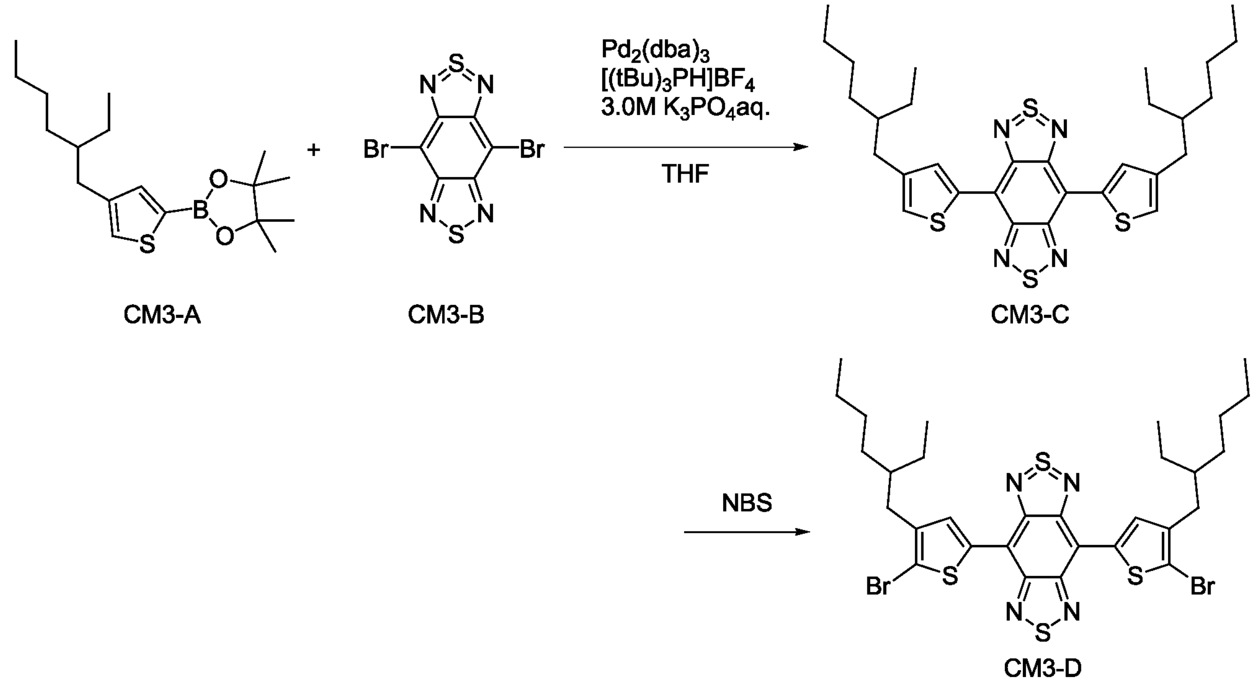


CM3-A CM3-B CM3-C

CM3-D

[Chemical Formula 56]

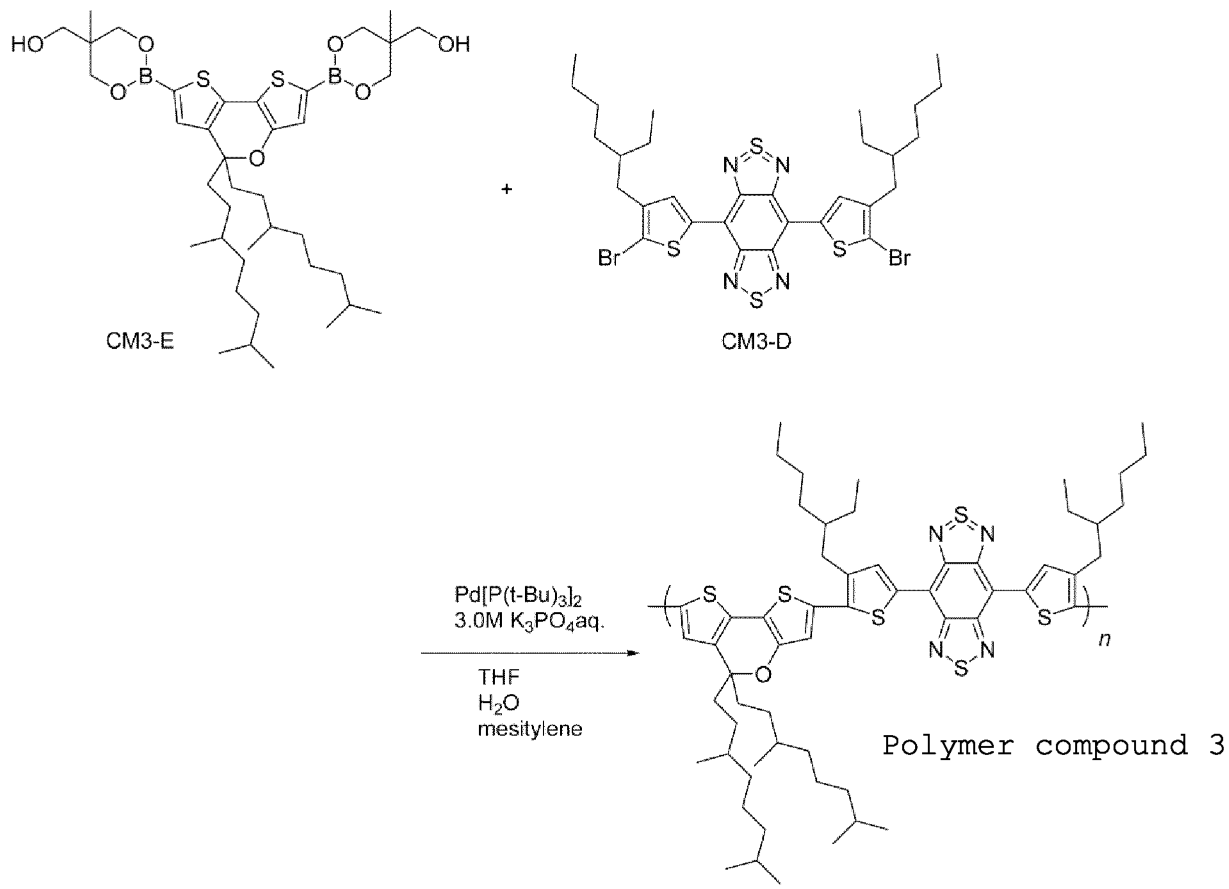


CM3-E CM3-D

Polymer compound 3

1-2(1). Synthesis Example 1 (Synthesis of Compound CM3-C)

The inside of a reaction vessel was brought into an inert gas atmosphere, to which 0.86 g of CM3-A (manufactured by Tokyo Chemical Industry Co., Ltd.), 1.66 g of CM3-B (manufactured by Luminescence Technology Corp.), 0.29 g of tris(dibenzylideneacetone)dipalladium(0), 0.30 g of tert-butylphosphonium tetrafluoroborate, 52 mL of tetrahydrofuran, and 8.2 mL of a 3.0 M aqueous potassium phosphate solution were then added, and the mixture was stirred at 50°C for 2 hours. The obtained reaction product was cooled to room temperature (25°C), to which toluene was then added, and subsequently washed with ion-exchanged water. The obtained organic layer was concentrated under reduced pressure to obtain a crude product. The crude product was washed with methanol, thus obtaining compound CM3-C (0.13 g, 9% yield) as a green solid.

1H

NMR($CDCl_3$,400MHz):$\delta$(ppm)=8.82(d,2H),7.27(d,2H),2.71(d,4H),1 .81-1.76(m,2H),1.41-1.28(m,16H),0.95-0.88(m,12H).

1-2(2). Synthesis Example 2 (Synthesis of Compound CM3-D)

The inside of a reaction vessel was brought into an inert gas atmosphere, in which 86 mg of CM3-C was then dissolved in 26 mL of chloroform and 26 mL of acetic acid, and the solution was cooled to 0°C, to which a solution of 53 mg of N-bromosuccinimide, 6.5 mL of chloroform, and 6.5 mL of acetic acid was then added dropwise. The reaction mixture was stirred at 0°C for 3 hours, 26 mL of chloroform was then added thereto, and the mixture was washed with ion-exchanged water. The obtained organic layer was concentrated under reduced pressure to obtain a crude product. The crude product was washed with acetonitrile, thus obtaining compound CM3-D (129 mg, 86% yield) as a green solid.

1H

NMR($CDCl_3$,400MHz):$\delta$(ppm)=8.69(s,2H),2.65(d,4H),1.82-1.74(m,2H),1.43-1.24(m,16H),0.97-0.87(m,12H).

1-2(3). Synthesis Example 3 (Synthesis of Polymer Compound 3)

The inside of a reaction vessel was brought into an inert gas atmosphere, in which 50 mg of compound CM-3D and 52 mg of compound CM3-E were then dissolved in 1.3 mL of mesitylene, 3.9 mL of ion-exchanged water, and 2.0 mL of tetrahydrofuran. To the solution, 3.5 mg of bis(tri-t-butylphosphine)palladium(0), 0.9 mL of tetrahydrofuran, and 0.2 mL of a 3.0 M aqueous potassium phosphate solution were added, and then the mixture was reacted at 70°C for 2 hours. To the reaction product, 1.3 mL of mesitylene was added, and the mixture was washed with 10% acetic acid and water. The obtained organic layer was concentrated under reduced pressure to obtain a crude product. The crude product was redissolved in 6 mL of mesitylene, and then the solution was added dropwise to 35 mL of methanol with stirring. The obtained precipitate was washed with methanol and then dried under reduced pressure, thus obtaining polymer compound 3 (54 mg, 72% yield, Mw = 5100) as a green solid.

1-3. Synthesis of Polymer Compound C1

Polymer compound C1 was synthesized according to the method of US 2015/0031996 A.

[Chemical Formula 57]

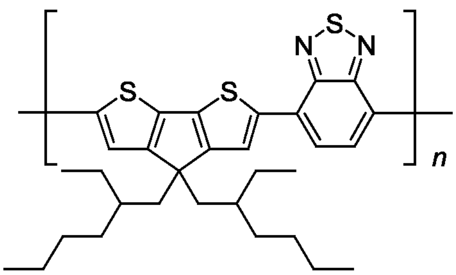

Polymer compound C1

1-4. Measurement of Wavelength of Absorption Maximum and Gram Absorption Coefficient of Polymer Compound

The wavelength of the absorption maximum (λmax) and a gram absorption coefficient (ε) of the obtained polymer compound were measured in a xylene solution using a spectrophotometer (Cary 5000 UV-Vis-NIR spectrophotometer manufactured by Agilent Technologies, Inc.). The measurement procedure was performed, for example, as follows.

(Preparation of Xylene Solution of Polymer Compound)

Xylene (manufactured by KANTO CHEMICAL CO.,INC.) was added to 3 mg of the obtained polymer compound to adjust the whole amount to 300 mg, and then the solution was stirred at 60°C for 6 hours to prepare a 1% by mass xylene solution of the polymer compound.

(Preparation of Sample for Spectrophotometric Measurement and Sample Measurement)

Then, the solution prepared was diluted 50 times, 125 times, 250 times, and 500 times with xylene. Each solution thus prepared was placed in a 1 cm square quartz cell, and an optical spectrum was measured using the spectrophotometer. λmax was a maximum wavelength for the maximum in each optical spectrum. ε was a value of a slope of linear approximation in the case that the horizontal axis was the concentration of the polymer compound, and the vertical axis was the absorbance at λmax.
Xmax in the xylene solution of polymer compound 1 was 793 nm, and ε was 87.1 Lcm$^{-1}$g$^{-1}$.
Xmax in the xylene solution of polymer compound 2 was 901 nm, and ε was 57.5 Lcm$^{-1}$g$^{-1}$.
Xmax in the xylene solution of polymer compound C1 was 710 nm, and ε was 89.1 Lcm$^{-1}$g$^{-1}$.

1-5. Synthesis of Polymer Compound 1A

Polymer compound 1A can be synthesized from polymer compound 1B described in WO 2013/051676 A with reference to the description of the prior literature Chem. Lett. 2018, 47, 927. A synthesis example is described below.

[Chemical Formula 58]

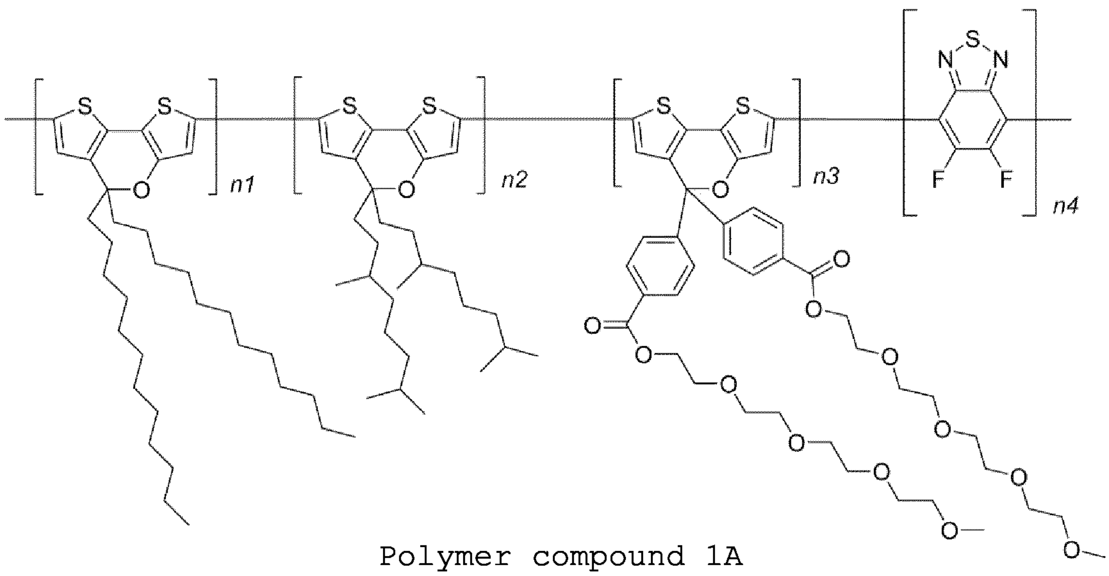

Polymer compound 1A

[Chemical Formula 59]

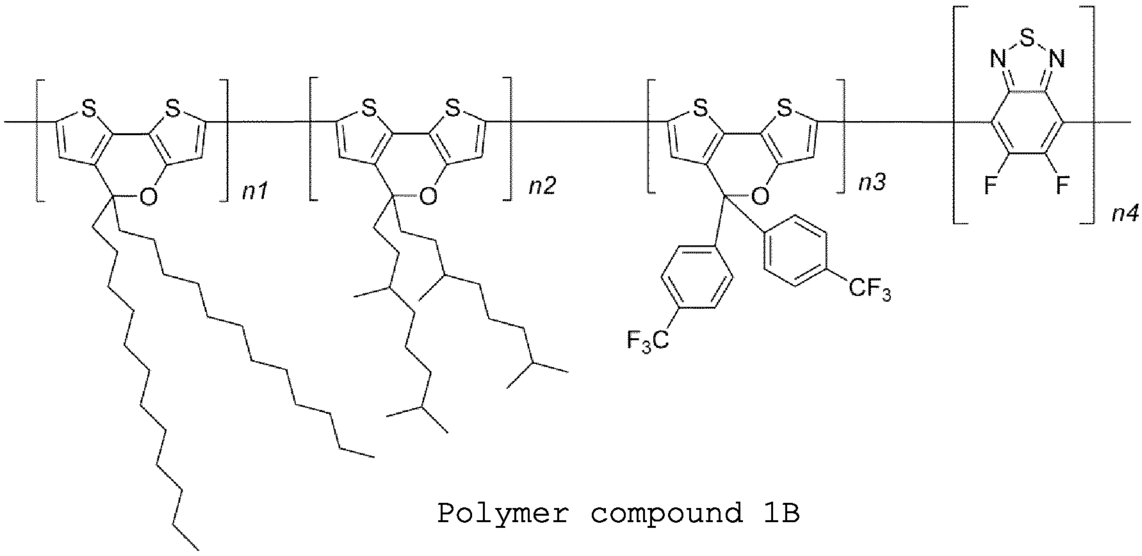

Polymer compound 1B

Polymer compound 1B, bismuth(III) trifluoromethanesulfonate, tetraethylene glycol monoether, and 1,2-dichloroethane are mixed, and the mixture is stirred at 110°C and then cooled to room temperature (25°C). Water is added to the obtained reaction mixture, a compound is extracted with toluene, an organic layer is dehydrated with anhydrous sodium sulfate, and insoluble matters are removed by filtration. The obtained filtrate is removed under reduced pressure to synthesize polymer compound 1A.

2. Examples and Comparative Examples

2-1. Example 1 (Preparation of Nanoparticles 1)

(Step 1-1) Xylene was added to 5 mg of polymer compound 1 so that the whole amount was 1 g, and then the mixture was stirred at 60°C for 6 hours to prepare a 0.5% by mass xylene solution of polymer compound 1 (polymer compound solution 1).

(Step 1-2) To 25 mg of EMULGEN 350 (registered trademark, manufactured by Kao Corporation) as amphiphilic molecules, 25.7 mL of THF (manufactured by KANTO CHEMICAL CO.,INC.) and 17.2 mL of ion-exchanged water (IEW) were added to prepare a 0.5% by mass THF/IEW solution of EMULGEN 350.

(Step 1-3) Ion-exchanged water was added to a phosphate buffer powder (manufactured by FUJIFILM Wako Pure Chemical Corporation, 0.01 mol/L, pH 7.2 to 7.4) so that the whole amount was 1 L to prepare 0.01 mol/L of a phosphate buffer.

(Step 1-4) An aliquot of 40 mL of the phosphate buffer prepared in step 1-3 was taken, to which 1000 mg of the EMULGEN 350 solution prepared in step 1-2 and 800 mg of polymer compound solution 1 prepared in step 1-1 were then added, the mixture was stirred vigorously, and then ultrasonic waves were applied thereto for 30 minutes to prepare an emulsion.

(Step 1-5) The emulsion was stirred at 20°C for 16 hours to volatilize the organic solvent from the dispersoid. Thereafter, the solid content was removed using a 0.45 um filter (manufactured by ADVANTEC TOYO KAISHA, LTD., syringe filter DISMIC CS type) to obtain a dispersion liquid of nanoparticles 1 (dispersion liquid 1).

2-2. Example 2 (Preparation of Nanoparticles 2)

(Step 2-1) Xylene was added to 30 mg of polymer compound 1 so that the whole amount was 3 g, and then the mixture was stirred at 60°C for 6 hours to prepare a 1.0% by mass xylene solution of polymer compound 1 (polymer compound solution 2).

(Step 2-2) An aliquot of 30 mL of the phosphate buffer prepared in step 1-3 was taken, to which 30 mg of Tween (registered trademark) 80 (manufactured by Tokyo Chemical Industry Co., Ltd.) as amphiphilic molecules and 600 mg of polymer compound solution 2 prepared in step 2-1 were then added, the mixture was stirred vigorously, and then ultrasonic waves were applied thereto for 30 minutes to prepare an emulsion.

(Step 2-3) The emulsion was stirred at 20°C for 3 days to volatilize the organic solvent from the dispersoid.

Thereafter, the solid content was removed using a 1 μm filter (manufactured by ADVANTEC TOYO KAISHA, LTD., syringe filter membrane filter, hydrophilic PTFE type) to obtain a dispersion liquid of nanoparticles 2 (dispersion liquid 2).

2-3. Example 3 (Preparation of Nanoparticles 3)

(Step 3-1) "Polymer compound 1" in step 1-1 was replaced with "polymer compound 2" to prepare a 0.5% by mass xylene solution of polymer compound 2 (polymer compound solution 3).

(Step 3-2) An aliquot of 20 mL of the 1mol/L phosphate buffer prepared in step 1-3 was taken, to which 300 mg of the EMULGEN 350 solution prepared in step 1-2 and 600 mg of polymer compound solution 3 prepared in step 3-1 were then added, the mixture was stirred vigorously, and then ultrasonic waves were applied thereto for 30 minutes to prepare an emulsion.

(Step 3-3) The emulsion was stirred at 20°C for 16 hours to volatilize the organic solvent from the dispersoid. Thereafter, the solid content was removed using a centrifuge (manufactured by KOKUSAN Co.Ltd., microvolume high-speed centrifuge H-1500F, 4000 rpm, 10 minutes) to obtain a dispersion liquid of nanoparticles 3 (dispersion liquid 3).

2-4. Example 4 (Preparation of Nanoparticles 4)

(Step 4-1) To 10 mL of ion-exchanged water, 20 mg of DSPE-PEG-2k Amine (manufactured by BroadPharm) as amphiphilic molecules and 400 mg of polymer compound solution 2 prepared in step (2-1) were added, the mixture was stirred vigorously, and then ultrasonic waves were applied thereto for 30 minutes to prepare an emulsion.

(Step 4-2) The emulsion was stirred at 20°C for 16 hours to volatilize the organic solvent from the dispersoid. Thereafter, the solid content was removed using a 1 um filter (manufactured by ADVANTEC TOYO KAISHA, LTD., syringe filter membrane filter, hydrophilic PTFE type) to obtain a dispersion liquid of nanoparticles 4 (dispersion liquid 4).

2-5. Example 5 (Preparation of Nanoparticles 5)

(Step 5-1) To 15 mL of ion-exchanged water, 15 mg of Tween 80 as amphiphilic molecules and 300 mg of polymer compound solution 1 prepared in step (1-1) were added, the mixture was vigorously stirred, and then ultrasonic waves were applied thereto for 30 minutes to prepare an emulsion.

(Step 5-2) The emulsion was stirred at 20°C for 16 hours to volatilize the organic solvent from the dispersoid. Thereafter, the solid content was removed using a 1 μm filter (manufactured by ADVANTEC TOYO KAISHA, LTD., syringe filter membrane filter, hydrophilic PTFE type) to obtain a nanoparticle dispersion liquid.

(Step 5-3) To 5 mL of the nanoparticle dispersion liquid, 68 μL of a 28% ammonia aqueous solution (manufactured by KANTO CHEMICAL CO.,INC.) and 0.18 mL of triethoxysilane (manufactured by Tokyo Chemical Industry Co., Ltd.) were added, and the mixture was stirred for 16 hours. The solid content of the obtained reaction product was removed using a 1 μm filter (manufactured by ADVANTEC TOYO KAISHA, LTD., syringe filter membrane filter, hydrophilic PTFE type), and then ultrafiltration was performed using an ultracentrifuge tube (at 4000 rpm for 30 min, twice) of 100 KDa (manufactured by Millipore Corporation) to obtain a dispersion liquid of nanoparticles 5 (dispersion liquid 5).

2-6. Example 6 (Preparation of Nanoparticles 6)

(Step 6-1) "Polymer compound 1" in step 2-1 was replaced with "polymer compound 3" to prepare a 1% by mass xylene solution of polymer compound 3 (polymer compound solution 4).

(Step 6-2) To 10 mL of ion-exchanged water, 5 mg of Tween 80 and 100 mg of polymer compound solution 4 prepared in step (6-1) were added, the mixture was vigorously stirred, and then ultrasonic waves were applied thereto for 30 minutes to prepare an emulsion.

(Step 6-3) The emulsion was stirred at 20°C for 16 hours to volatilize the organic solvent from the dispersoid. Thereafter, the solid content was removed using a 1 μm filter to obtain a dispersion liquid of nanoparticles 6 (dispersion liquid 6).

2-7. Example 7 (Preparation of Nanoparticles 7)

(Step 7-1) To 16 mL of ion-exchanged water, 10 mg of Tween 80, 10 mg of DSPE-PEG-2k-Amine, and 400 mg of polymer compound solution 1 prepared in step 1-1 were added, the mixture was vigorously stirred, and then ultrasonic waves were applied thereto for 30 minutes to prepare an emulsion.

(Step 7-2) The emulsion was stirred at 20°C for 16 hours to volatilize the organic solvent from the dispersoid. Thereafter, insoluble matters were removed using a 0.45 μm filter, and then the whole amount thereof was transferred to a centrifugal concentrator (molecular weight cutoff: 300 K) (manufactured by Sartorius AG, Vipaspin(R) 20, catalog

number: VS2051) and centrifuged (4000 rpm, 20 min) to remove a supernatant. An operation of further adding ion-exchanged water and centrifuging the mixture was repeated twice to remove excess amphiphilic molecules. A small amount of ion-exchanged water was added thereto to collect the reaction product from the filtration membrane, thus obtaining a dispersion liquid of nanoparticles 7 (dispersion liquid 7).

2-8. Example 8 (Preparation of Nanoparticles 8)

A dispersion liquid of nanoparticles 8 (dispersion liquid 8) was obtained in the same manner as in Example 7, except for adding "50 mg of Tween 80, 50 mg of DSPE-PEG-2k-CH2COOH (manufactured by BroadPharm), and 1000 mg of polymer compound solution 4 prepared in step 6-1 to 100 mL of ion-exchanged water" instead of adding "10 mg of Tween 80, 10 mg of DSPE-PEG-2k-Amine, and 400 mg of polymer compound solution 1 prepared in step 1-1 to 16 mL of ion-exchanged water" described in step 7-1.

2-9. Example 9 (Preparation of Nanoparticles 9)

CM4 was synthesized according to the method described in Sci. Rep. 2015, 5, 9321.

[Chemical Formula 60]

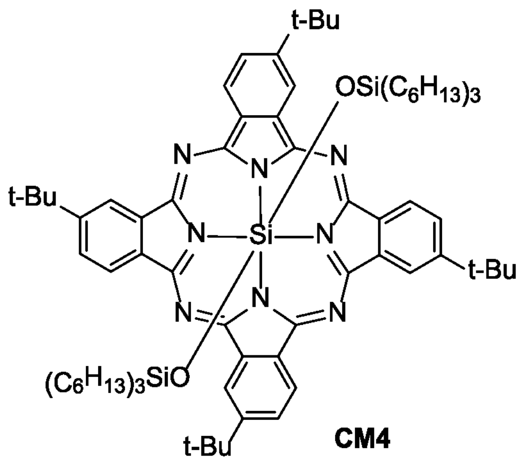

CM4

A dispersion liquid of nanoparticles 9 (dispersion liquid 9) was obtained in the same manner as in Example 7, except for adding "50 mg of Tween 80, 50 mg of DSPE-PEG-2k-CH2COOH, 1mg of CM4, and 1000 mg of polymer compound solution 4 prepared in step 6-1 to 100 mL of ion-exchanged water" instead of adding "10 mg of Tween 80, 10 mg of DSPE-PEG-2k-Amine, and 400 mg of polymer compound solution 1 prepared in step 1-1 to 16 mL of ion-exchanged water" described in step 7-1.

2-10. Comparative Example 1 (Preparation of Dye Solution C1)

(Step C1-1) The phosphate buffer prepared in step 1-3 was added to 400 mg of bovine serum-derived albumin (FUJIFILM Wako Pure Chemical Corporation) to make the whole amount 10 mL, thus obtaining a 4% by mass albumin solution.

(Step C2-2) To 10 mL of the albumin solution, 25 mg of indocyanine green as amphiphilic molecules was added as a dye to obtain a dye solution C1.

2-11. Comparative Example 2 (Preparation of Nanoparticles C2)

(Step C2-1) "Polymer compound 1" described in step 2-1 was replaced with "polymer compound C1" to prepare a 1.0% by mass xylene solution of polymer compound C1 (polymer compound solution C1).

(Step C2-2) To 10 mL of ion-exchanged water, 2.5 mg of Tween 80 as amphiphilic molecules and 50 mg of polymer compound solution C1 prepared in step C2-1 were added, the mixture was vigorously stirred, and then ultrasonic waves were applied thereto for 30 minutes to prepare an emulsion.

(Step C2-3) The emulsion was stirred at 20°C for 16 hours to volatilize the organic solvent from the dispersoid. Thereafter, the solid content was removed using a 1 μm filter (manufactured by ADVANTEC TOYO KAISHA, LTD.,

syringe filter membrane filter, hydrophilic PTFE type) to obtain a dispersion liquid of nanoparticles C2 (dispersion liquid C2).

2-12. Measurement of Wavelength of Absorption Maximum of Nanoparticles and Wavelength of Absorption Maximum of Dye, Measurement of Concentration of Polymer Compound in Dispersion Liquid and Concentration of Dye in Dye Solution, and Calculation of Nanoparticulation Rate

The wavelength of the absorption maximum of the nanoparticles of Examples 1 to 9 and Comparative Example 2 and the wavelength of the absorption maximum of the dye of Comparative Example 1 were measured. In addition, the concentration of the polymer compound in the dispersion liquid of Examples 1 to 9 and Comparative Example 2 and the concentration of the dye in the dye solution of Comparative Example 1 were measured. Furthermore, nanoparticulation rate of Examples 1 to 5 and Comparative Example 2 were calculated.

The wavelength of the absorption maximum, λmax', of the nanoparticles or dye was measured from the obtained dispersion liquid or dye solution using the spectrophotometer used above. The concentration, c, of the polymer compound in the dispersion liquid or the concentration, c, of the dye in the dye solution was calculated from the following Formula (1) based on Lambert-Beer law.

$$c = \alpha A/(\varepsilon l) \quad (1)$$

wherein c represents the concentration of the polymer compound in the dispersion liquid or the concentration of the dye in the dye solution;
α represents a dilution factor;
A represents the absorbance at the wavelength of the absorption maximum, λmax', of the polymer compound in the diluted dispersion liquid or the dye in the diluted dye solution;
ε represents the gram absorption coefficient of the polymer compound solution or dye solution; and
l represents an optical path length in spectrophotometric measurement.

The concentrations c's of the polymer compounds in the dispersion liquids of Examples 1 to 9 and Comparative Example 2 and the concentration c of the dye in the dye solution of Comparative Example 1 measured by the above procedure are each shown in Table 1.

The nanoparticulation yield x of Examples 1 to 9 and Comparative Example 2 was calculated from Formula (2) below. Note that, the dye in the dye solution of Comparative Example 1, which is a low-molecular compound, thus does not contain nanoparticles. Therefore, the nanoparticulation yield x was not measured for Comparative Example 1.

$$x = cw/(c_0 w_0) \qquad (2)$$

wherein, x represents a nanoparticulation yield;
c has the same meaning as described above;
w represents the mass of the nanoparticle dispersion liquid;
$c_0$ represents the concentration of the polymer compound in the polymer compound solution; and
$w_0$ represents the mass of the polymer compound solution.

The nanoparticulation yields x's of the dispersion liquids in Examples 1 to 9 and Comparative Example 2, which were calculated by the above procedure, are each shown in Table 1.

2-13. Particle Diameter of Nanoparticles

An average particle diameter in a dynamic light scattering method using a Zetasizer nano-ZS (manufactured by Malvern Panalytical Ltd) was used as a particle diameter of the nanoparticles. The particle diameters of the nanoparticles 1 to 9 in Examples 1 to 9 are each shown in Table 1.

[Table 1]

| Example/ Comparative Example | Polymer compound or dye molecule | Amphiphilic molecule | Encapsulant | Polymer compound concentration or dye concentration [c (mg/L)] | Nanoparticulation rate [x (%)] | Particle diameter (nm) |
|---|---|---|---|---|---|---|
| Example 1 | Polymer compound 1 | EMULGEN 350 | None | 15 | 15 | 239 |
| Example 2 | Polymer compound 1 | Tween 80 | None | 128 | 64 | 162 |
| Example 3 | Polymer compound 2 | EMULGEN 350 | None | 56 | 56 | 233 |
| Example 4 | Polymer compound 1 | DSPE-PEG-2k Amine | None | 186 | 91 | 146 |
| Example 5 | Polymer compound 1 | Tween 80 | Silica | 61 | 65 | 212 |
| Example 6 | Polymer compound 3 | Tween 80 | None | 113 | 95 | 79 |
| Example 7 | Polymer compound 1 | Tween 80 DSPE-PEG-2k Amine | None | 370 | 15 | 187 |
| Example 8 | Polymer compound 3 | Tween 80 DSPE-PEG-2k-CH2COOH | None | 223 | 65 | 155 |
| Example 9 | Polymer compound 3/ CM4 | Tween 80 DSPE-PEG-2k-CH2COOH | None | 348 (Polymer compound concentration) 51 (dye concentration) | 47 | 138 |
| Comparative Example 1 | Indocyanine green | Albumin | None | 2500 | - | - |
| Comparative Example 2 | Polymer compound C1 | Tween 80 | None | 14.8 | 30 | - |

2-14. Measurement of Fluorescence Quantum Yield of Nanoparticles

An aliquot of 1 mL of the dispersion liquid of the obtained nanoparticles 1 was placed into a 10 mL volumetric flask, to which a dispersion medium is then added until the meniscus is aligned with the marked line of the 10 mL volumetric flask, thus diluting the dispersion liquid. Then, the fluorescence quantum yield was measured at an excitation light wavelength of 794 nm at room temperature (25°C) in the atmosphere for the diluted liquid of nanoparticles 1 prepared, using an apparatus for measuring an absolute PL quantum yield (Hamamatsu Photonics K.K.). The fluorescence quantum yield of nanoparticles 1 in Example 1 was 0.008%.

2-15. Measurement of Maximum Wavelength for Photoacoustic Signal and Generation Efficiency of Photoacoustic Signal

(System for Measuring Photoacoustic Signal)

As an excitation light source, an idler light output from a tunable optical parametric oscillator (OPO, Versascan MBI-FE, Spectra-Physics, CA, USA) excited by third harmonic generation of a pulsed Nd:YAG laser (Quanta-Ray Pro-190-THDA-FE, Spectra-Physics) was used. The excitation light had a pulse width of 6 to 8 ns and a pulse repetition frequency of 10 Hz. The pulse energy of the excitation light was continuously monitored, in which the reflected light by a beam sampler (BSF-A, Thorlabs, Inc., Newton, NJ, USA) inserted into the optical path between the light source and the optical fiber was observed using an energy meter (PE25-C, Ophir Optroniics, Jerusalem, Israel), and the observed value was divided by a ratio of branching by the beam sampler. The excitation light was introduced into the optical fiber (M40L02, Thorlabs, Inc.) having a core diameter of 400 μm, and the emission end of the optical fiber was fixed through the center of a non-focal ring type acoustic sensor having an outer diameter of 4.0 mm and an inner diameter of 1.4 mm. The sensor composed of a 20 μm thick P (VDF-TrFE) film (KF piezo film, Kureha Corporation) had a -6 dB bandwidth of 3.0 to 19.5 MHz. In order to amplify the photoacoustic signal detected by the sensor, a low noise field effect transistor amplifier (SA220F5, NF Corporation) was used. The amplified photoacoustic signal was recorded by a 10-bit resolution PXI digitizer (M9210A, Agilent Technologies, Inc., Santa Clara, CA, USA) operating at a sampling frequency of 100 MHz.

(Method for Measuring Photoacoustic Signal)

The photoacoustic signal of the nanoparticles of Examples 1 to 6 and Comparative Example 2 and the dye of Comparative Example 1 was measured using the photoacoustic measurement system described above. The acoustic sensor was immersed in a water tank filled with degassed water, and the distance between the detection surface of the sensor and the bottom surface of the water tank was fixed to 10 mm. A cover glass (Micro Cover Glass No. 00, Matsunami Glass Ind.,Ltd.) having a thickness of 80 μm was attached to the bottom surface of the water tank. A dispersion liquid of nanoparticles or the dye solution (100 μE) of the dye was dropped onto the slide glass, and the cover glass on the bottom surface of the water tank was fixed in contact with the aqueous solution and at a position 1 mm away from the slide glass surface. In order to measure the photoacoustic signal, excitation light having a pulse energy of 100 μJ per pulse was applied to the aqueous solution. The wavelength of the excitation light was set to the range of 700 to 1100 nm or 700 to 1200 nm every 10 nm. The photoacoustic signal generated for each pulse of the excitation light was observed 50 times per wavelength and averaged. The maximum value of the photoacoustic signal was measured with the offset removed. The offset is derived from absorption, by the acoustic sensor, of a pyroelectric signal generated by the excitation light reflected back. The signal intensity per pulse energy was calculated by dividing the maximum value of the photoacoustic signal by the pulse energy of the excitation light. As dispersion liquids of the nanoparticles or the dye solution of the dye, samples diluted with a dispersion solvent so as to have absorbances of 1.0 $cm^{-1}$, 1.5 $cm^{-1}$, and 2.0 $cm^{-1}$ were used. The generation efficiency of a photoacoustic signal was calculated from the slope of linear approximation for a plot in the case that the horizontal axis was the absorbance of the photoacoustic signal at the maximum wavelength and the vertical axis was the signal intensity per pulse energy. The maximum wavelengths for photoacoustic signals and generation efficiencies of photoacoustic signals measured by the above procedure are each shown in Table 2.

[Table 2]

| Example/ Comparative Example | Polymer compound or dye molecule | Amphiphilic molecule | Photoacoustic signal | |
|---|---|---|---|---|
| | | | Maximum wavelength (nm) | Generation efficiency (V·cm/J) |
| Example 1 | Polymer compound 1 | EMULGEN 350 | 790 | 180 |
| Example 2 | Polymer compound 1 | Tween 80 | 790 | 125 |
| Example 3 | Polymer compound 2 | EMULGEN 350 | 900 | 182 |
| Example 4 | Polymer compound 1 | DSPE-PEG-2k Amine | 800 | 121 |
| Example 5 | Polymer compound 1 | Tween 80 | 790 | 134 |
| Example 6 | Polymer compound 3 | Tween 80 | 1160 | 116 |

(continued)

| Example/ Comparative Example | Polymer compound or dye molecule | Amphiphilic molecule | Photoacoustic signal | |
|---|---|---|---|---|
| | | | Maximum wavelength (nm) | Generation efficiency (V·cm/J) |
| Comparative Example 1 | Indocyanine green | Albumin | 790 | 50 |
| Comparative Example 2 | Polymer compound C1 | Tween 80 | 700 | 82 |

As shown in Table 2, it was found that the nanoparticles of Examples 1 to 6 are excellent in generation efficiency of a photoacoustic signal, as compared with the dye of Comparative Example 1 and the nanoparticles of Comparative Example 2. In addition, nanoparticles of Examples 7 to 9, which have the same configuration as the nanoparticles of Examples 1 to 6, are therefore presumed to be excellent in generation efficiency of a photoacoustic signal. From the above, it was confirmed that the nanoparticles of the present invention can improve generation efficiency of a photoacoustic signal.

2-16. Example 10 (Preparation of antibody-conjugated Nanoparticles 1)

(Step 10-1) As an antibody, a mouse monoclonal antibody (antibody name: 3B1E2) that binds to human pancreatic cancer cell line KP-3L (Japanese Collection of Research Bioresources (JCRB) Cell Bank, cell registration number: JCRB0178.1) was used. Using 50 μg of the antibody, a biotin-labeled antibody was prepared with Biotin Labeling Kit-$NH_2$ (manufactured by Dojindo Laboratories, catalog number: LK03).

(Step 10-2) To 10 μg of nanoparticles 7 described in Example 7, streptavidin in a molar ratio of 90 times to nanoparticles 7 and 1 μL of a Modifier reagent included in a FastLink Streptavidin Labeling Kit (manufactured by Abnova Corporation, catalogue number: KA1556) were added, and the mixture was left for 3 hours at room temperature (25°C) under a light-shielding condition. Next, 15 μL of Quencher reagent included in the FastLink Streptavidin Labeling Kit was added to the mixture, which was then left for 15 minutes to stop the reaction, and then the whole amount of the mixture was transferred to a Nanosep centrifugal filtration device (molecular weight cutoff: 300 K) (manufactured by Pall Corporation, catalogue number: OD300C33) and centrifuged (15000 rpm, 5 min) to remove unreacted streptavidin. The operation of adding 300 μL of distilled water and centrifuging the mixture was repeated three times to wash the filtration membrane, and then 50 μL of distilled water was added twice to collect streptavidin-bound nanoparticles from the filtration membrane.

(Step 10-3) The streptavidin-bound nanoparticles described in step 10-2 were added to the biotin-labeled antibody described in step 10-1, the mixture was blended at 4°C for 1 hour, and then the whole amount of the mixture was added to the Nanosep centrifugal filtration device (molecular weight cutoff: 300 K, manufactured by Pall Corporation, catalogue number: OD300C33) and centrifuged. After washing the filtration membrane with distilled water, 50 μL of distilled water was added twice to collect the reaction product from the filtration membrane, thus obtaining antibody-conjugated nanoparticles 1.

2-17. Example 11 (Preparation of antibody-conjugated Nanoparticles 2)

antibody-conjugated nanoparticles 2 were obtained in the same manner as in Example 10 except that the "mouse monoclonal antibody (antibody name: 3B1E2) that binds to human pancreatic cancer cell line KP-3L (JCRB Cell Bank, cell registration number: JCRB0178.1)" described in step 10-1 were replaced with "Control IgG".

2-18. Example 12 (Preparation of antibody-conjugated Nanoparticles 3)

Using 4-(4,6-dimethoxy-1,3,5-triazin-2-yl)-4-methyl-morpholinium chloride, 100 μg of nanoparticles 8 described in Example 8 were reacted with an antibody 3B1E2 overnight at room temperature (25°C). The reaction solution was transferred to the Nanosep centrifugal filtration device (molecular weight cutoff: 300 K) and centrifuged (10,000 rpm, 1 min). The operation of adding 100 μL of ion-exchanged water and centrifuging the mixture was repeated three times in total. To the mixture, 50 μL of distilled water was added twice to collect the reaction product from the filtration membrane, thus obtaining antibody-conjugated nanoparticles 3.

2-19. Example 13 (Preparation of antibody-conjugated Nanoparticles 4)

antibody-conjugated nanoparticles 4 were obtained in the same manner as in Example 12 except that the "antibody 3B1E2" described in Example 12 was replaced with a "Control IgG".

2-20. Example 14 (Preparation of antibody-conjugated Nanoparticles 5)

antibody-conjugated nanoparticles 5 were obtained in the same manner as in Example 12 except that the "nanoparticles 8 described in Example 8" described in Example 12 were replaced with the "nanoparticles 9 described in Example 9".

2-21. Example 15 (Preparation of antibody-conjugated Nanoparticles 6)

antibody-conjugated nanoparticles 6 were obtained in the same manner as in Example 12, except that the "nanoparticles 8 described in Example 8" described in Example 12 were replaced with the "nanoparticles 9 described in Example 9", and the "antibody 3B1E2" was replaced with "Control IgG".

2-22. Measurement of Mouse IgG Concentration

A peroxidase-labeled rabbit anti-mouse IgG polyclonal antibody was prepared using 10 μg of a rabbit anti-mouse IgG polyclonal antibody (manufactured by Bethyl Laboratories, Inc., catalogue number: A90-217A) and Ab-10 Rapid Peroxidase Labeling Kit (manufactured by Dojindo Laboratories, catalog number: LK33). In a MaxiSorp 96 well plate, 100 μL of a 10 μg/mL rabbit anti-mouse IgG polyclonal antibody was added to each well, which was then left at 4°C for 16 hours. Next, each well was washed three times with 200 μL of a washing liquid (50 mM Tris-HCl (pH 8.0) containing 0.05% Triton-X 100, 140 mM NaCl), and 200 μL of a washing liquid containing 5% bovine serum albumin (BSA) was added to each well, which was then left at room temperature for 1 hour. Each well was washed three times, and 100 μL specimens (antibody-conjugated nanoparticles 1 to 6) or 100 μL mouse IgGs (manufactured by BioLegend, Inc., catalogue number: 400102) with different concentrations were each added to the well, which was then left at room temperature for 1 hour. Thereafter, each well was washed five times, and 100 μL of the peroxidase-labeled rabbit anti-mouse IgG polyclonal antibody diluted 1000 times with a specimen diluting solution was added to each well, which was then left at room temperature for 1 hour. Each well was washed five times, and then 100 μL of a peroxidase substrate solution (manufactured by SeraCare Life Sciences, Inc., catalog number: 5120-0053) was added to each well, which was then left at room temperature for 30 minutes. After the reaction, 50 μL of 2% sulfuric acid was added to stop the reaction, the absorbance for each well at 450 nm was measured with a microplate reader, and the concentration of mouse IgG in the specimen was calculated from the calibration curve with mouse IgG at each concentration. The antibody concentrations in the aqueous dispersions of the antibody-conjugated nanoparticles of Examples 10 to 15 calculated by the above procedure are each shown in Table 3.

[Table 3]

| Example/ Comparative Example | Antibody-conjugated nanoparticles | Nanoparticles | Antibody | Antibody concentration (μg $mL^{-1}$) |
|---|---|---|---|---|
| Example 10 | Antibody-conjugated nanoparticles 1 | Nanoparticles 7 | 3B1E2 | 2.0 |
| Example 11 | Antibody-conjugated nanoparticles 2 | Nanoparticles 7 | Control IgG | 2.0 |
| Example 12 | Antibody-conjugated nanoparticles 3 | Nanoparticles 8 | 3B1E2 | 0.30 |
| Example 13 | Antibody-conjugated nanoparticles 4 | Nanoparticles 8 | Control IgG | 0.30 |
| Example 14 | Antibody-conjugated nanoparticles 5 | Nanoparticles 9 | 3B1E2 | 0.19 |
| Example 15 | Antibody-conjugated nanoparticles 6 | Nanoparticles 9 | Control IgG | 0.12 |

2-23. Evaluation of Accumulation of antibody-conjugated Nanoparticles and Nanoparticles in Cancer Cell Line

(Evaluation of Accumulation of antibody-conjugated Nanoparticles of Example 10, antibody-conjugated Nanoparticles of Example 11, and Nanoparticles of Example 2 in Cancer Cell Line)

antibody-conjugated nanoparticles 1 of Example 10, antibody-conjugated nanoparticles 2 of Example 11, and nanoparticles 2 of Example 2 were used to evaluate accumulation thereof in a cancer cell line. KP-3L cells were seeded in a 96 well plate, and 4% paraformaldehyde was added to fix the cells at 4 °C for 20 minutes. The cells were washed three times with phosphate-buffered saline (PBS)(-) and blocked with PBS(-) containing 2% fetal bovine serum, and then a cancer cell line of the antibody-conjugated nanoparticles or nanoparticles was added to react the mixture at room temperature for 1 hour. Next, the cells were washed three times with PBS(-), and then an Alexa Fluor 594-labeled goat anti-mouse IgG antibody (Invitrogen, catalogue number: A11032) diluted 1000 times and Hoechst 33342 were added to react the mixture at room temperature for 1 hour. After washing the cells three times with PBS(-), the cells were observed with a fluorescence microscope.

Fig. 1 is a fluorescence microscopic observation image for evaluating accumulation of the antibody-conjugated nanoparticles and the nanoparticles in the cancer cell line. Fig. 1(a) is a fluorescence microscopic observation image in the case of using antibody-conjugated nanoparticles 1 of Example 10, Fig. 1(b) is a fluorescence microscopic observation image in the case of using antibody-conjugated nanoparticles 2 of Example 11, and Fig. 1(c) is a fluorescence microscopic observation image in the case of using nanoparticles 2 of Example 2. In Fig. 1(a), red luminescence was observed at positions surrounded by dotted circles and around the cells, and it was confirmed that antibody-conjugated nanoparticles 1 of Example 10 were accumulated in the KP-3L cells. On the other hand, in Figs. 1(b) and 1(c), such red luminescence was not observed, and it was confirmed that antibody-conjugated nanoparticles 2 of Example 11 and nanoparticles 2 of Example 2 were not accumulated in the KP-3L cells.

(Evaluation of Accumulation of antibody-conjugated Nanoparticles of Examples 12 to 15 in Cancer Cell Line)

antibody-conjugated nanoparticles 3 of Example 12, antibody-conjugated nanoparticles 4 of Example 13, antibody-conjugated nanoparticles 5 of Example 14, and antibody-conjugated nanoparticles 6 of Example 15 were used to evaluate accumulation thereof in a cancer cell line. The day after KP-3L cells were seeded in a 96 well plate, the cells were washed twice with PBS, to which 4% paraformaldehyde was then added, and were fixed at room temperature for 30 minutes. The cells were washed twice with Blocking One (Nacalai Tesque, Inc., catalog number: 03953-66, hereinafter referred to as "Blocking buffer") diluted 5 times with ion-exchanged water, and then antibody-conjugated nanoparticles of Examples 12 to 15 were each added thereto, and the plate was left at room temperature (25 °C) for 1 hour. The cells were washed three times with the Blocking buffer, to which an Alexa Fluor 488-labeled donkey anti-mouse IgG antibody (Invitrogen, catalogue number: A32766) diluted 1000 times with the Blocking buffer and Hoechst 33342 were then added, and the mixture was reacted at room temperature for 1 hour. After washing the cells twice with the Blocking buffer, the cells were observed with a fluorescence microscope.

Fig. 2 is a fluorescence microscopic observation image for evaluating accumulation of the antibody-conjugated nanoparticles in the cancer cell line. Fig. 2(a) is a fluorescence microscopic observation image in the case of using antibody-conjugated nanoparticles 3 of Example 12, and Fig. 2(b) is a fluorescence microscopic observation image in the case of using antibody-conjugated nanoparticles 4 of Example 13. In Fig. 2(a), green luminescence was observed around the cells, and it was confirmed that antibody-conjugated nanoparticles 3 of Example 12 were accumulated in the KP-3L cells. On the other hand, in Fig. 2(b), such green luminescence was not observed, and it was confirmed that antibody-conjugated nanoparticles 4 of Example 13 were not accumulated in the KP-3L cells.

Fig. 3 is a fluorescence microscopic observation image for evaluating accumulation of the antibody-conjugated nanoparticles in the cancer cell line. Fig. 3 (a) is a fluorescence microscopic observation image in the case of using antibody-conjugated nanoparticles 5 of Example 14, and Fig. 3 (b) is a fluorescence microscopic observation image in the case of using antibody-conjugated nanoparticles 6 of Example 15. In Fig. 3(a), green luminescence was observed around the cells, and it was confirmed that antibody-conjugated nanoparticles 5 of Example 14 were accumulated in the KP-3L cells. On the other hand, in Fig. 3(b), such green luminescence was not observed, and it was confirmed that antibody-conjugated nanoparticles 6 of Example 15 were not accumulated in the KP-3L cells.

## Claims

1. A nanoparticle comprising at least one amphiphilic molecule and a polymer compound having a constitutional unit represented by Formula (1):

[Chemical Formula 1]

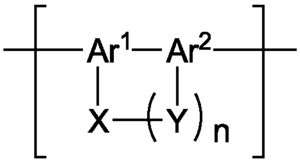

(1)

wherein, $Ar^1$ and $Ar^2$ each independently represent a trivalent aromatic hydrocarbon ring group or a trivalent aromatic heterocyclic group, and these groups optionally have a substituent;

X and Y each independently represent -O-, -S-, - C(=O)-, -S(=O)-, -SOz-, $-CR_2-$, $-SiR_2-$, -NR-, -BR-, -PR-, or - P (=0) (R) - ;

R represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, or a heteroaryloxycarbonyl group, and these groups optionally have a substituent;

in the case that a plurality of Rs are present, they may be identical or different from each other;

n represents an integer of 1 or more; and

in the case that n is 2 or more, a plurality of Ys may be identical or different from each other.

**2.** The nanoparticle according to claim 1, wherein $Ar^1$ and $Ar^2$ are trivalent aromatic heterocyclic groups in Formula (1).

**3.** The nanoparticle according to claim 1 or 2,
wherein the constitutional unit represented by Formula (1) is a constitutional unit represented by Formula (1a):

[Chemical Formula 2]

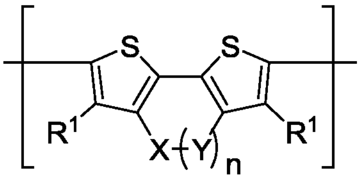

(1a)

wherein, X, Y, and n have the same meanings as described above; and

$R^1$ represents a hydrogen atom or an alkyl group optionally having a substituent.

**4.** The nanoparticle according to any one of claims 1 to 3, wherein n is 1.

**5.** The nanoparticle according to any one of claims 1 to 4, wherein the constitutional unit represented by Formula (1) is a constitutional unit represented by Formula (1b) :

[Chemical Formula 3]

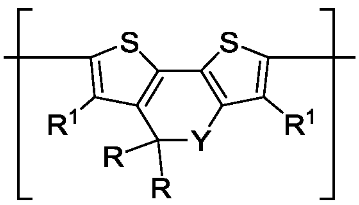

(1b)

wherein, R, $R^1$, and Y have the same meanings as described above.

**6.** The nanoparticle according to any one of claims 1 to 5, wherein Y is -O-.

**7.** The nanoparticle according to any one of claims 1 to 6, wherein the polymer compound further has a constitutional unit represented by Formula (3):

[Chemical Formula 4]

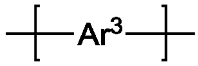
(3)

wherein $Ar^3$ is different from the constitutional unit represented by Formula (1) and represents an arylene group optionally having a substituent or a divalent heterocyclic group optionally having a substituent.

**8.** The nanoparticle according to claim 7, wherein the constitutional unit represented by Formula (3) includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e):

[Chemical Formula 5]

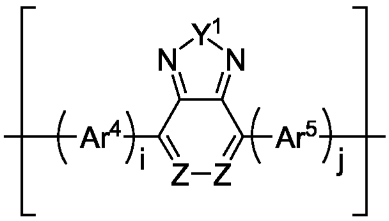
(2a)

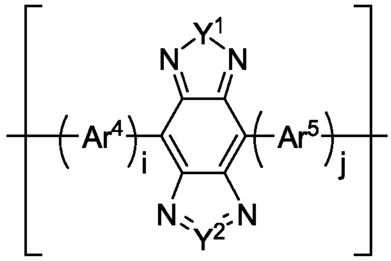
(2b)

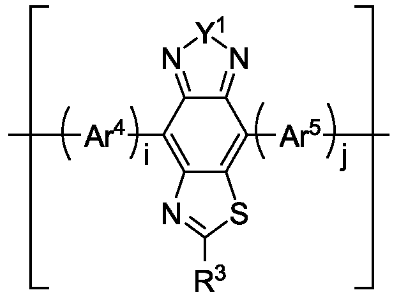
(2c)

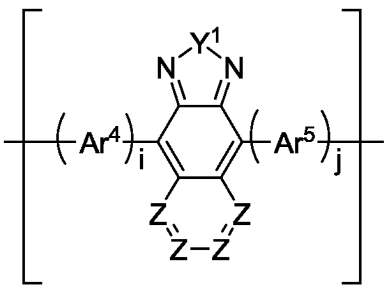
(2d)

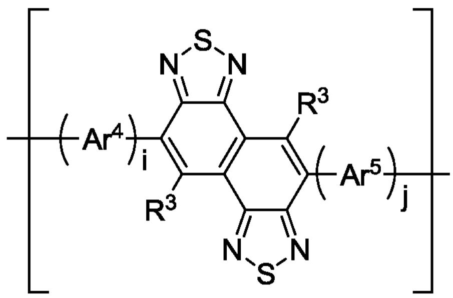
(2e)

wherein, $R^3$ represents a hydrogen atom, a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, an amino group, a silyl group, a silyloxy group, a silylthio group, a silylamino group, a monovalent heterocyclic group, a heteroaryloxy group, a heteroarylthio group, an arylalkenyl group, an arylethynyl group, a carboxyl group, an alkyloxycarbonyl group, an aryloxycarbonyl group, an arylalkyloxycarbonyl group, a heteroaryloxycarbonyl group, a nitro group, or a cyano group; these groups optionally have a substituent; and a plurality of $R^3$s may be identical or different from each other;

$Ar^4$ and $Ar^5$ are different from the constitutional unit represented by Formula (1) and each independently represent an arylene group or a divalent heterocyclic group, and these groups optionally have a substituent;

i and j each independently represent an integer of 0 or 1;

$Y^1$ represents -O-, -S-, -S(=O)-, $-SO_2-$, -Se-, -Te-, or $-N(R^3)-$;

$Y^2$ represents =S=, =Se=, or =Te=;
Z represents -C($R^4$)= or -N=; $R^4$ has the same meaning as $R^3$; and a plurality of $R^4$s may be identical or different from each other and may be bound to each other to make a ring; and
a plurality of Zs may be identical or different from each other.

**9.** The nanoparticle according to any one of claims 1 to 8, wherein the amphiphilic molecule has a group represented by Formula (5a):

[Chemical Formula 6]

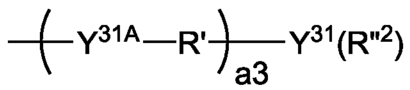
(5a)

wherein, a3 represents an integer of 0 or 1 or more;
R' represents an alkylene group, a cycloalkylene group, or an arylene group, and these groups optionally have a substituent;
in the case that a plurality of R's are present, they may be identical or different from each other;
$R''^2$ represents a hydrogen atom, an alkyl group, a cycloalkyl group, or an aryl group, and these groups optionally have a substituent;
$Y^{31}$ represents -O-, -S-, -C(=O)O-, or -N($R''^2$)-;
$Y^{31A}$ represents -O- or -S-; and
in the case that a plurality of $Y^{31A}$s are present, they may be identical or different from each other.

**10.** The nanoparticle according to claim 9, wherein $R''^2$ represents a hydrogen atom, and $Y^{31}$ represents -S-, - C(=O)O-, or -NH- in Formula (5a).

**11.** The nanoparticle according to any one of claims 1 to 10, wherein at least one capture molecule selected from the group consisting of an antibody, a sugar chain, an aptamer, a receptor, a peptide, a transport protein, and an enzyme is bound to at least one of the polymer compound or the amphiphilic molecule.

**12.** A nanoparticle comprising a polymer compound having a group represented by Formula (5) and having a constitutional unit represented by Formula (1A):

[Chemical Formula 7]

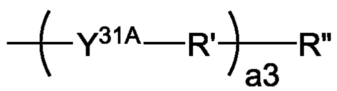
(5)

wherein, a3 represents an integer of 0 or 1 or more;
R' represents an alkylene group, a cycloalkylene group, or an arylene group, and these groups optionally have a substituent;
in the case that a plurality of R's are present, they may be identical or different from each other;
R" represents -$Y^{31}$($R''^2$) or -$Y^{31}$(M) ;
$R''^2$ represents a hydrogen atom, an alkyl group, a cycloalkyl group, or an aryl group, and these groups optionally have a substituent;
M represents an alkali metal cation or a quaternary ammonium;
$Y^{31}$ represents -O-, -S-, -C(=O)O-, or -N($R''^2$)-;
$Y^{31A}$ represents -O- or -S-; and
in the case that a plurality of $Y^{31A}$s are present, they may be identical or different from each other, and

[Chemical Formula 8]

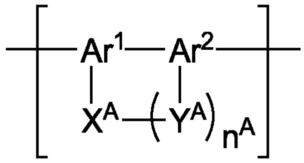

(1A)

wherein, $Ar^1$ and $Ar^2$ each independently represent a trivalent aromatic hydrocarbon ring group or a trivalent aromatic heterocyclic group, and these groups optionally have a substituent;
$X^A$ and $Y^A$ each independently represent -O-, -S-, - C(=O)-, -S(=O)-, $-SO_2-$, $-CR^A{}_2-$, $-SiR^A{}_2-$, $-NR^A-$, $-BR^A-$, $-PR^A-$, or $-P(=O)(R^A)-$;
$R^A$ represents a hydrogen atom, an alkyl group, an alkenyl group, an alkynyl group, a hydroxy group, an alkyloxy group, an aryl group, an aryloxy group, an arylalkyl group, an acyl group, an acyloxy group, an amide group, an amino group, a monovalent aromatic heterocyclic group, a heteroaryloxy group, a heteroarylthio group, a carboxyl group, an alkyloxycarbonyl group, an aryloxycarbonyl group, an arylalkyloxycarbonyl group, a heteroaryloxycarbonyl group, or the group represented by Formula (5), and a group other than the group represented by Formula (5) optionally has the group represented by Formula (5) or a substituent;
in the case that a plurality of $R^A$s are present, they may be identical or different from each other;
$n^A$ represents an integer of 1 or more; and
in the case that $n^A$ is 2 or more, a plurality of $Y^A$s may be identical or different from each other.

**13.** The nanoparticle according to claim 12, wherein $Ar^1$ and $Ar^2$ are trivalent aromatic heterocyclic groups in Formula (1A).

**14.** The nanoparticle according to claim 12 or 13, wherein the constitutional unit represented by Formula (1A) is a constitutional unit represented by Formula (1Aa):

[Chemical Formula 9]

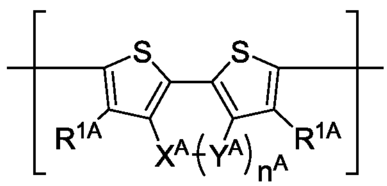

(1Aa)

wherein, $X^A$, $Y^A$, and $n^A$ have the same meanings as described above; and
$R^{1A}$ represents a hydrogen atom, the group represented by Formula (5), or an alkyl group optionally having the group represented by Formula (5) or a substituent.

**15.** The nanoparticle according to any one of claims 12 to 14, wherein $n^A$ is 1.

**16.** The nanoparticle according to any one of claims 12 to 15, wherein the constitutional unit represented by Formula (1A) is a constitutional unit represented by Formula (1Ab):

[Chemical Formula 10]

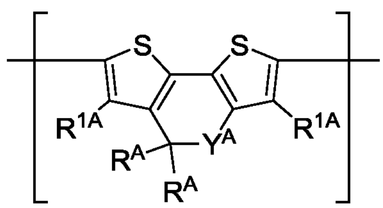

(1Ab)

wherein, $R^A$, $R^{1A}$, and $Y^A$ have the same meanings as described above.

**17.** The nanoparticle according to any one of claims 12 to 16, wherein $Y^A$ is -O-.

**18.** The nanoparticle according to any one of claims 12 to 17, wherein the polymer compound further has a constitutional unit represented by Formula (3):

[Chemical Formula 11]

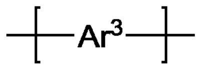

(3)

wherein $Ar^3$ is different from the constitutional unit represented by Formula (1A) and represents an arylene group optionally having a substituent or a divalent heterocyclic group optionally having a substituent.

**19.** The nanoparticle according to claim 18, wherein the constitutional unit represented by Formula (3) includes at least one constitutional unit selected from the group consisting of a constitutional unit represented by Formula (2a), a constitutional unit represented by Formula (2b), a constitutional unit represented by Formula (2c), a constitutional unit represented by Formula (2d), and a constitutional unit represented by Formula (2e):

[Chemical Formula 12]

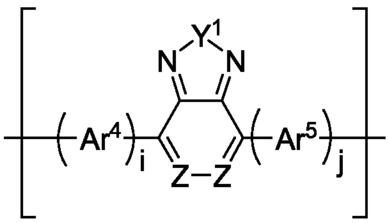

(2a)

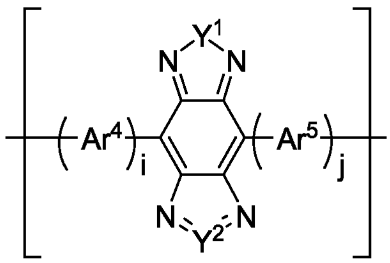

(2b)

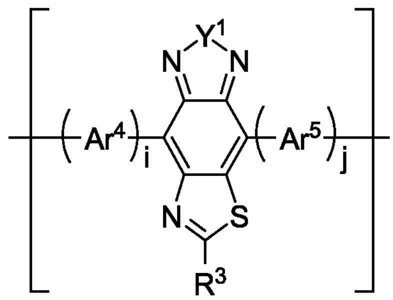

(2c)

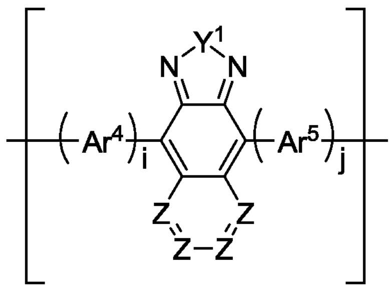

(2d)

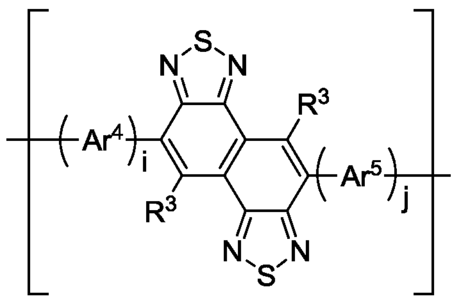

(2e)

wherein, $R^3$ represents a hydrogen atom, a halogen atom, an alkyl group, an alkyloxy group, an alkylthio group, an aryl group, an aryloxy group, an arylthio group, an arylalkyl group, an arylalkyloxy group, an arylalkylthio group, an acyl group, an acyloxy group, an amide group, an acid imide group, an imine residue, an amino group, a silyl group, a silyloxy group, a silylthio group, a silylamino group, a monovalent heterocyclic group, a heteroaryloxy group, a heteroarylthio group, an arylalkenyl group, an arylethynyl group, a carboxyl group, an alkyloxycarbonyl group, an aryloxycarbonyl group, an arylalkyloxycarbonyl group, a heteroaryloxycarbonyl group, a nitro group, or a cyano group; these groups optionally have a substituent; and a plurality of $R^3$s may be identical or different from each other;

$Ar^4$ and $Ar^5$ are different from the constitutional unit represented by Formula (1A) and each independently represent an arylene group or a divalent heterocyclic group, and these groups optionally have a substituent;

i and j each independently represent an integer of 0 or 1;
$Y^1$ represents -O-, -S-, -S(=O)-, $-SO_2-$, -Se-, -Te-, or $-N(R^3)-$;
$Y^2$ represents =S=, =Se=, or =Te=;
Z represents $-C(R^4)=$ or -N=; $R^4$ has the same meaning as $R^3$; and a plurality of $R^4$s may be identical or different from each other and may be bound to each other to make a ring; and
a plurality of Zs may be identical or different from each other.

**20.** The nanoparticle according to any one of claims 12 to 19, wherein the group represented by Formula (5) is a group represented by Formula (5a):

[Chemical Formula 13]

$$-\left(Y^{31A}-R'\right)_{a3}-Y^{31}(R''^{2}) \qquad (5a)$$

wherein, a3, R', $Y^{31A}$, $R''^{2}$, and $Y^{31}$ have the same meanings as described above.

**21.** The nanoparticle according to claim 20, wherein $R''^{2}$ represents a hydrogen atom, and $Y^{31}$ represents -S-, - C(=O)O-, or -NH- in Formula (5a).

**22.** The nanoparticle according to any one of claims 12 to 21, wherein at least one capture molecule selected from the group consisting of an antibody, a sugar chain, an aptamer, a receptor, a peptide, a transport protein, and an enzyme is bound to the polymer compound.

**23.** The nanoparticle according to any one of claims 1 to 22, which is used for a photoacoustic contrast agent, a photodynamic therapy drug, or a photothermal therapy drug.

**24.** A photoacoustic contrast agent comprising the nanoparticle according to any one of claims 1 to 22.

**25.** A photodynamic therapy drug comprising the nanoparticle according to any one of claims 1 to 22.

**26.** A photothermal therapy drug comprising the nanoparticle according to any one of claims 1 to 22.

[Figure 1]

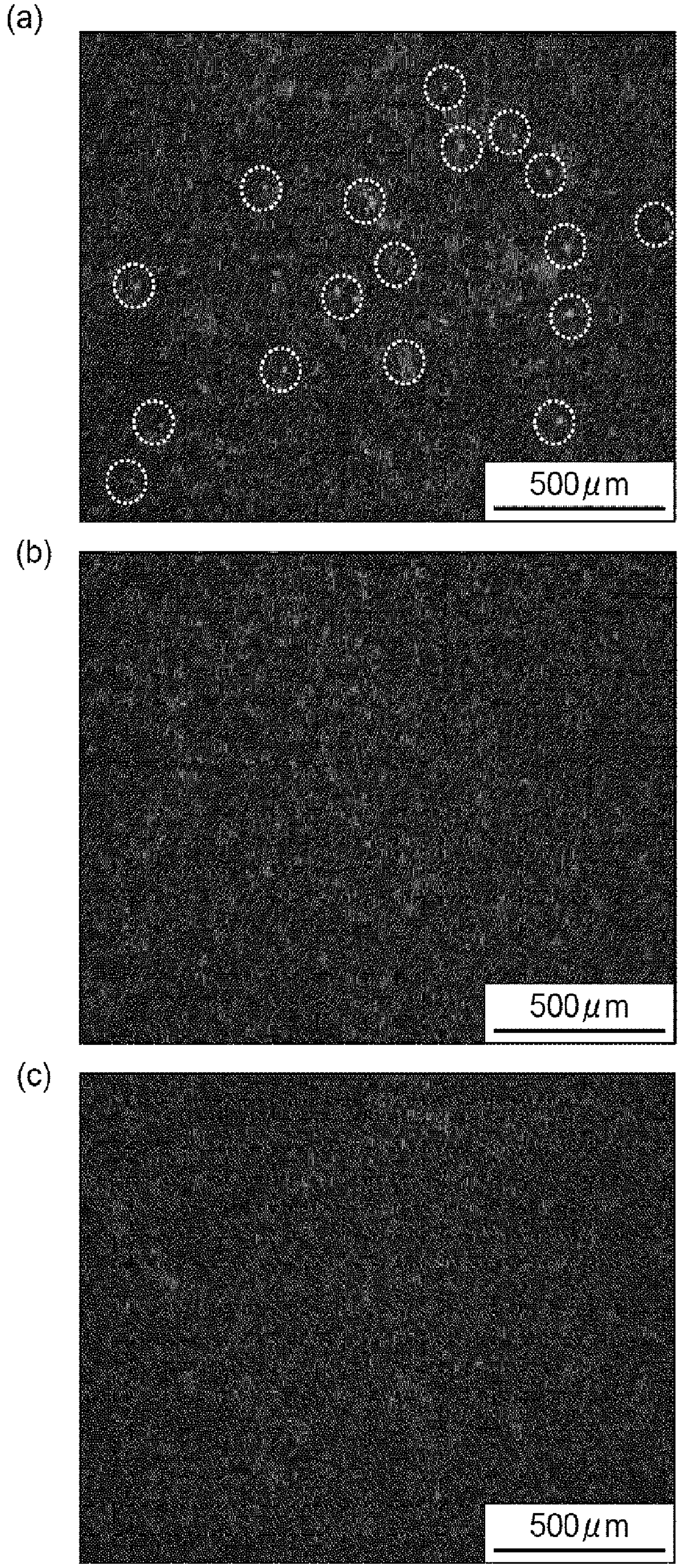

[Figure 2]

(a)

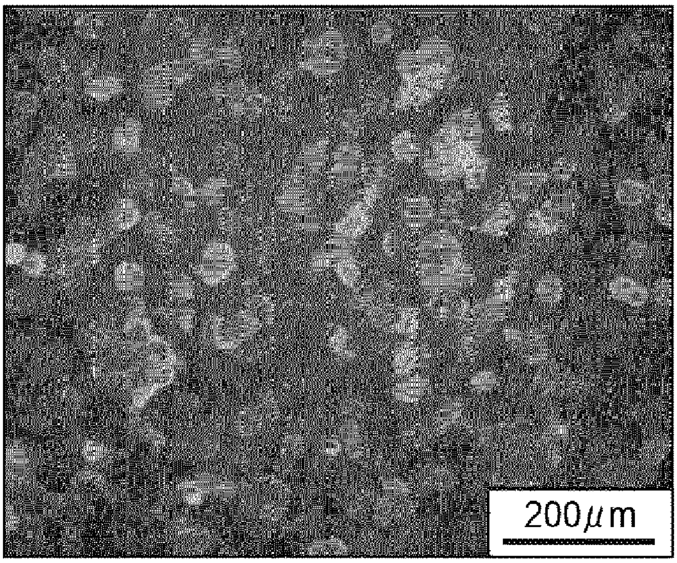


(b)

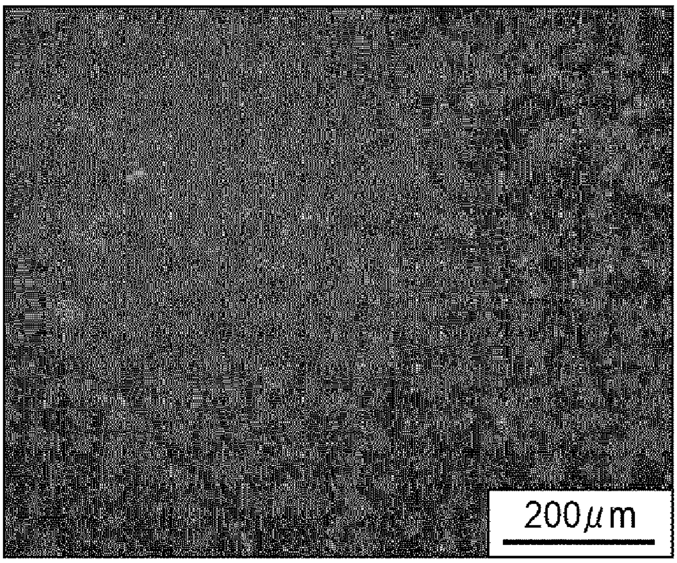

[Figure 3]

(a)

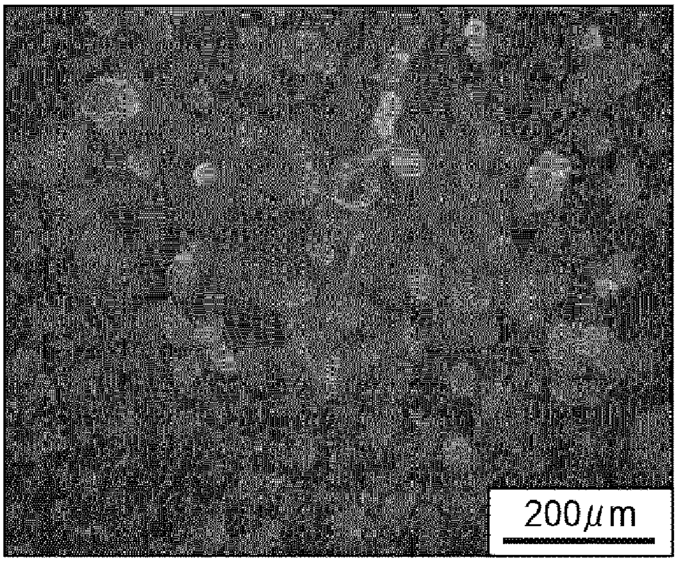


(b)

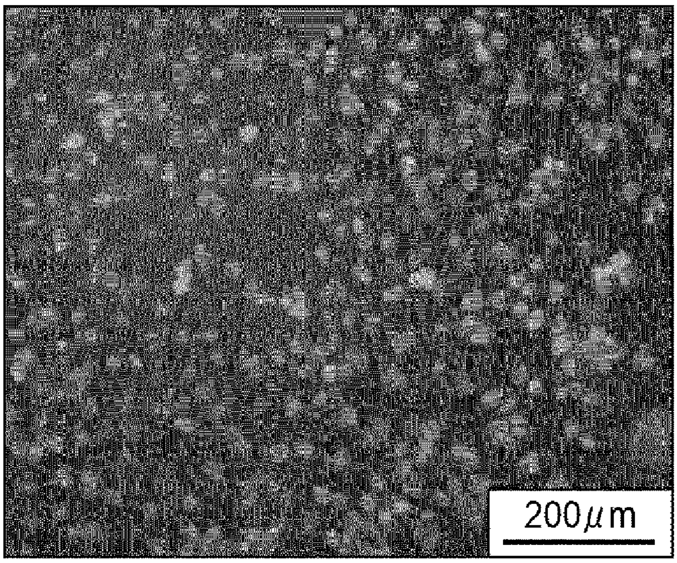

## INTERNATIONAL SEARCH REPORT

International application No.
**PCT/JP2021/038728**

**A. CLASSIFICATION OF SUBJECT MATTER**

***A61P 35/00***(2006.01)i; ***A61P 43/00***(2006.01)i; ***C08G 61/12***(2006.01)i; ***A61K 47/56***(2017.01)i; ***A61K 47/61***(2017.01)i; ***A61K 47/64***(2017.01)i; ***A61K 47/68***(2017.01)i; ***A61K 49/00***(2006.01)i; ***A61K 41/00***(2020.01)i
FI: C08G61/12; A61K49/00; A61P35/00; A61K41/00; A61P43/00 125; A61K47/64; A61K47/68; A61K47/61; A61K47/56

According to International Patent Classification (IPC) or to both national classification and IPC

**B. FIELDS SEARCHED**

Minimum documentation searched (classification system followed by classification symbols)

A61P35/00; A61P43/00; C08G61/12; A61K47/56; A61K47/61; A61K47/64; A61K47/68; A61K49/00; A61K41/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 2011/052709 A1 (SUMITOMO CHEMICAL CO., LTD.) 05 May 2011 (2011-05-05) | 1-26 |
| A | JP 2015-514157 A (WAKE FOREST UNIV.) 18 May 2015 (2015-05-18) | 1-26 |
| A | JP 2017-17165 A (FUJIFILM CORP.) 19 January 2017 (2017-01-19) | 1-26 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 December 2021** | **18 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/038728**

| Patent document cited in search report | Publication date (day/month/year) | Patent family member(s) | Publication date (day/month/year) |
|---|---|---|---|
| WO 2011/052709 A1 | 05 May 2011 | JP 2012-36357 A | |
| | | US 2012/0205596 A1 | |
| | | US 2016/0372675 A1 | |
| | | CN 102597047 A | |
| | | KR 10-2012-0100899 A | |
| | | CN 105198909 A | |
| | | CN 105254645 A | |
| | | CN 105601662 A | |
| JP 2015-514157 A | 18 May 2015 | US 2015/0069302 A1 | |
| | | WO 2013/155463 A1 | |
| | | EP 2836533 A1 | |
| | | CN 104364292 A | |
| JP 2017-17165 A | 19 January 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2014129317 A
- WO 2011052709 A
- WO 2018060722 A
- US 20150031996 A
- WO 2013051676 A


### Non-patent literature cited in the description


- *Chem. Lett.,* 2018, vol. 47, 927
- *Sci. Rep.,* 2015, vol. 5, 9321